(19) Europäisches Patentamt
European Patent Office
Office européen des brevets

(11) EP 1 632 778 A2

(12) **EUROPEAN PATENT APPLICATION**

(43) Date of publication:
**08.03.2006 Bulletin 2006/10**

(51) Int Cl.:
***G01N 33/74*** *(2006.01)*

(21) Application number: **05021581.3**

(22) Date of filing: **09.07.2002**

(84) Designated Contracting States:
**AT BE BG CH CY CZ DE DK EE ES FI FR GB GR IE IT LI LU MC NL PT SE SK TR**

(30) Priority: **09.07.2001 US 303858 P**
**13.07.2001 US 905253**

(62) Document number(s) of the earlier application(s) in accordance with Art. 76 EPC:
**02754857.7 / 1 405 083**

(71) Applicant: **Euroscreen S.A.**
**1070 Brussels (BE)**

(72) Inventors:
- **Wittamer, Valerie**
  **1410 Waterloo (BE)**
- **Communi, David**
  **1440 Braine-le-Chateau (BE)**
- **Vandenbogaerde, Ann**
  **80993 München (DE)**
- **Detheux, Michel**
  **7190 Ecaussinnes (BE)**
- **Parmentier, Marc**
  **1650 Beersel (BE)**

(74) Representative: **De Clercq, Ann G. Y. et al**
**De Clercq, Brants & Partners c.v.,**
**Edgard Gevaertdreef 10a**
**9830 Sint-Martens-Latem (BE)**

Remarks:
This application was filed on 03 - 10 - 2005 as a divisional application to the application mentioned under INID code 62.

(54) **Natural ligand of gpcr chemr23 and uses thereof**

(57) The invention relates to the identification of TIG2, the polypeptide product of Tazarotene-Induced Genc-2, as a natural ligand of the ChemR23 G-protein coupled receptor (GPCR). The invention encompasses the use of the interaction of ChemR23 polypeptides and TIG2 polypeptides as the basis of screening assays for agents that modulate the activity of the ChemR23 receptor. The invention also encompasses diagnostic assays based upon the ChemR23/TIG2 interaction, as well as kits for performing diagnostic and screening assays.

**Figure 14**

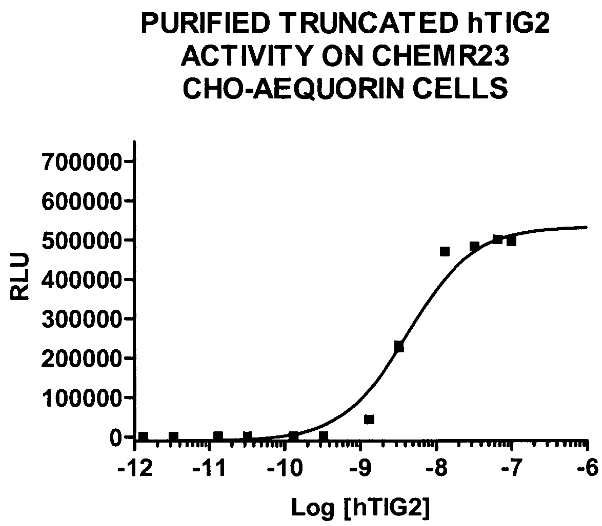


EP 1 632 778 A2

SEQUENCE LISTING

<110> Euroscreen S.A.

<120> Natural ligand of G protein coupled receptor CHEMR23 and uses thereof

<130> EUR-006-EP-DIV1

<150> US 60/303,858
<151> 2001-07-09

<150> PCT/EP02/07647
<151> 2002-07-09

<150> EP 02754857.7
<151> 2002-07-09

<150> US 09/905,253
<151> 2001-07-13

<160> 58

<170> PatentIn version 3.3

<210> 1
<211> 1116
<212> DNA
<213> Homo sapiens

<400> 1
atggaggatg aagattacaa cacttccatc agttacggtg atgaataccc tgattattta 60

gactccattg tggttttgga ggacttatcc cccttggaag ccagggtgac caggatcttc 120

ctggtggtgg tctacagcat cgtctgcttc ctcgggattc tgggcaatgg tctggtgatc 180

atcattgcca ccttcaagat gaagaagaca gtgaacatgg tctggttcct caacctggca 240

gtggcagatt tcctgttcaa cgtcttcctc ccaatccata tcacctatgc cgccatggac 300

taccactggg ttttcgggac agccatgtgc aagatcagca acttccttct catccacaac 360

atgttcacca gcgtcttcct gctgaccatc atcagctctg accgctgcat ctctgtgctc 420

ctccctgtct ggtcccagaa ccaccgcagc gttcgcctgg cttacatggc ctgcatggtc 480

atctgggtcc tggctttctt cttgagttcc ccatctctcg tcttccggga cacagccaac 540

ctgcatggga aaatatcctg cttcaacaac ttcagcctgt ccacacctgg gtcttcctcg 600

tggcccactc actcccaaat ggaccctgtg gggtatagcc ggcacatggt ggtgactgtc 660

acccgcttcc tctgtggctt cctggtccca gtcctcatca tcacagcttg ctacctcacc 720

atcgtctgca aactgcagcg caaccgcctg gccaagacca agaagccctt caagattatt 780

gtgaccatca tcattacctt cttcctctgc tggtgcccct accacacact caacctccta 840

gagctccacc acactgccat gcctggctct gtcttcagcc tgggtttgcc cctggccact 900

gcccttgcca ttgccaacag ctgcatgaac cccattctgt atgttttcat gggtcaggac 960

ttcaagaagt tcaaggtggc cctcttctct cgcctggtca atgctctaag tgaagataca 1020

ggccactctt cctaccccag ccatagaagc tttaccaaga tgtcatcaat gaatgagagg 1080

acttctatga atgagaggga gaccggcatg ctttga 1116

<210> 2
<211> 371
<212> PRT
<213> Homo sapiens

<400> 2

```
Met Glu Asp Glu Asp Tyr Asn Thr Ser Ile Ser Tyr Gly Asp Glu Tyr
1               5                   10                  15

Pro Asp Tyr Leu Asp Ser Ile Val Val Leu Glu Asp Leu Ser Pro Leu
            20                  25                  30

Glu Ala Arg Val Thr Arg Ile Phe Leu Val Val Val Tyr Ser Ile Val
        35                  40                  45

Cys Phe Leu Gly Ile Leu Gly Asn Gly Leu Val Ile Ile Ile Ala Thr
    50                  55                  60

Phe Lys Met Lys Lys Thr Val Asn Met Val Trp Phe Leu Asn Leu Ala
65                  70                  75                  80

Val Ala Asp Phe Leu Phe Asn Val Phe Leu Pro Ile His Ile Thr Tyr
                85                  90                  95

Ala Ala Met Asp Tyr His Trp Val Phe Gly Thr Ala Met Cys Lys Ile
            100                 105                 110

Ser Asn Phe Leu Leu Ile His Asn Met Phe Thr Ser Val Phe Leu Leu
        115                 120                 125

Thr Ile Ile Ser Ser Asp Arg Cys Ile Ser Val Leu Leu Pro Val Trp
    130                 135                 140

Ser Gln Asn His Arg Ser Val Arg Leu Ala Tyr Met Ala Cys Met Val
145                 150                 155                 160

Ile Trp Val Leu Ala Phe Phe Leu Ser Ser Pro Ser Leu Val Phe Arg
                165                 170                 175

Asp Thr Ala Asn Leu His Gly Lys Ile Ser Cys Phe Asn Asn Phe Ser
            180                 185                 190

Leu Ser Thr Pro Gly Ser Ser Ser Trp Pro Thr His Ser Gln Met Asp
        195                 200                 205

Pro Val Gly Tyr Ser Arg His Met Val Val Thr Val Thr Arg Phe Leu
    210                 215                 220

Cys Gly Phe Leu Val Pro Val Leu Ile Ile Thr Ala Cys Tyr Leu Thr
225                 230                 235                 240
```

```
Ile Val Cys Lys Leu Gln Arg Asn Arg Leu Ala Lys Thr Lys Lys Pro
                245                 250                 255

Phe Lys Ile Ile Val Thr Ile Ile Ile Thr Phe Phe Leu Cys Trp Cys
            260                 265                 270

Pro Tyr His Thr Leu Asn Leu Leu Glu Leu His His Thr Ala Met Pro
        275                 280                 285

Gly Ser Val Phe Ser Leu Gly Leu Pro Leu Ala Thr Ala Leu Ala Ile
    290                 295                 300

Ala Asn Ser Cys Met Asn Pro Ile Leu Tyr Val Phe Met Gly Gln Asp
305                 310                 315                 320

Phe Lys Lys Phe Lys Val Ala Leu Phe Ser Arg Leu Val Asn Ala Leu
                325                 330                 335

Ser Glu Asp Thr Gly His Ser Ser Tyr Pro Ser His Arg Ser Phe Thr
            340                 345                 350

Lys Met Ser Ser Met Asn Glu Arg Thr Ser Met Asn Glu Arg Glu Thr
        355                 360                 365

Gly Met Leu
    370

<210>  3
<211>  1116
<212>  DNA
<213>  Mus musculus

<400>  3
atggagtacg acgcttacaa cgactccggc atctatgatg atgagtactc tgatggcttt      60
ggctactttg tggacttgga ggaggcgagt ccgtgggagg ccaaggtggc cccggtcttc     120
ctggtggtga tctacagctt ggtgtgcttc ctcggtctcc taggcaacgg cctggtgatt     180
gtcatcgcca ccttcaagat gaagaagacc gtgaacactg tgtggtttgt caacctggct     240
gtggccgact tcctgttcaa catctttttg ccgatgcaca tcacctacgc ggccatggac     300
taccactggg tgttcgggaa ggccatgtgc aagatcagca acttcttgct cagccacaac     360
atgtacacca gcgtcttcct gctgactgtc atcagctttg accgctgcat ctccgtgctg     420
ctccccgtct ggtcccagaa ccaccgcagc atccgcctgg cctacatgac ctgctcggcc     480
gtctgggtcc tggctttctt cttgagctcc ccgtcccttg tcttccggga caccgccaac     540
attcatggga agataacctg cttcaacaac ttcagcttgg ccgcgcctga gtcctcccca     600
catcccgccc actcgcaagt agtttccaca gggtacagca gacacgtggc ggtcactgtc     660
acccgcttcc tttgcggctt cctgatcccc gtcttcatca tcacggcctg ctaccttacc     720
atcgtcttca agctgcagcg caaccgcctg gccaagaaca agaagccctt caagatcatc     780
```

atcaccatca tcatcacctt cttcctctgc tggtgcccct accacaccct ctacctgctg 840
gagctccacc acacagctgt gccaagctct gtcttcagcc tggggctacc cctggccacg 900
gccgtcgcca tcgccaacag ctgcatgaac cccattctgt acgtcttcat gggccacgac 960
ttcagaaaat tcaaggtggc cctcttctcc cgcctggcca acgccctgag tgaggacaca 1020
ggcccctcct cctaccccag tcacaggagc ttcaccaaga tgtcgtcttt gaatgagaag 1080
gcttcggtga atgagaagga gaccagtacc ctctga 1116

<210> 4
<211> 371
<212> PRT
<213> Mus musculus

<400> 4

Met Glu Tyr Asp Ala Tyr Asn Asp Ser Gly Ile Tyr Asp Asp Glu Tyr
1 5 10 15

Ser Asp Gly Phe Gly Tyr Phe Val Asp Leu Glu Glu Ala Ser Pro Trp
20 25 30

Glu Ala Lys Val Ala Pro Val Phe Leu Val Val Ile Tyr Ser Leu Val
35 40 45

Cys Phe Leu Gly Leu Leu Gly Asn Gly Leu Val Ile Val Ile Ala Thr
50 55 60

Phe Lys Met Lys Lys Thr Val Asn Thr Val Trp Phe Val Asn Leu Ala
65 70 75 80

Val Ala Asp Phe Leu Phe Asn Ile Phe Leu Pro Met His Ile Thr Tyr
85 90 95

Ala Ala Met Asp Tyr His Trp Val Phe Gly Lys Ala Met Cys Lys Ile
100 105 110

Ser Asn Phe Leu Leu Ser His Asn Met Tyr Thr Ser Val Phe Leu Leu
115 120 125

Thr Val Ile Ser Phe Asp Arg Cys Ile Ser Val Leu Leu Pro Val Trp
130 135 140

Ser Gln Asn His Arg Ser Ile Arg Leu Ala Tyr Met Thr Cys Ser Ala
145 150 155 160

Val Trp Val Leu Ala Phe Phe Leu Ser Ser Pro Ser Leu Val Phe Arg
165 170 175

Asp Thr Ala Asn Ile His Gly Lys Ile Thr Cys Phe Asn Asn Phe Ser
180 185 190

Leu Ala Ala Pro Glu Ser Ser Pro His Pro Ala His Ser Gln Val Val
195 200 205

Ser Thr Gly Tyr Ser Arg His Val Ala Val Thr Val Thr Arg Phe Leu
210 215 220

Cys Gly Phe Leu Ile Pro Val Phe Ile Ile Thr Ala Cys Tyr Leu Thr
225 230 235 240

Ile Val Phe Lys Leu Gln Arg Asn Arg Leu Ala Lys Asn Lys Lys Pro
245 250 255

Phe Lys Ile Ile Ile Thr Ile Ile Ile Thr Phe Phe Leu Cys Trp Cys
260 265 270

Pro Tyr His Thr Leu Tyr Leu Leu Glu Leu His His Thr Ala Val Pro
275 280 285

Ser Ser Val Phe Ser Leu Gly Leu Pro Leu Ala Thr Ala Val Ala Ile
290 295 300

Ala Asn Ser Cys Met Asn Pro Ile Leu Tyr Val Phe Met Gly His Asp
305 310 315 320

Phe Arg Lys Phe Lys Val Ala Leu Phe Ser Arg Leu Ala Asn Ala Leu
325 330 335

Ser Glu Asp Thr Gly Pro Ser Ser Tyr Pro Ser His Arg Ser Phe Thr
340 345 350

Lys Met Ser Ser Leu Asn Glu Lys Ala Ser Val Asn Glu Lys Glu Thr
355 360 365

Ser Thr Leu
370

<210> 5
<211> 1116
<212> DNA
<213> Rattus rattus

<400> 5
atggagtacg agggttacaa cgactccagc atctacggtg aggagtattc tgacggctcg 60

gactacatcg tggacttgga ggaggcgggt ccactggagg ccaaggtggc cgaggtcttc 120

ctggtggtaa tctacagctt ggtgtgcttc ctcgggatcc taggcaatgg cctggtgatt 180

gtcatcgcca ccttcaagat gaagaagacg gtgaacaccg tgtggtttgt caacctggcc 240

gtggctgact tcctgttcaa catcttcttg cccatccaca tcacctatgc cgctatggac 300

taccactggg tgttcgggaa agccatgtgc aagattagta gctttctgct aagccacaac 360

atgtacacca gcgtcttcct gctcactgtc atcagcttcg accgctgcat ctccgtgctc 420

ctccccgtct ggtcccagaa ccaccgcagc gtgcgtctgg cctacatgac ctgcgtggtt 480

gtctgggtct ggctttcttc tgagtctccc ccgtccctcg tcttcggaca cgtcagcacc 540

agccacggga agataacctg cttcaacaac ttcagcctgg cggcgcccga gcctttctct 600

cattccaccc acccgcgaac agacccggta gggtacagca gacatgtggc ggtcaccgtc 660

acccgcttcc tctgtggctt cctgatcccc gtcttcatca tcacggcctg ttacctcacc 720

atcgtcttca agttgcagcg caaccgccag gccaagacca agaagccctt caagatcatc 780

atcaccatca tcatcacctt cttcctctgc tggtgcccct accacacact ctacctgctg 840

gagctccacc acacggctgt gccagcctct gtcttcagcc tgggactgcc cctggccaca 900

gccgtcgcca tcgccaacag ctgtatgaac cccatcctgt acgtcttcat gggccacgac 960

ttcaaaaaat tcaaggtggc ccttttctcc cgcctggtga atgccctgag cgaggacaca 1020

ggaccctcct cctaccccag tcacaggagc ttcaccaaga tgtcctcatt gattgagaag 1080

gcttcagtga atgagaaaga gaccagcacc ctctga 1116

<210> 6
<211> 371
<212> PRT
<213> Rattus rattus

<400> 6

Met Glu Tyr Glu Gly Tyr Asn Asp Ser Ser Ile Tyr Gly Glu Glu Tyr
1 5 10 15

Ser Asp Gly Ser Asp Tyr Ile Val Asp Leu Glu Glu Ala Gly Pro Leu
20 25 30

Glu Ala Lys Val Ala Glu Val Phe Leu Val Val Ile Tyr Ser Leu Val
35 40 45

Cys Phe Leu Gly Ile Leu Gly Asn Gly Leu Val Ile Val Ile Ala Thr
50 55 60

Phe Lys Met Lys Lys Thr Val Asn Thr Val Trp Phe Val Asn Leu Ala
65 70 75 80

Val Ala Asp Phe Leu Phe Asn Ile Phe Leu Pro Ile His Ile Thr Tyr
85 90 95

Ala Ala Met Asp Tyr His Trp Val Phe Gly Lys Ala Met Cys Lys Ile
100 105 110

Ser Ser Phe Leu Leu Ser His Asn Met Tyr Thr Ser Val Phe Leu Leu
115 120 125

Thr Val Ile Ser Phe Asp Arg Cys Ile Ser Val Leu Leu Pro Val Trp
130 135 140

Ser Gln Asn His Arg Ser Val Arg Leu Ala Tyr Met Thr Cys Val Val
145 150 155 160

Val Trp Val Trp Leu Ser Ser Glu Ser Pro Pro Ser Leu Val Phe Gly
165 170 175

His Val Ser Thr Ser His Gly Lys Ile Thr Cys Phe Asn Asn Phe Ser
180 185 190

Leu Ala Ala Pro Glu Pro Phe Ser His Ser Thr His Pro Arg Thr Asp
195 200 205

Pro Val Gly Tyr Ser Arg His Val Ala Val Thr Val Thr Arg Phe Leu
210 215 220

Cys Gly Phe Leu Ile Pro Val Phe Ile Ile Thr Ala Cys Tyr Leu Thr
225 230 235 240

Ile Val Phe Lys Leu Gln Arg Asn Arg Gln Ala Lys Thr Lys Lys Pro
245 250 255

Phe Lys Ile Ile Ile Thr Ile Ile Ile Thr Phe Phe Leu Cys Trp Cys
260 265 270

Pro Tyr His Thr Leu Tyr Leu Leu Glu Leu His His Thr Ala Val Pro
275 280 285

Ala Ser Val Phe Ser Leu Gly Leu Pro Leu Ala Thr Ala Val Ala Ile
290 295 300

Ala Asn Ser Cys Met Asn Pro Ile Leu Tyr Val Phe Met Gly His Asp
305 310 315 320

Phe Lys Lys Phe Lys Val Ala Leu Phe Ser Arg Leu Val Asn Ala Leu
325 330 335

Ser Glu Asp Thr Gly Pro Ser Ser Tyr Pro Ser His Arg Ser Phe Thr
340 345 350

Lys Met Ser Ser Leu Ile Glu Lys Ala Ser Val Asn Glu Lys Glu Thr
355 360 365

Ser Thr Leu
370

<210> 7
<211> 492
<212> DNA
<213> Homo sapiens

<400> 7
atgcgacggc tgctgatccc tctggccctg tggctgggtg cggtgggcgt gggcgtcgcc 60

gagctcacgg aagcccagcg ccggggcctg caggtggccc tggaggaatt tcacaagcac 120

ccgcccgtgc agtgggcctt ccaggagacc agtgtggaga gcgccgtgga cacgcccttc 180

ccagctggaa tatttgtgag gctggaattt aagctgcagc agacaagctg ccggaagagg 240

gactggaaga aacccgagtg caaagtcagg cccaatggga ggaaacggaa atgcctggcc 300

tgcatcaaac tgggctctga ggacaaagtt ctgggccggt tggtccactg ccccatagag 360

acccaagttc tgcgggaggc tgaggagcac caggagaccc agtgcctcag ggtgcagcgg 420

gctggtgagg acccccacag cttctacttc cctggacagt tcgccttctc caaggccctg 480

ccccgcagct aa 492

<210> 8
<211> 163
<212> PRT
<213> Homo sapiens

<400> 8

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe Ser Lys Ala Leu
145 150 155 160

Pro Arg Ser

<210> 9
<211> 489
<212> DNA
<213> Mus musculus

<400> 9
atgaagtgct tgctgatctc cctagcccta tggctgggca cagtgggcac acgtgggaca 60

gagcccgaac tcagcgagac ccagcgcagg agcctacagg tggctctgga ggagttccac 120

aaacacccac ctgtgcagtt ggccttccaa gagatcggtg tggacagagc tgaagaagtg 180

ctcttctcag ctggcacctt tgtgaggttg gaatttaagc tccagcagac caactgcccc 240

aagaaggact ggaaaaagcc ggagtgcaca atcaaaccaa acgggagaag gcggaaatgc 300

ctggcctgca ttaaaatgga ccccaagggt aaaattctag gccggatagt ccactgccca 360

attctgaagc aagggcctca ggatcctcag gagttgcaat gcattaagat agcacaggct 420

ggcgaagacc cccacggcta cttcctacct ggacagtttg ccttctccag ggccctgaga 480

accaaataa 489

<210> 10
<211> 162
<212> PRT
<213> Mus musculus

<400> 10

Met Lys Cys Leu Leu Ile Ser Leu Ala Leu Trp Leu Gly Thr Val Gly
1 5 10 15

Thr Arg Gly Thr Glu Pro Glu Leu Ser Glu Thr Gln Arg Arg Ser Leu
20 25 30

Gln Val Ala Leu Glu Glu Phe His Lys His Pro Pro Val Gln Leu Ala
35 40 45

Phe Gln Glu Ile Gly Val Asp Arg Ala Glu Glu Val Leu Phe Ser Ala
50 55 60

Gly Thr Phe Val Arg Leu Glu Phe Lys Leu Gln Gln Thr Asn Cys Pro
65 70 75 80

Lys Lys Asp Trp Lys Lys Pro Glu Cys Thr Ile Lys Pro Asn Gly Arg
85 90 95

Arg Arg Lys Cys Leu Ala Cys Ile Lys Met Asp Pro Lys Gly Lys Ile
100 105 110

Leu Gly Arg Ile Val His Cys Pro Ile Leu Lys Gln Gly Pro Gln Asp
115 120 125

Pro Gln Glu Leu Gln Cys Ile Lys Ile Ala Gln Ala Gly Glu Asp Pro
130 135 140

His Gly Tyr Phe Leu Pro Gly Gln Phe Ala Phe Ser Arg Ala Leu Arg
145 150 155 160

Thr Lys

<210> 11
<211> 13
<212> PRT
<213> Artificial Sequence

<220>
<223> Synthetic peptide

<400> 11

Arg Arg Leu Ile Glu Asp Ala Glu Tyr Ala Ala Arg Gly
1 5 10

<210> 12
<211> 8
<212> DNA
<213> Artificial Sequence

<220>
<223> Oligonucleotide

<400> 12
tgacgtca 8

<210> 13
<211> 9
<212> PRT
<213> Homo sapiens

<220>
<221> MISC_FEATURE
<222> (1)..(1)
<223> If present, X = Lys

<220>
<221> MISC_FEATURE
<222> (9)..(9)
<223> If present, X = Lys

<400> 13

Xaa Leu Gln Gln Thr Ser Cys Arg Xaa
1 5

<210> 14
<211> 10
<212> PRT
<213> Homo sapiens

<220>
<221> MISC_FEATURE
<222> (1)..(1)
<223> If present, X = Arg

<220>
<221> MISC_FEATURE
<222> (10)..(10)
<223> If present, X = Val

<400> 14

Xaa Asp Trp Lys Lys Pro Glu Cys Lys Xaa
1 5 10

<210> 15
<211> 13
<212> PRT
<213> Homo sapiens

<220>
<221> MISC_FEATURE
<222> (1)..(1)
<223> If present, X = Arg

<220>
<221> MISC_FEATURE
<222> (13)..(13)
<223> If present, X = His

<400> 15

Xaa Gly Leu Gln Val Ala Leu Glu Glu Phe His Lys Xaa
1 5 10

<210> 16
<211> 14
<212> PRT
<213> Homo sapiens

<220>
<221> MISC_FEATURE
<222> (1)..(1)
<223> If present, X = Lys

<220>
<221> MISC_FEATURE
<222> (14)..(14)
<223> If present, X = Val

<400> 16

Xaa Cys Leu Ala Cys Ile Lys Leu Gly Ser Glu Asp Lys Xaa
1 5 10

<210> 17
<211> 14
<212> PRT
<213> Homo sapiens

<220>
<221> MISC_FEATURE
<222> (1)..(1)
<223> If present, X = Arg

<220>
<221> MISC_FEATURE

<222> (14)..(14)
<223> If present, X = Glu

<400> 17

Xaa Leu Val His Cys Pro Ile Glu Thr Gln Val Leu Arg Xaa
1 5 10

<210> 18
<211> 14
<212> PRT
<213> Homo sapiens

<220>
<221> MISC_FEATURE
<222> (1)..(1)
<223> If present, X = Arg

<220>
<221> MISC_FEATURE
<222> (14)..(14)
<223> If present, X = His

<400> 18

Xaa Arg Gly Leu Gln Val Ala Leu Glu Glu Phe His Lys Xaa
1 5 10

<210> 19
<211> 14
<212> PRT
<213> Homo sapiens

<220>
<221> MISC_FEATURE
<222> (1)..(1)
<223> If present, X = Arg

<220>
<221> MISC_FEATURE
<222> (14)..(14)
<223> If present, X = Val

<400> 19

Xaa Glu Ala Glu Glu His Gln Glu Thr Gln Cys Leu Arg Xaa
1 5 10

<210> 20
<211> 28
<212> DNA
<213> Artificial Sequence

<220>
<223> Oligonucleotide

<400> 20
caggaattca gcatgcgacg gctgctga 28

<210> 21
<211> 29
<212> DNA

<213> Artificial Sequence

<220>
<223> Oligonucleotide

<400> 21
gctctagatt agctgcgggg cagggcctt 29

<210> 22
<211> 48
<212> DNA
<213> Mus musculus

<400> 22
tctctcgaga aaagagaggc tgaagctaca cgtgggacag agcccgaa 48

<210> 23
<211> 48
<212> DNA
<213> Homo sapiens

<400> 23
tctctcgaga aaagagaggc tgaagctggc gtcgccgagc tcacggaa 48

<210> 24
<211> 48
<212> DNA
<213> Homo sapiens

<400> 24
tctctcgaga aaagagaggc tgaagctgtg ggcgtcgccg agctcacg 48

<210> 25
<211> 30
<212> DNA
<213> Mus musculus

<400> 25
agggaattct tatttggttc tcagggccct 30

<210> 26
<211> 30
<212> DNA
<213> Homo sapiens

<400> 26
agggaattct tagctgcggg gcagggcctt 30

<210> 27
<211> 28
<212> DNA
<213> Mus musculus

<400> 27
caggaattcg ccatgaagtg cttgctga 28

<210> 28
<211> 28
<212> DNA
<213> Homo sapiens

<400> 28
caggaattca gcatgcgacg gctgctga 28

<210> 29
<211> 29
<212> DNA
<213> Mus musculus

<400> 29
gctctagatt tggttctcag ggccctgga 29

<210> 30
<211> 29
<212> DNA
<213> Homo sapiens

<400> 30
gctctagagc tgcggggcag ggccttgga 29

<210> 31
<211> 160
<212> PRT
<213> Rattus rattus

<400> 31

Met Lys Cys Leu Leu Ile Ser Leu Ala Leu Trp Leu Gly Thr Ala Asp
1 5 10 15

Ile His Gly Thr Glu Leu Glu Leu Ser Glu Thr Gln Arg Arg Gly Leu
20 25 30

Gln Val Ala Leu Glu Glu Phe His Arg His Pro Pro Val Gln Trp Ala
35 40 45

Phe Gln Glu Ile Gly Val Asp Ser Ala Asp Asp Leu Phe Phe Ser Ala
50 55 60

Gly Thr Phe Val Arg Leu Glu Phe Lys Leu Gln Gln Thr Ser Cys Leu
65 70 75 80

Lys Lys Asp Trp Lys Lys Pro Glu Cys Thr Ile Lys Pro Asn Gly Arg
85 90 95

Lys Arg Lys Cys Leu Ala Cys Ile Lys Leu Asp Pro Lys Gly Lys Val
100 105 110

Leu Gly Arg Met Val His Cys Pro Ile Leu Lys Gln Gly Pro Gln Gln
115 120 125

Glu Pro Gln Glu Ser Gln Cys Ser Lys Ile Ala Gln Ala Gly Glu Asp
130 135 140

Ser Arg Ile Tyr Phe Phe Pro Gly Gln Phe Ala Phe Ser Arg Ala Leu
145 150 155 160

<210> 32
<211> 163
<212> PRT
<213> Sus scrofa

<400> 32

Met Trp Gln Leu Leu Leu Pro Leu Ala Leu Trp Leu Gly Thr Met Gly
1 5 10 15

Leu Gly Arg Ala Glu Leu Thr Ala Ala Gln Leu Arg Gly Leu Gln Val
20 25 30

Ala Leu Glu Glu Phe His Lys His Pro Pro Val Gln Trp Ala Phe Arg
35 40 45

Glu Thr Gly Val Asn Ser Ala Met Asp Thr Pro Phe Pro Ala Gly Thr
50 55 60

Phe Val Arg Leu Glu Phe Lys Leu Gln Gln Thr Ser Cys Arg Lys Arg
65 70 75 80

Asp Trp Lys Lys Ala Glu Cys Lys Val Lys Pro Asn Gly Arg Lys Arg
85 90 95

Lys Cys Leu Ala Cys Ile Lys Leu Asn Ser Glu Asp Lys Val Leu Gly
100 105 110

Arg Met Val His Cys Pro Ile Glu Thr Gln Val Gln Arg Glu Pro Glu
115 120 125

Glu Arg Gln Glu Ala Gln Cys Ser Arg Val Glu Arg Ala Gly Glu Asp
130 135 140

Pro His Ser Tyr Tyr Phe Pro Gly Gln Phe Ala Phe Phe Lys Ala Leu
145 150 155 160

Pro Pro Ser

<210> 33
<211> 160
<212> PRT
<213> Bos taurus

<400> 33

Met Trp Gln Leu Leu Leu Pro Leu Ala Leu Gly Leu Gly Thr Met Gly
1 5 10 15

Leu Gly Arg Ala Glu Leu Thr Thr Ala Gln His Arg Gly Leu Gln Val
20 25 30

Ala Leu Glu Glu Phe His Lys His Pro Pro Val Leu Trp Ala Phe Gln

35 40 45

Val Thr Ser Val Asp Asn Ala Ala Asp Thr Leu Phe Pro Ala Gly Gln
50 55 60

Phe Val Arg Leu Glu Phe Lys Leu Gln Gln Thr Ser Cys Arg Lys Lys
65 70 75 80

Asp Trp Arg Lys Glu Asp Cys Lys Val Lys Pro Asn Gly Arg Lys Arg
85 90 95

Lys Cys Leu Ala Cys Ile Lys Leu Asp Ser Lys Asp Gln Val Leu Gly
100 105 110

Arg Met Val His Cys Pro Ile Gln Thr Gln Val Gln Arg Glu Leu Asp
115 120 125

Asp Ala Gln Asp Ala Gln Cys Ser Arg Val Glu Arg Ala Gly Glu Asp
130 135 140

Pro His Ser Tyr Tyr Leu Pro Gly Gln Phe Ala Phe Ile Lys Ala Leu
145 150 155 160

<210> 34
<211> 165
<212> PRT
<213> Gallus gallus

<400> 34

Arg Ala Val Gly Met Lys Leu Leu Leu Gly Ile Ala Val Val Val Leu
1 5 10 15

Ala Leu Ala Asp Ala Gly Gln Ser Pro Leu Gln Arg Arg Val Val Lys
20 25 30

Asp Val Leu Asp Tyr Phe His Ser Arg Ser Asn Val Gln Phe Leu Phe
35 40 45

Arg Glu Gln Ser Val Glu Gly Ala Val Glu Arg Val Asp Ser Ser Gly
50 55 60

Thr Phe Val Gln Leu His Leu Asn Leu Ala Gln Thr Ala Cys Arg Lys
65 70 75 80

Gln Ala Gln Arg Lys Gln Asn Cys Arg Ile Met Glu Asn Arg Arg Lys
85 90 95

Pro Val Cys Leu Ala Cys Tyr Lys Phe Asp Ser Ser Asp Val Pro Lys
100 105 110

Val Leu Asp Lys Tyr Tyr Asn Cys Gly Pro Ser His His Leu Ala Met
115 120 125

Lys Asp Ile Lys His Arg Asp Glu Ala Glu Cys Arg Ala Val Glu Glu
    130                 135                 140

Ala Gly Lys Thr Ser Asp Val Leu Tyr Leu Pro Gly Met Phe Ala Phe
145                 150                 155                 160

Ser Lys Gly Leu Pro
                165

<210> 35
<211> 7
<212> PRT
<213> Artificial Sequence

<220>
<223> Synthetic peptide

<220>
<221> MISC_FEATURE
<222> (1)..(1)
<223> X = Ac-F

<220>
<221> MISC_FEATURE
<222> (7)..(7)
<223> X = L-NH2

<400> 35

Xaa Lys Lys Ser Phe Lys Xaa
1               5

<210> 36
<211> 11
<212> DNA
<213> Artificial Sequence

<220>
<223> Oligonucleotide

<400> 36
ggggactttc c    11

<210> 37
<211> 17
<212> DNA
<213> Artificial Sequence

<220>
<223> Oligonucleotide

<400> 37
gcagacaagc tgccgga    17

<210> 38
<211> 23
<212> DNA
<213> Artificial Sequence

<220>
<223> Oligonucleotide

<400> 38
aacccgagtg caaagtcagg ccc 23

<210> 39
<211> 19
<212> DNA
<213> Artificial Sequence

<220>
<223> Oligonucleotide

<400> 39
agtttgatgc aggccaggc 19

<210> 40
<211> 18
<212> DNA
<213> Artificial Sequence

<220>
<223> Oligonucleotide

<400> 40
gtcccagaac caccgcag 18

<210> 41
<211> 23
<212> DNA
<213> Artificial Sequence

<220>
<223> Oligonucleotide

<400> 41
ttcgcctggc ttacatggcc tgc 23

<210> 42
<211> 21
<212> DNA
<213> Artificial Sequence

<220>
<223> Oligonucleotide

<400> 42
aagaaagcca ggacccagat g 21

<210> 43
<211> 19
<212> DNA
<213> Artificial Sequence

<220>
<223> Oligonucleotide

<400> 43
gaaggtgaag gtcggagtc 19

<210> 44

<211> 20
<212> DNA
<213> Artificial Sequence

<220>
<223> Oligonucleotide

<400> 44
agctctcccg ccggcctctg 20

<210> 45
<211> 20
<212> DNA
<213> Artificial Sequence

<220>
<223> Oligonucleotide

<400> 45
gaagatggtg atgggatttc 20

<210> 46
<211> 157
<212> PRT
<213> Homo sapiens

<400> 46

```
Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
            20                  25                  30

Ala Leu Glu Glu Phe His Lys His Pro Pro Val Gln Trp Ala Phe Gln
        35                  40                  45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg
                85                  90                  95

Lys Cys Leu Ala Cys Ile Lys Leu Gly Ser Glu Asp Lys Val Leu Gly
            100                 105                 110

Arg Leu Val His Cys Pro Ile Glu Thr Gln Val Leu Arg Glu Ala Glu
        115                 120                 125

Glu His Gln Glu Thr Gln Cys Leu Arg Val Gln Arg Ala Gly Glu Asp
    130                 135                 140

Pro His Ser Phe Tyr Phe Pro Gly Gln Phe Ala Phe Ser
145                 150                 155
```

<210> 47
<211> 471
<212> DNA
<213> Homo sapiens

<400> 47
atgcgacggc tgctgatccc tctggccctg tggctgggtg cggtgggcgt gggcgtcgcc 60

gagctcacgg aagcccagcg ccggggcctg caggtggccc tggaggaatt tcacaagcac 120

ccgcccgtgc agtgggcctt ccaggagacc agtgtggaga gcgccgtgga cacgcccttc 180

ccagctggaa tatttgtgag gctggaattt aagctgcagc agacaagctg ccggaagagg 240

gactggaaga aacccgagtg caaagtcagg cccaatggga ggaaacggaa atgcctggcc 300

tgcatcaaac tgggctctga ggacaaagtt ctgggccggt tggtccactg ccccatagag 360

acccaagttc tgcgggaggc tgaggagcac caggagaccc agtgcctcag ggtgcagcgg 420

gctggtgagg acccccacag cttctacttc cctggacagt tcgccttctc c 471

<210> 48
<211> 151
<212> PRT
<213> Homo sapiens

<400> 48

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro
145 150

<210> 49
<211> 152
<212> PRT
<213> Homo sapiens

<400> 49

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly
145 150

<210> 50
<211> 153
<212> PRT
<213> Homo sapiens

<400> 50

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg
                85                  90                  95

Lys Cys Leu Ala Cys Ile Lys Leu Gly Ser Glu Asp Lys Val Leu Gly
            100                 105                 110

Arg Leu Val His Cys Pro Ile Glu Thr Gln Val Leu Arg Glu Ala Glu
        115                 120                 125

Glu His Gln Glu Thr Gln Cys Leu Arg Val Gln Arg Ala Gly Glu Asp
    130                 135                 140

Pro His Ser Phe Tyr Phe Pro Gly Gln
145                 150

<210> 51
<211> 154
<212> PRT
<213> Homo sapiens

<400> 51

Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
            20                  25                  30

Ala Leu Glu Glu Phe His Lys His Pro Pro Val Gln Trp Ala Phe Gln
        35                  40                  45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg
                85                  90                  95

Lys Cys Leu Ala Cys Ile Lys Leu Gly Ser Glu Asp Lys Val Leu Gly
            100                 105                 110

Arg Leu Val His Cys Pro Ile Glu Thr Gln Val Leu Arg Glu Ala Glu
        115                 120                 125

```
Glu His Gln Glu Thr Gln Cys Leu Arg Val Gln Arg Ala Gly Glu Asp
    130                 135                 140

Pro His Ser Phe Tyr Phe Pro Gly Gln Phe
145                 150

<210>  52
<211>  155
<212>  PRT
<213>  Homo sapiens

<400>  52

Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
            20                  25                  30

Ala Leu Glu Glu Phe His Lys His Pro Pro Val Gln Trp Ala Phe Gln
        35                  40                  45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg
                85                  90                  95

Lys Cys Leu Ala Cys Ile Lys Leu Gly Ser Glu Asp Lys Val Leu Gly
            100                 105                 110

Arg Leu Val His Cys Pro Ile Glu Thr Gln Val Leu Arg Glu Ala Glu
        115                 120                 125

Glu His Gln Glu Thr Gln Cys Leu Arg Val Gln Arg Ala Gly Glu Asp
    130                 135                 140

Pro His Ser Phe Tyr Phe Pro Gly Gln Phe Ala
145                 150                 155

<210>  53
<211>  156
<212>  PRT
<213>  Homo sapiens

<400>  53

Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
```

20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe
145 150 155

<210> 54
<211> 158
<212> PRT
<213> Homo sapiens

<400> 54

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe Ser Lys
145 150 155

<210> 55
<211> 159
<212> PRT
<213> Homo sapiens

<400> 55

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe Ser Lys Ala
145 150 155

<210> 56
<211> 160
<212> PRT
<213> Homo sapiens

<400> 56

Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
            20                  25                  30

Ala Leu Glu Glu Phe His Lys His Pro Pro Val Gln Trp Ala Phe Gln
        35                  40                  45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg
                85                  90                  95

Lys Cys Leu Ala Cys Ile Lys Leu Gly Ser Glu Asp Lys Val Leu Gly
            100                 105                 110

Arg Leu Val His Cys Pro Ile Glu Thr Gln Val Leu Arg Glu Ala Glu
        115                 120                 125

Glu His Gln Glu Thr Gln Cys Leu Arg Val Gln Arg Ala Gly Glu Asp
    130                 135                 140

Pro His Ser Phe Tyr Phe Pro Gly Gln Phe Ala Phe Ser Lys Ala Leu
145                 150                 155                 160

<210> 57
<211> 161
<212> PRT
<213> Homo sapiens

<400> 57

Met Arg Arg Leu Leu Ile Pro Leu Ala Leu Trp Leu Gly Ala Val Gly
1               5                   10                  15

Val Gly Val Ala Glu Leu Thr Glu Ala Gln Arg Arg Gly Leu Gln Val
            20                  25                  30

Ala Leu Glu Glu Phe His Lys His Pro Pro Val Gln Trp Ala Phe Gln
        35                  40                  45

Glu Thr Ser Val Glu Ser Ala Val Asp Thr Pro Phe Pro Ala Gly Ile
    50                  55                  60

Phe Val Arg Leu Glu Phe Lys Leu Gln Gln Thr Ser Cys Arg Lys Arg
65                  70                  75                  80

Asp Trp Lys Lys Pro Glu Cys Lys Val Arg Pro Asn Gly Arg Lys Arg

85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe Ser Lys Ala Leu
145 150 155 160

Pro

<210> 58
<211> 162
<212> PRT
<213> Homo sapiens

<400> 58

Met Arg Arg Leu Leu Ile Pro Leu Ala Leu Trp Leu Gly Ala Val Gly
1 5 10 15

Val Gly Val Ala Glu Leu Thr Glu Ala Gln Arg Arg Gly Leu Gln Val
20 25 30

Ala Leu Glu Glu Phe His Lys His Pro Pro Val Gln Trp Ala Phe Gln
35 40 45

Glu Thr Ser Val Glu Ser Ala Val Asp Thr Pro Phe Pro Ala Gly Ile
50 55 60

Phe Val Arg Leu Glu Phe Lys Leu Gln Gln Thr Ser Cys Arg Lys Arg
65 70 75 80

Asp Trp Lys Lys Pro Glu Cys Lys Val Arg Pro Asn Gly Arg Lys Arg
85 90 95

Lys Cys Leu Ala Cys Ile Lys Leu Gly Ser Glu Asp Lys Val Leu Gly
100 105 110

Arg Leu Val His Cys Pro Ile Glu Thr Gln Val Leu Arg Glu Ala Glu
115 120 125

Glu His Gln Glu Thr Gln Cys Leu Arg Val Gln Arg Ala Gly Glu Asp
130 135 140

Pro His Ser Phe Tyr Phe Pro Gly Gln Phe Ala Phe Ser Lys Ala Leu
145 150 155 160

Pro Arg

## Description

FIELD OF THE INVENTION

The invention relates to the identification of the natural ligand for the orphan G-Protein Coupled Receptor (GPCR) ChemR23 and uses thereof.

BACKGROUND OF THE INVENTION

G-protein coupled receptors (GPCRs) are proteins responsible for transducing a signal within a cell. GPCRs have usually seven transmembrane domains. Upon binding of a ligand to an extra-cellular portion or fragment of a GPCR, a signal is transduced within the cell that results in a change in a biological or physiological property or behaviour of the cell. GPCRs, along with G-proteins and effectors (intracellular enzymes and channels modulated by G-proteins), are the components of a modular signaling system that connects the state of intra-cellular second messengers to extra-cellular inputs.

GPCR genes and gene products can modulate various physiological processes and are potential causative agents of disease. The GPCRs seem to be of critical importance to both the central nervous system and peripheral physiological processes.

The GPCR protein superfamily is represented in five families : Family I, receptors typified by rhodopsin and the beta2-adrenergic receptor and currently represented by over 200 unique members; Family II, the parathyroid hormone/calcitonin/secretin receptor family; Family III, the metabotropic glutamate receptor family, Family IV, the CAMP receptor family, important in the chemotaxis and development of *D. discoideum;* and Family V, the fungal mating pheromone receptor such as STE2.

G proteins represent a family of heterotrimeric proteins composed of $\alpha$, $\beta$ and $\gamma$ subunits, that bind guanine nucleotides. These proteins are usually linked to cell surface receptors (receptors containing seven transmembrane domains) for signal transduction. Indeed, following ligand binding to the GPCR, a conformational change is transmitted to the G protein, which causes the $\alpha$-subunit to exchange a bound GDP molecule for a GTP molecule and to dissociate from the $\beta\gamma$-subunits.

The GTP-bound form of the $\alpha$, $\beta$ and $\gamma$-subunits typically functions as an effector-modulating moiety, leading to the production of second messengers, such as cAMP (e.g. by activation of adenyl cyclase), diacylglycerol or inositol phosphates.

Greater than 20 different types of $\alpha$-subunits are known in humans. These subunits associate with a small pool of $\beta$ and $\gamma$ subunits. Examples of mammalian G proteins include Gi, Go, Gq, Gs and Gt. G proteins are described extensively in Lodish et al., *Molecular Cell Biology* (Scientific American Books Inc., New York, N.Y., 1995; and also by Downes and Gautam, 1999, The G-Protein Subunit Gene Families. *Genomics* 62:544-552), the contents of both of which are incorporated herein by reference.

Known and uncharacterized GPCRs currently constitute major targets for drug action and development. There are ongoing efforts to identify new G protein coupled receptors which can be used to screen for new agonists and antagonists having potential prophylactic and therapeutical properties.

More than 300 GPCRs have been cloned to date, excluding the family of olfactory receptors. Mechanistically, approximately 50-60% of all clinically relevant drugs act by modulating the functions of various GPCRs (Cudermann et al., *J. Mol. Med.,* 73:51-63, 1995).

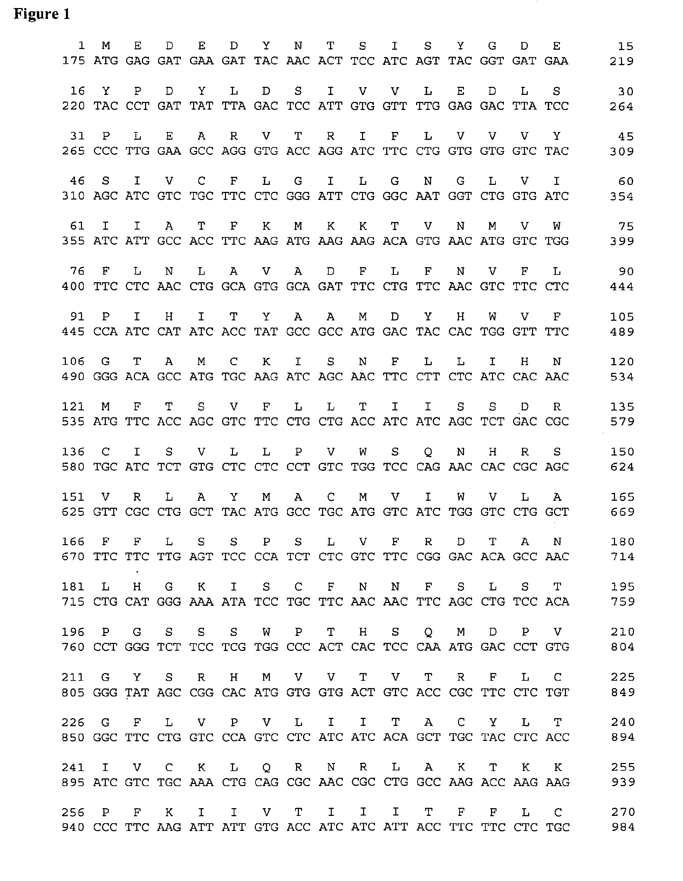

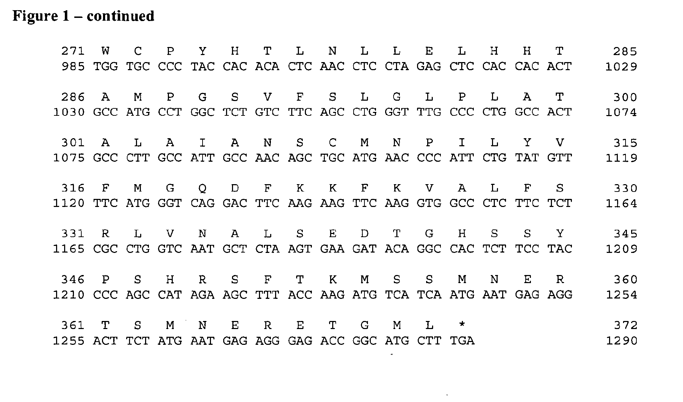

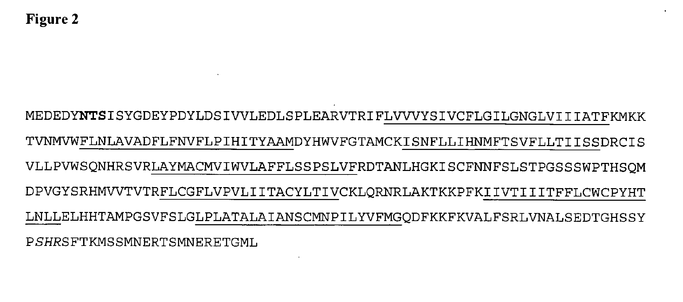

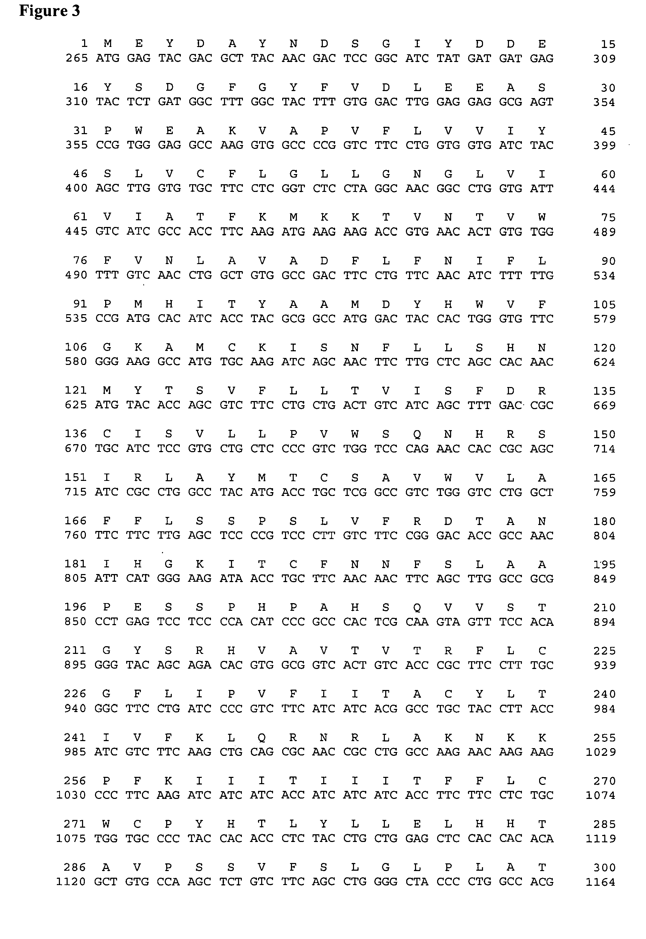

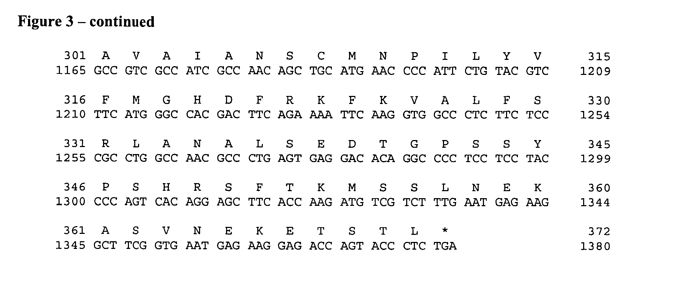

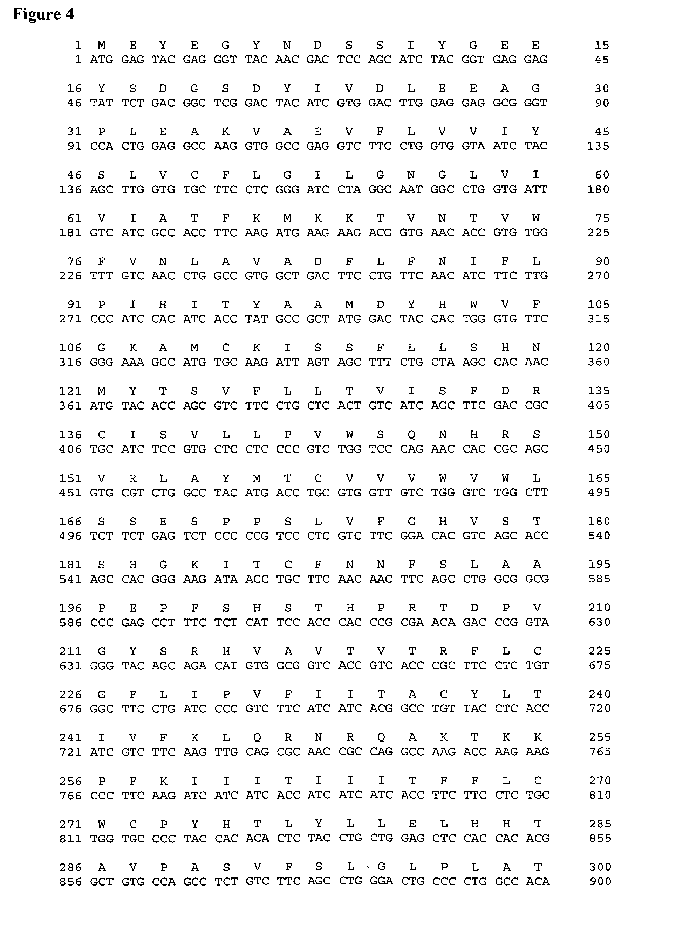

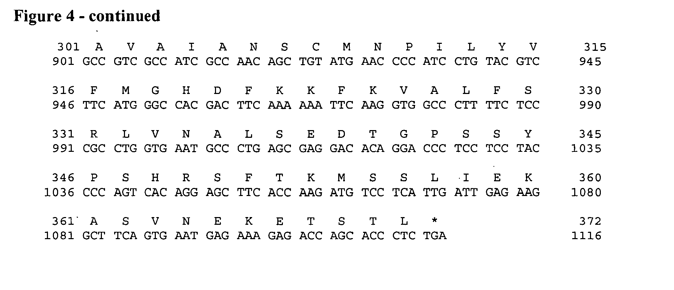

ChemR23, also called Dez [Sequence ID Nos: 1 (human polynucleotide sequence, Fig. 1); 2 (human amino acid sequence, Fig. 2); 3 (mouse polynucleotide sequence, Fig. 3); 4 (mouse amino acid sequence, Fig. 3); 5 (rat polynucleotide sequence; Fig. 4); and 6 (rat amino acid sequence, Fig. 4)] has been described as an orphan G protein coupled receptor related to GPR-1 (38% overall amino acid identity), C3a receptor (38%), C5a anaphylatoxin receptor (36%) and formyl Met-Leu-Phe receptors (35%). ChemR23 is more distantly related to the chemokine receptors subfamily (Methner A, Hermey G, Schinke B, Hermans-Borgmeyer I. (1997) *Biochem Biophys Res Commun* 233:336-42; Samson M, Edinger AL, Stordeur P, Rucker J, Verhasselt V, Sharron M, Govaerts C, Mollereau C, Vassart G, Doms RW, Parmentier M. (1998) *Eur J Immunol* 28:1689-700). ChemR23 transcripts were found to be abundant in monocyte-derived dendritic cells and macrophages, with or without treatment with LPS. Low expression can also be detected by reverse transcription-PCR in CD4+ T lymphocytes. In situ hybridization experiments also showed that the receptor was differentially regulated during development, with a prominent expression in developing osseous and cartilaginous tissues. It was also detectable in the adult parathyroid glands, indicating a possible function in phosphocalic metabolism.

The gene encoding ChemR23 was assigned by radiation hybrid mapping to the q21.2-21.3 region of human chromosome 12, outside the gene clusters identified so far for chemoattractant receptors. ChemR23 was tested in fusion assays for potential coreceptor activity by a range of HIV-1, HIV-2 and SIV viral strains. Several SIV strains (SIVmac316, SIVmac239, SIVmacl7E-Fr and SIVsm62A), as well as a primary HIV-1 strain (92UG024-2) efficiently used ChemR23 as a co-receptor. This receptor therefore appears to be a coreceptor for immunodeficiency viruses that does not belong to the chemokine receptor family. It is also a putative chemoattractant receptor and it could play an important role in the recruitment or trafficking of leukocyte cell populations.

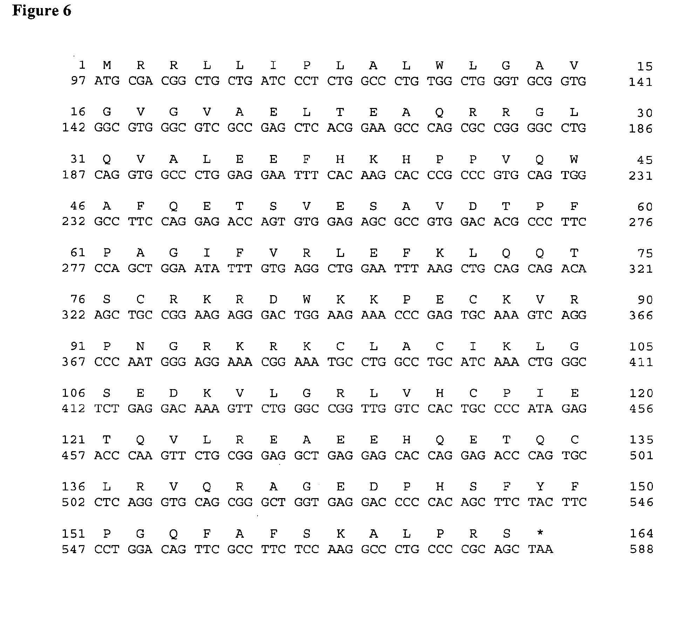

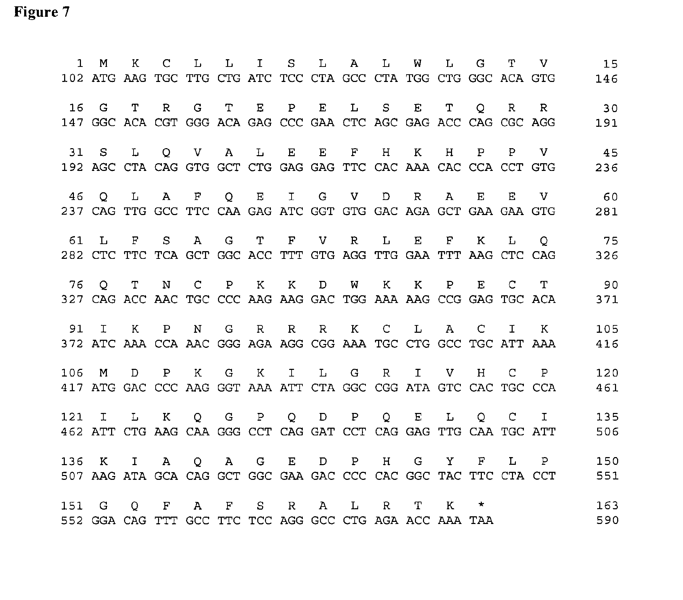

TIG2 (Tazarotene-induced gene 2 [Sequence ID Nos: 7 (human TIG2 polynucleotide sequence, Fig. 6); 8 (human amino acid sequence, Fig. 6); 9 (mouse polynucleotide sequence, Fig. 7); and 10 (mouse amino acid sequence, Fig. 7)] was identified as a cDNA, the expression of which is up-regulated by the treatment of skin raft cultures by the retinoic acid receptor (RAR) beta/gamma-selective anti-psoriatic synthetic retinoid, tazarotene [AGN 190168/ethyl 6-[2-(4,4-dimethylthiochroman-6-yl)-ethynyl] nicotinate] (Nagpal S, Patel S, Jacobe H, DiSepio D, Ghosn C, Malhotra M, Teng M, Duvic M, Chandraratna RA. (1997) *J Invest Dermatol* 109: 91-5). The retinoid-mediated up-regulation in the expression of TIG2 was confirmed by Northern blot analysis. The TIG2 cDNA is 830 bp long and encodes a putative protein product of 164 amino acids. TIG2 is expressed and induced by tazarotene in culture only when keratinocytes and fibroblasts form a tissue-like 3-dimensional structure. RAR-specific retinoids were also shown to increase TIG2 mRNA levels. In contrast, neither RXR-specific retinoids nor 1,25-dihydroxyvitamin D3 increased TIG2 levels in these cells. TIG2 is also expresssed at high levels in nonlesional psoriatic skin but at lower levels in the psoriatic lesion and its expression is up-regulated in psoriatic lesions after topical application of tazarotene. In addition, TIG2 has been shown to be dramatically upregulated by 1,25 dihydroxyvitamin D3 and dexamethasone in osteoclast-supporting stromal cells (Adams AE, Abu-Amer Y, Chappel J, Stueckle S, Ross FP, Teitelbaum SL, Suva LJ. (1999) *J Cell Biochem* 74: 587-95).

## SUMMARY OF THE INVENTION

The invention relates to the identification of TIG2, the polypeptide product of Tazarotene-Induced Gene 2, as a natural ligand of the ChemR23 GPCR (G-protein coupled receptor). The invention encompasses the use of the interaction of ChemR23 polypeptides and TIG2 polypeptides as the basis of screening assays for agents that modulate the activity of the ChemR23 receptor. The invention also encompasses diagnostic assays based upon the ChemR23/TIG2 interaction, as well as kits for performing diagnostic and screening assays.

The invention encompasses a method of identifying an agent that modulates the function of ChemR23, the method comprising: a) contacting a ChemR23 polypeptide with a TIG2 polypeptide in the presence and absence of a candidate modulator under conditions permitting the binding of the TIG2 polypeptide to the ChemR23 polypeptide; and b) measuring the binding of the ChemR23 polypeptide to the TIG2 polypeptide, wherein a decrease in binding in the presence of the candidate modulator, relative to the binding in the absence of the candidate modulator, identifies the candidate modulator as an agent that modulates the function of ChemR23.

The invention further encompasses a method of detecting the presence, in a sample, of an agent that modulates the function of ChemR23 in a sample, the method comprising a) contacting a ChemR23 polypeptide with a TIG2 polypeptide in the presence and absence of the sample under conditions permitting the binding of the TIG2 polypeptide to the ChemR23 polypeptide; and b) measuring the binding of the ChemR23 polypeptide to the TIG2 polypeptide, wherein a decrease in binding in the presence of the sample, relative to the binding in the absence of the candidate modulator, indicates the presence, in the sample of an agent that modulates the function of ChemR23.

In a preferred embodiment of either of the preceding methods, the measuring is performed using a method selected from label displacement, surface plasmon resonance, fluorescence resonance energy transfer, fluorescence quenching, and fluorescence polarization.

The invention further encompasses a method of identifying an agent that modulates the function of ChemR23, the method comprising: a) contacting a ChemR23 polypeptide with a TIG2 polypeptide in the presence and absence of a candidate modulator; and b) measuring a signaling activity of the ChemR23 polypeptide, wherein a change in the activity in the presence of the candidate modulator relative to the activity in the absence of the candidate modulator identifies the candidate modulator as an agent that modulates the function of ChemR23.

The invention further encompasses a method of identifying an agent that modulates the function of ChemR23, the method comprising: a) contacting a ChemR23 polypeptide with a candidate modulator; b) measuring a signaling activity of the ChemR23 polypeptide in the presence of the candidate modulator; and c) comparing the activity measured in the presence of the candidate modulator to the activity measured in a sample in which the ChemR23 polypeptide is contacted with a TIG2 polypeptide at its $EC_{50}$, wherein the candidate modulator is identified as an agent that modulates the function of ChemR23 when the amount of the activity measured in the presence of the candidate modulator is at least 50% of the amount induced by the TIG2 polypeptide present at its $EC_{50}$.

The invention further encompasses a method of detecting the presence, in a sample, of an agent that modulates the function of ChemR23, the method comprising: a) contacting a ChemR23 polypeptide with TIG2 polypeptide in the presence and absence of the sample; b) measuring a signaling activity of the ChemR23 polypeptide; and c) comparing the amount of the activity measured in a reaction containing ChemR23 and TIG2 polypeptides without the sample to the amount of the activity measured in a reaction containing ChemR23, TIG2 and the sample, wherein a change in the activity in the presence of the sample relative to the activity in the absence of the sample indicates the presence, in the sample, of an agent that modulates the function of ChemR23.

The invention further encompasses a method of detecting the presence, in a sample, of an agent that modulates the function of ChemR23, the method comprising: a) contacting a ChemR23 polypeptide with the sample; b) measuring a signaling activity of the ChemR23 polypeptide in the presence of the sample; and c) comparing the activity measured in the presence of the sample to the activity measured in a reaction in which the ChemR23 polypeptide is contacted with a TIG2 polypeptide present at its $EC_{50}$, wherein an agent that modulates the function of ChemR23 is detected if the amount of the activity measured in the presence of the sample is at least 50% of the amount induced by the TIG2 polypeptide present at its $EC_{50}$.

In a preferred embodiment of each of the preceding methods, the TIG2 polypeptide is detectably labeled. It is preferred that the TIG2 polypeptide is detectably labeled with a moiety selected from the group consisting of a radioisotope, a fluorophore, a quencher of fluorescence, an enzyme, an affinity tag, and an epitope tag.

In one embodiment of any of the preceding methods, the contacting is performed in or on a cell expressing the ChemR23 polypeptide.

In another embodiment of any of the preceding methods, the contacting is performed in or on synthetic liposomes (see Tajib et al., 2000, *Nature Biotechnology* 18: 649 - 654, which is incorporated herein by reference) or virus-induced budding membranes containing a ChemR23 polypeptide. (see WO0102551, 2001, incorporated herein by reference).

In another embodiment of any of the preceding methods, the method is performed using a membrane fraction from cells expressing the ChemR23 polypeptide.

In another embodiment, the agent is selected from the group consisting of a peptide, a polypeptide, an antibody or antigen-binding fragment thereof, a lipid, a carbohydrate, a nucleic acid, and a small organic molecule.

In another embodiment, the step of measuring a signaling activity of the ChemR23 polypeptide comprises detecting a change in the level of a second messenger.

In another embodiment, the step of measuring a signaling activity comprises measurement of guanine nucleotide binding or exchange, adenylate cyclase activity, cAMP, Protein Kinase C activity, phosphatidylinosotol breakdown, diacylglycerol, inositol triphosphate, intracellular calcium, arachinoid acid, MAP kinase activity, tyrosine kinase activity, or reporter gene expression.

In a preferred embodiment, the measuring a signaling activity comprises using an aequorin-based assay.

The invention further encompasses a method of modulating the activity of a ChemR23 polypeptide in a cell, the method comprising the step of delivering to the cell an agent that modulates the activity of a ChemR23 polypeptide, such that the activity of ChemR23 is modulated.

The invention further encompasses a method of diagnosing a disease or disorder characterized by dysregulation of ChemR23 signaling, the method comprising: a) contacting a tissue sample with an antibody specific for a ChemR23 polypeptide; b) detecting binding of the antibody to the tissue sample; and c) comparing the binding detected in step (b) with a standard, wherein a difference in binding relative to the standard is diagnostic of a disease or disorder characterized by dysregulation of ChemR23.

The invention further encompasses a method of diagnosing a disease or disorder characterized by dysregulation of ChemR23 signaling, the method comprising: a) contacting a tissue sample with an antibody specific for a TIG2 polypeptide; b) detecting binding of the antibody to the tissue sample; and c) comparing the binding detected in step (b) with a standard, wherein a difference in binding relative to the standard is diagnostic of a disease or disorder characterized by dysregulation of ChemR23.

The invention further encompasses a method of diagnosing a disease or disorder characterized by dysregulation of ChemR23 signaling, the method comprising: a) contacting a tissue sample with an antibody specific for a ChemR23 polypeptide and an antibody specific for a TIG2 polypeptide; b) detecting binding of the antibodies to the tissue sample; and c) comparing the binding detected in step (b) with a standard, wherein a difference in the binding of either antibody or both, relative to the standard, is diagnostic of a disease or disorder characterized by dysregulation of ChemR23.

The invention further encompasses a method of diagnosing a disease or disorder characterized by dysregulation of ChemR23 signaling, the method comprising: a) isolating nucleic acid from a tissue sample; b) amplifying a ChemR23 polynucleotide, using the nucleic acid as a template; and c) comparing the amount of amplified ChemR23 polynucleotide produced in step (b) with a standard, wherein a difference in the amount of amplified ChemR23 polynucleotide relative to the standard is diagnostic of a disease or disorder characterized by dysregulation of ChemR23. In a preferred embodiment, the step of amplifying comprises RT/PCR. In another preferred embodiment, the step of comparing the amount is performed on a microarray.

The invention further encompasses a method of diagnosing a disease or disorder characterized by dysregulation of ChemR23 signaling, the method comprising: a) isolating nucleic acid from a tissue sample; b) amplifying a ChemR23 polynucleotide, using the nucleic acid as a template; and c) comparing the sequence of the amplified ChemR23 polynucleotide produced in step (b) with a standard, wherein a difference in the sequence, relative to the standard is diagnostic of a disease or disorder characterized by dysregulation of ChemR23. In a preferred embodiment, the step of amplifying comprises RT/PCR. In another preferred embodiment, the standard is SEQ ID NO: 1. In another preferred embodiment, the step of comparing the sequence comprises minisequencing. In another preferred embodiment, the step of comparing the sequence is performed on a microarray.

The invention further encompasses a method of diagnosing a disease or disorder characterized by dysregulation of ChemR23 signaling, the method comprising: a) isolating nucleic acid from a tissue sample; b) amplifying a TIG2 polynucleotide, using the nucleic acid as a template; and c) comparing the amount of amplified TIG2 polynucleotide produced in step (b) with a standard, wherein a difference in the amount of amplified TIG2 polynucleotide relative to the standard is diagnostic of a disease or disorder characterized by dysregulation of ChemR23. In a preferred embodiment, the step of amplifying comprises RT/PCR. In another preferred embodiment, the step of comparing the amount is performed on a microarray.

The invention further encompasses a method of diagnosing a disease or disorder characterized by dysregulation of ChemR23 signaling, the method comprising: a) isolating nucleic acid from a tissue sample; b) amplifying a TIG2 polynucleotide, using the nucleic acid as a template; and c) comparing the sequence of the amplified TIG2 polynucleotide produced in step (b) with a standard, wherein a difference in the sequence, relative to the standard is diagnostic of a disease or disorder characterized by dysregulation of ChemR23. In a preferred embodiment, the step of amplifying comprises RT/PCR. In another preferred embodiment, the standard is SEQ ID NO: 7. In another preferred embodiment, the step of comparing the sequence comprises minisequencing. In another preferred embodiment, the step of comparing the sequence is performed on a microarray.

The invention further encompasses a composition comprising an isolated ChemR23 polypeptide and an isolated TIG2 polypeptide.

The invention further encompasses an antibody specific for a Chem R23 polypeptide or a TIG2 polypeptide.

The invention further encompasses a kit for screening for agents that modulate ChemR23 signaling, or for the diagnosis of a disease or disorder characterized by dysregulation of a ChemR23 polypeptide, the kit comprising an isolated ChemR23 polypeptide and packaging materials therefor. In a preferred embodiment, the kit further comprises a TIG2 polypeptide. Diagnostic kits according to the invention permit the determination of whether, for example, a tissue sample or an extract prepared from a tissue sample has an elevated level or activity of TIG2 or ChemR23. The kits also permit the identification of mutations in genes encoding ChemR23 or TIG2 and detection of abnormal levels of nucleic acids encoding ChemR23 or TIG2.

The invention further encompasses a kit for screening for agents that modulate ChemR23 signaling, or for the diagnosis of a disease or disorder characterized by dysregulation of a ChemR23 polypeptide, the kit comprising an isolated polynucleotide encoding a ChemR23 polypeptide and packaging materials therefor. In a preferred embodiment, the kit further comprises an isolated polynucleotide encoding a TIG2 polypeptide.

The invention further encompasses a kit for screening for agents that modulate ChemR23 signaling, or for the diagnosis of a disease or disorder characterized by dysregulation of a ChemR23 polypeptide, the kit comprising a cell transformed with a polynucleotide encoding a ChemR23 polypeptide and packaging materials therefor. In a preferred embodiment, the kit further comprises an isolated polynucleotide encoding a TIG2 polypeptide or a cell comprising a polynucleotide encoding a TIG2 polypeptide.

The invention further encompasses a non-human mammal having a homozygous null mutation in the gene encoding ChemR23.

The invention further encompasses a non-human mammal transgenic for a ChemR23 polynucleotide.

The invention further encompasses a non-human mammal transgenic for a TIG2 polynucleotide.

As used herein, the term "ChemR23 polypeptide" refers to a polypeptide having two essential properties: 1) a ChemR23 polypeptide has at least 70% amino acid identity, and preferably 80%, 90%, 95% or higher, including 100% amino acid identity, to SEQ ID NO: 2; and 2) a ChemR23 polypeptide has ChemR23 activity, i.e., the polypeptide binds a TIG2 polypeptide or à functional fragment thereof. Optimally, a "ChemR23 polypeptide" also has ChemR23 signaling activity as defined herein.

As used herein, the term "ChemR23 polynucleotide" refers to a polynucleotide that encodes a ChemR23 polypeptide as defined herein.

As used herein, the term "ChemR23 activity" refers to specific binding of a TIG2 polypeptide or a functional fragment thereof by a ChemR23 polypeptide.

As used herein, the term "ChemR23 signaling activity" refers to the initiation or propagation of signaling by a ChemR23 polypeptide. ChemR23 signaling activity is monitored by measuring a detectable step in a signaling cascade by assaying one or more of the following: stimulation of GDP for GTP exchange on a G protein; alteration of adenylate cyclase activity; protein kinase C modulation; phosphatidylinositol breakdown (generating second messengers diacylglycerol, and inositol triphosphate); intracellular calcium flux; activation of MAP kinases; modulation of tyrosine kinases; or modulation of gene or reporter gene activity. A detectable step in a signaling cascade is considered initiated or mediated if the measurable activity is altered by 10% or more above or below a baseline established in the substantial absence of a TIG2 polypeptide relative to any of the ChemR23 activity assays described herein below. The measurable activity can be measured directly, as in, for example, measurement of cAMP or diacylglycerol levels. Alternatively, the measurable activity can be measured indirectly, as in, for example, a reporter gene assay.

As used herein, the term "detectable step" refers to a step that can be measured, either directly, e.g., by measurement of a second messenger or detection of a modified (e.g., phosphorylated) protein, or indirectly, e.g., by monitoring a downstream effect of that step. For example, adenylate cyclase activation results in the generation of cAMP. The activity of adenylate cyclase can be measured directly, e.g., by an assay that monitors the production of cAMP in the assay, or indirectly, by measurement of actual levels of cAMP.

As used herein, the term "isolated" refers to a population of molecules, e.g., polypeptides or polynucleotides, the composition of which is less than 50% (by weight), preferably less than 40% and most preferably 2% or less, contaminating molecules of an unlike nature. When the term "isolated" is applied to a ChemR23 polypeptide, it is specifically meant to encompass a ChemR23 polypeptide that is associated with or embedded in a lipid membrane.

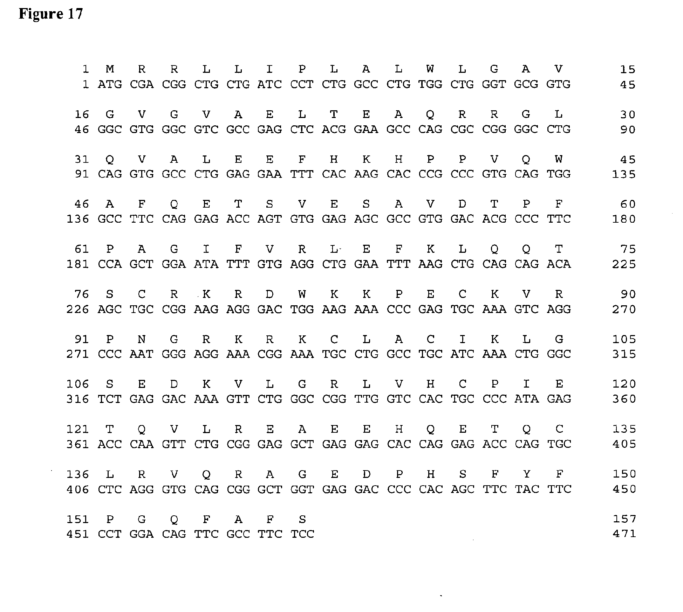

As used herein, the term "TIG2 polypeptide" refers to a polypeptide having at least 31 % identity (or higher identity, such as 45%, 55%, 65%, 75%, 85%, 95% or even 100%) to the polypeptide represented by SEQ ID NO: 8 that specifically binds to and activates a signaling activity of a ChemR23 polypeptide having the sequence of SEQ ID NO: 2. The term "specifically binds" means that the TIG2 polypeptide has an $EC_{50}$, $IC_{50}$,or a $K_d$ of 100nM or less. "TIG2 polypeptide" also refers to a fragment of a polypeptide meeting the preceding definition, wherein the fragment retains at least 50% of the binding activity and level of signaling activation of the full length polypeptide of SEQ ID NO:8. A TIG2 polypeptide can comprise additions, insertions, deletions or substitutions relative to SEQ ID NO: 8, as long as the resulting polypeptide retains at least 50% of the binding activity and level of signaling activation of the full length polypeptide represented by SEQ ID NO: 8. In one embodiment, a "TIG2 polypeptide" encompasses further the truncated TIG2 sequence of SEQ ID NO: 46 shown in Figure 17 (the nucleotide sequence shown in Figure 17, which encodes the truncated TIG2 polypeptide is SEQ ID NO: 47).

Derivatives may be similar polypeptides, fusion proteins or deletions thereof. The present invention illustrates that the truncated polypeptide as presented in SEQ ID NO:46, specifically binds to the a ChemR23 polypeptide and activates its signaling transduction pathway. Therefore, the present invention also relates to a truncated TIG2 polypeptide presented in SEQQ ID NO:46; a nucleotide sequence presented in SEQ ID NO:47 encoding said truncated TIG2 polypeptide, or derivatives thereof.

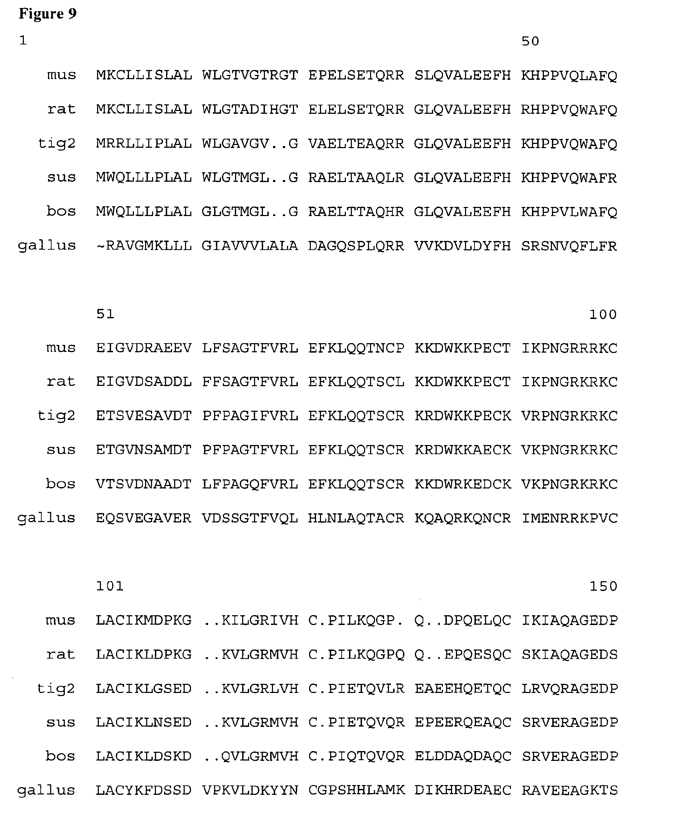

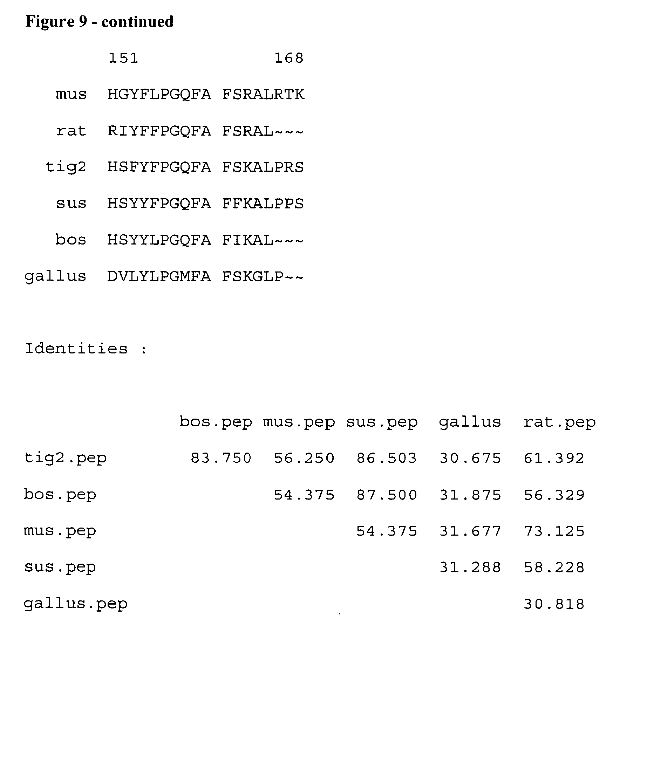

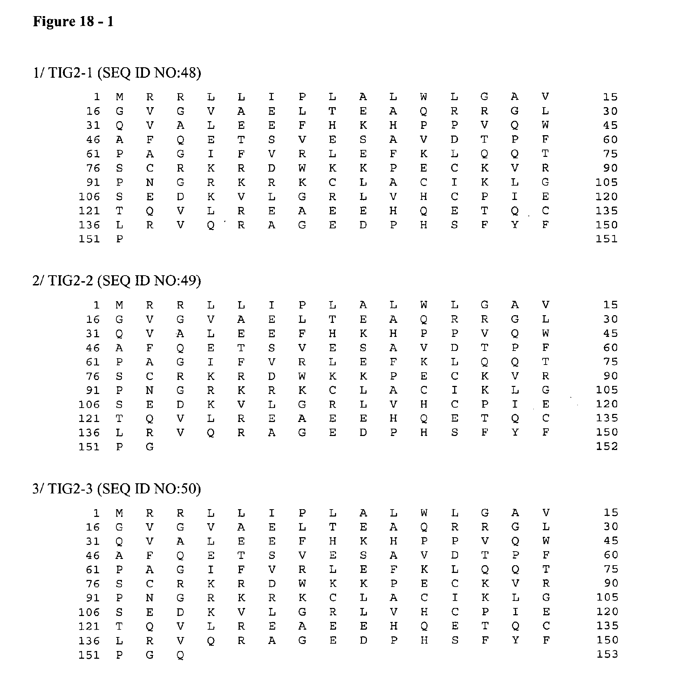

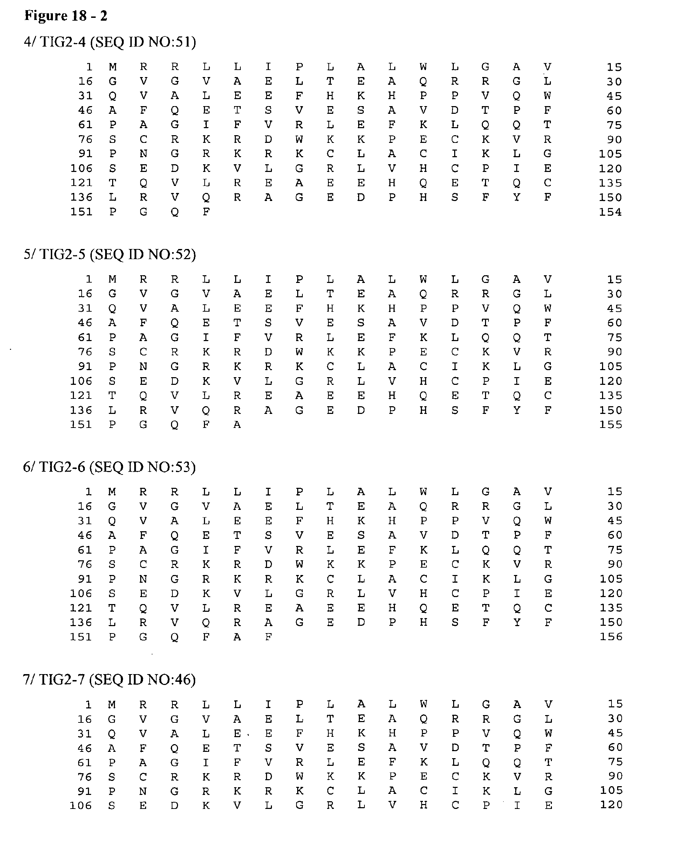

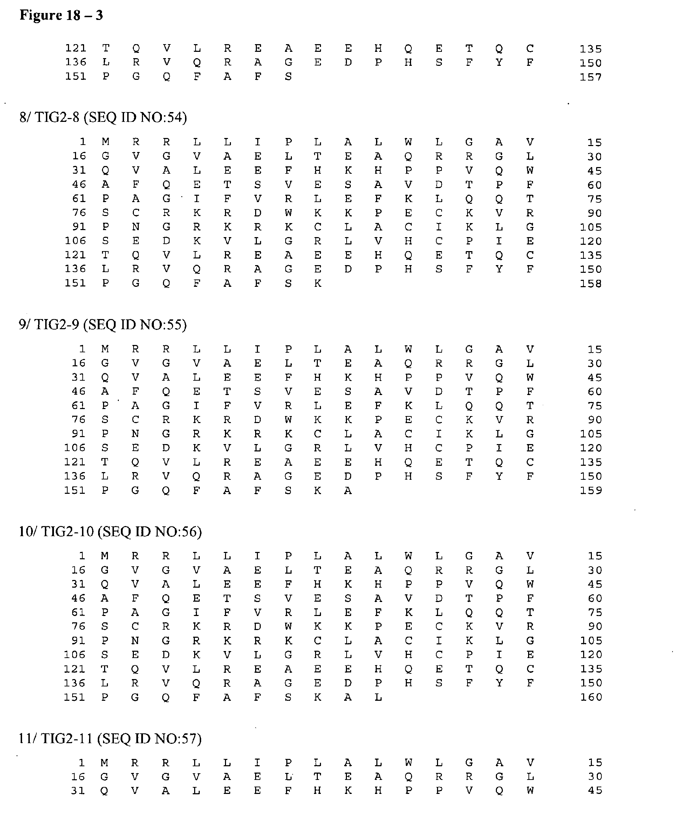

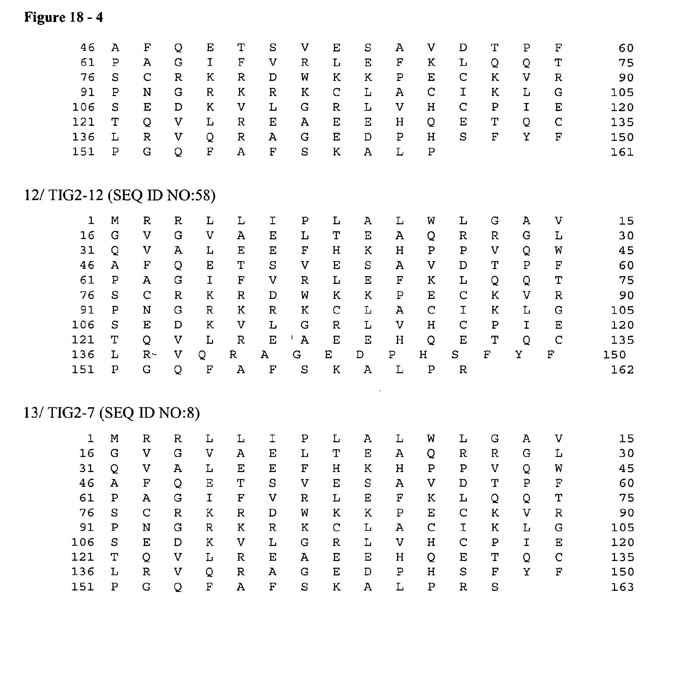

When comparing the sequence as given in SEQ ID NO:46 with the sequences as represented by SEQ ID NO: 8 or given in Figure 9 (tig2), it becomes clear that the truncation lies in the deletion of the "KALPRS"-tail. The rat sequences misses the "PRS"-tail; the gallus sequence misses the "RS"-tail, or an equivalent thereof depending which sequence is considered as reference sequence. From this comparison it becomes evident that the C-terminus of the TIG2 peptide may vary in length. The present invention further suggests that there may exist further deletions in said tail resulting in a functional peptide in respect of the ChemR23 polypeptide. Therefore, the present invention further relates to TIG2-related polypeptides represented by any of SEQ ID NO:48 to 58 as shown in Figure 18 . Insertions at position 18-19, 111-112, 122 as numbered according to Figure 9 are also possible.

In addition to the sequences necessary for binding to ChemR23 and activating a ChemR23 signaling actitity, a TIG2 polypeptide, including the truncated TIG2 polypeptide can comprise additional sequences, as in for example, a TIG2 fusion protein. Non-limiting examples of fusion partners include glutathione-S-transferase (GST), maltose binding protein, alkaline phosphatase, thioredoxin, green fluorescent protein (GFP), histidine tags (e.g., 6X or greater His), or epitope tags (e.g., Myc tag, FLAG tag).

As used herein, the term "TIG2 polynucleotide" refers to a polynucleotide that encodes a TIG2 polypeptide as defined herein, or the complement thereof. A "TIG2 polynucleotide" may be a polynucleotide sequence which encodes a truncated TIG2 polypeptide e.g., the truncated TIG2 polypeptide as shown in Figure 17 or any of the polypeptides as shown in Figure 18. The polynucleotide encoding a truncated polypeptide as represented by SEQ ID NO :46 , is shown in Figure 17 and represented by SEQ ID NO: 47. The polynucleotides encoding any of the polypeptides as shown in Figure 18 and represented by any of SEQ ID NO:48 to 58 may be deduced from the corresponding amino acid sequences by a person skilled in the art, as each amino acid may be encoded by a specific triplet (or a specific set) of nucleotides know by a person skileld in the art.

The present invention also relates to an agent identified or detected by a method as described above. In addition, the present invention relates to a composition comprising said agent.

The invention further contemplates the use of an agent or a composition according to the present invention, for the preparation of a medicament; especially for the preparation of a medicament for the treatment of a ChemR23-related disease or a ChemR23-related disorder; wherein said disease or disorder is preferentially chosen from the group consisting of Cancer, Tumor metastasis, Inflammatory diseases, Autoimmune diseases, Inherited or acquired immune deficiencies, Osteoporosis, Bone healing, Bone tissue grafts, Graft rejection, Psoriasis, Eczeme, Inflammatory infection and trophic diseases of skin, Viral, bacterial and parasitic infections, Female infertility, and, Ovary and uterus tumors; or, for the preparation of a medicament for the treatment of a TIG2-related disease or a TIG2-related disorder, wherein said disease or disorder is preferentially chosen from the group consisting of Osteoporosis, Bone healing, Bone tissue grafts, Graft rejection, Psoriasis, Eczeme, Inflammatory infection and trophic diseases of skin, Viral, bacterial and parasitic infection, Female infertility, and, Ovary and uterus tumors.

The present invention further relates to a truncated TIG2 peptide represented by SEQ ID NO:46, or represented by any of SEQ ID NO:48 to 58, a nucleotide sequence encoding said polypeptides, or derivatives thereof. It further relates to a nucleotide sequence represented by SEQ ID NO:47 encoding the truncated TIG2 polypeptide as represented by SEQ ID NO:46.

The present invention also relates to the use of a truncated TIG2 polypeptide or a polynucleotide sequence as described above, preferentially in a method according to the present invention. The full length TIG2 polypeptide or a nucleotide sequence encoding said full length polypeptide may also be used in one of the methods of the present invention.

Truncated TIG2 polypeptides according to the present invention or the full length TIG2 polypeptide may also be used for the production of a composition comprising an isolated ChemR23 polypeptide and an isolated TIG2 polypeptide. Alternatively, said truncated TIG2 polypeptides or a full length TIG2 polypeptide may be used for the production of an antibody, for the production of a kit for screening agents that modulate the signalling of ChemR23 or for the production of a kit for the diagnosis of a disease characterized by the degegulation of ChemR23 signalling.

The present invention also relates to the use of a truncated TIG2 polypeptide as described above, or a full length TIG2 polypeptide, as ligand for ChemR23.

In addition the present invention further relates to the use of a polynucleotide sequence coding for said truncated TIG2 polypeptide as described above , or a polynucleotide sequence coding for a full length TIG2 polypeptide for the production of a ligand for ChemR23.

The invention also relates to the use of a truncated TIG2 polypeptide according to the present inventionor a full length TIG2 polypeptide for the production of a medicament. Said medicament may be applied for for the treatment of a ChemR23-related disease or a ChemR23-related disorder; wherein said disease or disorder is preferentially chosen from the group consisting of Cancer, Tumor metastasis, Inflammatory diseases, Autoimmune diseases, Inherited or acquired immune deficiencies, Osteoporosis, Bone healing, Bone tissue grafts, Graft rejection, Psoriasis, Eczeme, Inflammatory infection and trophic diseases of skin, Viral, bacterial and parasitic infections, Female infertility, and, Ovary and uterus tumors; or, may be applied for the treatment of a TIG2-related disease or a TIG2-related disorder, wherein said disease or disorder is preferentially chosen from the group consisting of Osteoporosis, Bone healing, Bone tissue grafts, Graft rejection, Psoriasis, Eczeme, Inflammatory infection and trophic diseases of skin, Viral, bacterial and parasitic infection, Female infertility, and, Ovary and uterus tumors.

As used herein, the terms "candidate compound" and "candidate modulator" refer to a composition being evaluated for the ability to modulate ligand binding to a ChemR23 polypeptide or the ability to modulate an activity of a ChemR23 polypeptide. Candidate modulators can be natural or synthetic compounds, including, for example, small molecules, compounds contained in extracts of animal, plant, bacterial or fungal cells, as well as conditioned medium from such cells.

As used herein, the term "small molecule" refers to a compound having molecular mass of less than 3000 Daltons, preferably less than 2000 or 1500, still more preferably less than 1000, and most preferably less than 600 Daltons. A "small organic molecule" is a small molecule that comprises carbon.

As used herein, the term "change in binding" or "change in activity" and the equivalent terms "difference in binding" or "difference in activity" refer to an at least 10% increase or decrease in binding, or signaling activity in a given assay.

As used herein, the term "conditions permitting the binding of TIG2 to ChemR23" refers to conditions of, for example, temperature, salt concentration, pH and protein concentration under which TIG2 binds ChemR23. Exact binding conditions will vary depending upon the nature of the assay, for example, whether the assay uses viable cells or only membrane fraction of cells. However, because ChemR23 is a cell surface protein, and because TIG2 is a secreted polypeptide that interacts with ChemR23 on the cell surface, favored conditions will generally include physiological salt (90 mM) and pH (about 7.0 to 8.0). Temperatures for binding can vary from 15°C to 37°C, but will preferably be between room temperature and about 30°C. The concentration of TIG2 and ChemR23 polypeptide in a binding reaction will also vary, but will preferably be about 0.1 pM(e.g., in a reaction with radiolabeled tracer TIG2, where the concentration is generally below the $K_d$) to 1 μM (e.g., TIG2 as competitor). As an example, for a binding assay using ChemR23-expressing cells and purified, recombinant, labeled TIG2 polypeptide, binding is performed using 0.1 nM labeled TIG2, 100 nM cold TIG2, and 25,000 cells at 27°C in 250 μl of a binding buffer consisting of 50 mM HEPES (pH 7.4), 1 mM $CaCl_2$, and 0.5% Fatty acid free BSA.

As used herein, the term "sample" refers to the source of molecules being tested for the presence of an agent that modulates binding to or signaling activity of a ChemR23 polypeptide. A sample can be an environmental sample, a natural extract of animal, plant yeast or bacterial cells or tissues, a clinical sample, a synthetic sample, or a conditioned medium from recombinant cells or a fermentation processe. The term "tissue sample" refers to a tissue that is tested for the presence, abundance, quality or an activity of a ChemR23 polypeptide, a TIG2 polypeptide, a nucleic acid encoding a ChemR23 or TIG2 polypeptide, or an agent that modifies the ligand binding or activity of a ChemR23 polypeptide.

As used herein, a "tissue" is an aggregate of cells that perform a particular function in an organism. The term "tissue" as used herein refers to cellular material from a particular physiological region. The cells in a particular tissue can comprise several different cell types. A non-limiting example of this would be brain tissue that further comprises neurons and glial cells, as well as capillary endothelial cells and blood cells, all contained in a given tissue section or sample. In addition to solid tissues, the term "tissue" is also intended to encompass non-solid tissues, such as blood.

As used herein, the term "membrane fraction" refers to a preparation of cellular lipid membranes comprising a ChemR23 polypeptide. As the term is used herein, a "membrane fraction" is distinct from a cellular homogenate, in that at least a portion (i.e., at least 10%, and preferably more) of non-membrane-associated cellular constituents has been removed. The term "membrane associated" refers to those cellular constituents that are either integrated into a lipid membrane or are physically associated with a component that is integrated into a lipid membrane.

As used herein, the term "decrease in binding" refers to a decrease of at least 10% in the binding of a TIG2 polypeptide or other agonist to a ChemR23 polypeptide as measured in a binding assay as described herein.

As used herein, the term "second messenger" refers to a molecule, generated or caused to vary in concentration by the activation of a G-Protein Coupled Receptor, that participates in the transduction of a signal from that GPCR. Non-limiting examples of second messengers include cAMP, diacylglycerol, inositol triphosphates and intracellular calcium. The term "change in the level of a second messenger" refers to an increase or decrease of at least 10% in the detected level of a given second messenger relative to the amount detected in an assay performed in the absence of a candidate modulator.

As used herein, the term "aequorin-based assay" refers to an assay for GPCr activity that measures intracellular calcium flux induced by activated GPCRs, wherein intracellular calcium flux is measured by the luminescence of aequorin expressed in the cell.

As used herein, the term "binding" refers to the physical association of a ligand (e.g., a TIG2 polypeptide) with a receptor (e.g., ChemR23). As the term is used herein, binding is "specific" if it occurs with an $EC_{50}$ or a $K_d$ of 100 nM or less, generally in the range of 100 nM to 10 pM. For example, binding is specific if the $EC_{50}$ or $K_d$ is 100nM, 50nM, 10 nM, 1 nM, 950 pM, 900 pM, 850 pM, 800 pM, 750 pM, 700 pM, 650 pM, 600 pM, 550 pM, 500 pM, 450 pM, 400 pM, 350 pM, 300 pM, 250 pM, 200 pM, 150 pM, 100 pM, 75 pM, 50 pM, 25 pM or 10 pM or less.

As used herein, the term "$EC_{50}$," refers to that concentration of an agent at which a given activity, including binding of a TIG2 polypeptide or other ligand and a functional activity of a ChemR23 polypeptide, is 50% of the maximum for that ChemR23 activity measurable using the same assay. Stated differently, the "$EC_{50}$" is the concentration of agent that gives 50% activation, when 100% activation is set at the amount of activity that does not increase with the addition of more agonist. It should be noted that the "$EC_{50}$ of a TIG2 polypeptide" will vary with the identity of the TIG2 polypeptide; for example, variant TIG2 polypeptides (i.e., those containing insertions, deletions, substitutions or fusions with other polypeptides, including TIG2 molecules from species other than humans and variants of them that satisfy the definition of TIG2 polypeptide set forth above) can have $EC_{50}$ values higher than, lower than or the same as wild-type TIG2. Therefore, where a TIG2 variant sequence differs from wild-type TIG2 of SEQ ID NO:8, one of the skill in the art can determine the $EC_{50}$ for that variant according to conventional methods. The $EC_{50}$ of a given TIG2 polypeptide is measured by performing an assay for an activity of a fixed amount of ChemR23 polypeptide in the presence of doses of the TIG2 polypeptide that increase at least until the ChemR23 response is saturated or maximal, and then plotting the measured ChemR23 activity versus the concentration of TIG2 polypeptide.

As used herein, the term "$IC_{50}$" is the concentration of an antagonist or inverse agonist that reduces the maximal activation of a ChemR23 receptor by 50%.

As used herein, the term "detectably labeled" refers to the property of a molecule, e.g., a TIG2 polypeptide or other ChemR23 ligand, that has a structural modification that incorporates a functional group (label) that can be readily detected. Detectable labels include but are not limited to fluorescent compounds, isotopic compounds, chemiluminescent compounds, quantum dot labels, biotin, enzymes, electron-dense reagents, and haptens or proteins for which antisera or monoclonal antibodies are available. The various means of detection include but are not limited to spectroscopic, photochemical, radiochemical, biochemical, immunochemical, or chemical means.

As used herein, the term "affinity tag" refers to a label, attached to a molecule of interest (e.g., a TIG2 polypeptide or other ChemR23 ligand), that confers upon the labeled molecule the ability to be specifically bound by a reagent that binds the label. Affinity tags include, but are not limited to an epitope for an antibody (known as "epitope tags"), biotin, 6X His, and GST. Affinity tags can be used for the detection, as well as for the purification of the labeled species.

As used herein, the term "decrease in binding" refers to a decrease of at least 10% in the amount of binding detected in a given assay with a known or suspected modulator of ChemR23 relative to binding detected in an assay lacking that known or suspected modulator.

As used herein, the term "delivering," when used in reference to a drug or agent, means the addition of the drug or agent to an assay mixture, or to a cell in culture. The term also refers to the administration of the drug or agent to an animal. Such administration can be, for example, by injection (in a suitable carrier, e.g., sterile saline or water) or by inhalation, or by an oral, transdermal, rectal, vaginal, or other common route of drug administration.

As used herein, the term "effective amount" refers to that amount of a drug or ChemR23 modulating agent that results in a change in a ChemR23 activity as defined herein (i.e., at least 10% increase or decrease in a ChemR23 activity).

As used herein, the term "standard" refers to a sample taken from an individual who is not affected by a disease or disorder characterized by dysregulation of ChemR23 or TIG2 activity. The "standard" is used as a reference for the comparison of ChemR23 or TIG2 polypeptide or mRNA levels and quality (i.e., mutant vs. wild-type), as well as for the comparison of ChemR23 activities.

As used herein, the term "amplifying," when applied to a nucleic acid sequence, refers to a process whereby one or more copies of a nucleic acid sequence is generated from a template nucleic acid. A preferred method of "amplifying" is PCR or RT/PCR.

As used herein, the term "substantial absence" refers to a level of an activating or inhibiting factor that is below the level necessary to activate or inhibit GPCR function by at least 10% as measured by a given assay disclosed herein or known in the art.

As used herein, the term "G-Protein coupled receptor," or "GPCR" refers to a membrane-associated polypeptide with 7 alpha helical transmembrane domains. Functional GPCR's associate with a ligand or agonist and also associate with and activate G-proteins. ChemR23 is a GPCR.

As used herein, the term "agent that modulates the function of a ChemR23 polypeptide" is a molecule or compound that increases or decreases ChemR23 activity, including compounds that change the binding of TIG2 polypeptides or other agonists, and compounds that change ChemR23 downstream signaling activities.

As used herein, the term "transgenic animal" refers to any animal, preferably a non-human mammal, bird, fish or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extra-chromosomally replicating DNA. In the typical transgenic animals described herein, the transgene causes cells to express a recombinant form of one of the subject polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques.

As used herein, the term "antibody" is the conventional immunoglobulin molecule, as well as fragments thereof which are also specifically reactive with one of the subject polypeptides. Antibodies can be fragmented using conventional techniques and the fragments screened for utility in the same manner as described herein below for whole antibodies. For example, $F(ab)_2$ fragments can be generated by treating antibody with pepsin. The resulting $F(ab)_2$ fragment can be treated to reduce disulfide bridges to produce Fab fragments. The antibody of the present invention is further intended to include bispecific, single-chain, and chimeric and humanized molecules having affinity for a polypeptide conferred by at least one CDR region of the antibody. In preferred embodiments, the antibodies, the antibody further comprises a label attached thereto and able to be detected, (e.g., the label can be a radioisotope, fluorescent compound, chemiluminescent compound, enzyme, or enzyme co-factor).

As used herein, the term "null mutation" refers to an insertion, deletion, or substitution that modifies the chromosomal sequences encoding a polypeptide, such that the polypeptide is not expressed.

BRIEF DESCRIPTION OF THE FIGURES

Figure 1 shows the nucleotide (SEQ ID NO: 1) and deduced amino acid sequence of human chemR23/Dezb/CMKRL1 (AC075748).

Figure 2 shows the amino acid sequence of human ChemR23/Dezb/CMKRL1 (371 amino acids) (SEQ ID NO: 2). The seven predicted transmembrane domains are underlined. The consensus sequence for N-linked glycosylation (N-X-S/T) in the N terminus is bold, and the potential site of phosphorylation by PKC (S/T-X-R/K) in the C terminus is italicized.

Figure 3 shows the nucleotide (SEQ ID NO:3) and deduced amino acid (SEQ ID NO: 4) sequences of mouse Dez, the mouse orthologue of ChemR23 (AC u79525).

Figure 4 shows the nucleotide (SEQ ID NO: 5) and deduced amino acid (SEQ ID NO: 6) sequences of rat G-Protein -Coupled Chemoattractant-1, the rat orthologue of ChemR23/Dezb/CMKRL1 (AC NM_022218).

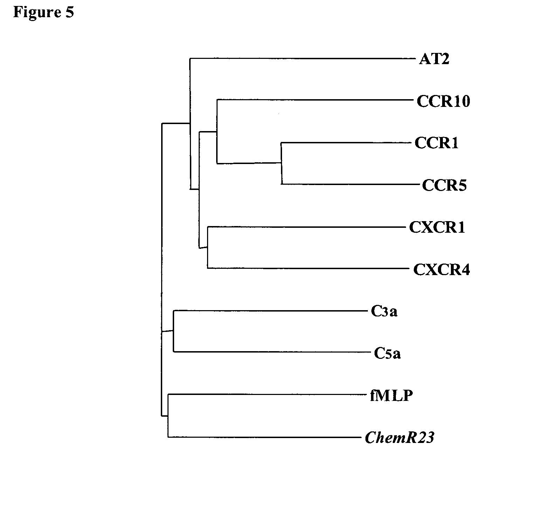

Figure 5 shows the structural similarities between the amino acid sequences of ChemR23/ Dezb/CMKRL1 and the sequences of AT2, C3a, c5a, and fMLP receptors and selected chemokine receptor sequences performed using the ClustalX algorithm. The dendrogram shown was constructed using the TreeView Algorithm.

Figure 6 shows the nucleotide (SEQ ID NO: 7) and deduced amino acid (SEQ ID NO: 8) sequences of human TIG2 (AC Q99969).

Figure 7 shows the nucleotide (SEQ ID NO: 9) and deduced amino acid (SEQ ID NO: 10) sequences of mouse TIG2.

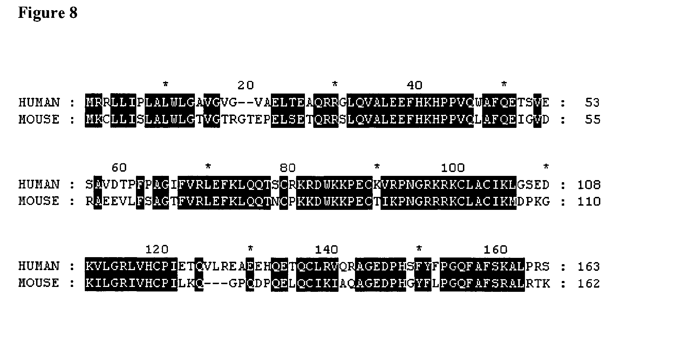

Figure 8 shows an alignment of the human (SEQ ID NO:8) and mouse (SEQ ID NO:10) TIG2 amino acid sequences. Identical amino acids and conservative substitutions are boxed.

Figure 9 shows an alignment of human («tig2»; SEQ ID NO:8), rat (SEQ ID NO:31), mouse (SEQ ID NO:10), sus (*Sus Scrofa*) (SEQ ID NO:32), bos (*Bos taurus*) (SEQ ID NO:33), and gallus (*Gallus gallusi*) (SEQ ID NO:34) TIG2 sequences. The figure provides the percent amino acid identity across any two species listed.

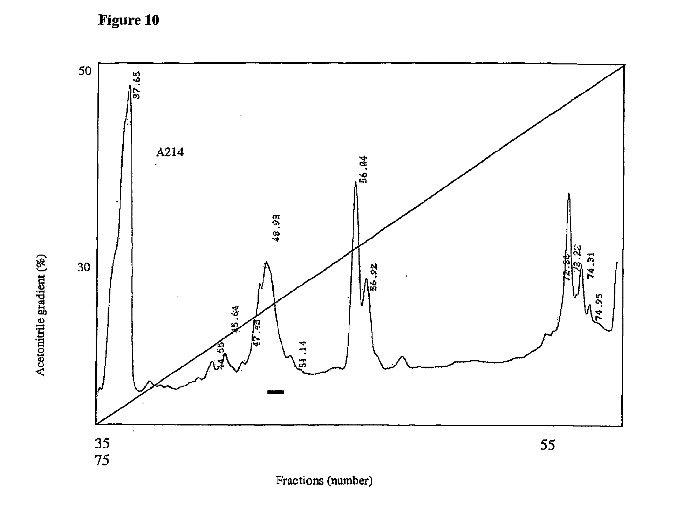

Figure 10 shows a partial chromatogram of the fifth step of purification of TIG2 from ascitic fluid. The active fractions (eluted with approximately 28% $CH_3CN$) of the previous step were diluted 6 fold with 0.1% TFA in $H_2O$ and directly loaded onto a C18 reverse phase column (1mm x 50 mm, Vydac) pre-equilabrated with 5% $CH_3CN$/0.1% TFA in $H_2O$ at a flow-rate of 0.1 ml/min. at room temperature. A 5-95% gradient of $CH_3CN$ in 0.1%TFA was applied with a 0.3%/min slope between 25 and 45%. The activity was eluted at 40% $CH_3CN$ (indicated by the black horizontal line).

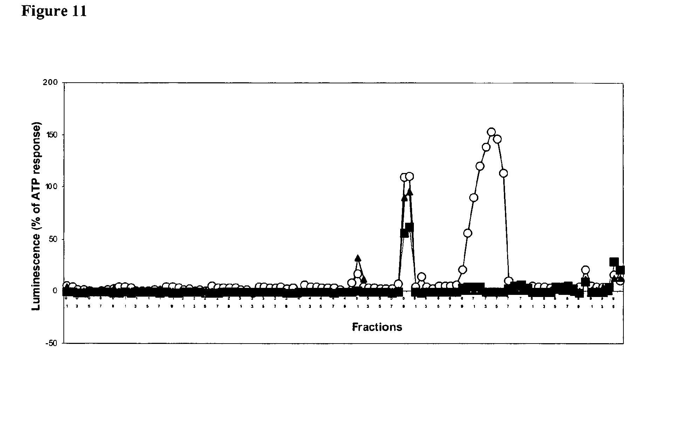

Figure 11 shows the identification of a specific response for ChemR23 following screening of HPLC fractions obtained from the fractionation of human ovary ascites. The different fractions obtained following fractionation of human ovary ascites were diluted fivefold in the assay buffer and tested in an aequorin assay using a cell line expressing ChemR23 (open circles) or cell lines expressing unrelated receptors (closed triangles and squares). The response obtained for each fraction was normalized using the ATP response of each cell line.

Figure 12 shows the activation of ChemR23 by conditioned medium of 293T cells transiently transfected with TIG2. 293T cells were transiently transfected with pcDNA3-TIG2 or with pcDNA3 alone (mock transfected). Increasing volumes of the supernatant collected 4 days after transfection were analyzed using a Microlumat in an aequorin-based assay with CHO cells expressing ChemR23. The assay was performed in triplicate, and SD is indicated. A representative experiment is shown.

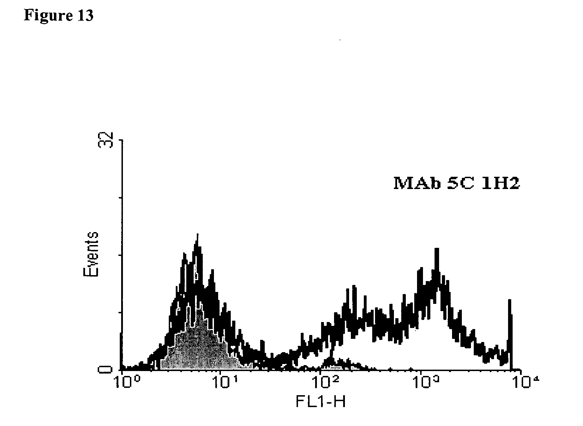

Figure 13 shows the characterization of antibodies directed against ChemR23 by flow cytometry. A mixture of recombinant cells made up of 2/3 recombinant ChemR23 CHO cells and 1/3 recombinant HCR CHO cells (negative control) was subject to react with either a supernatant of the anti ChemR23 5C 1H2 monoclonal antibody (thick line) or a supernatant with no known antibody activity (thin line, grey filling). After staining with FITC labeled anti mouse Ig these preparations were analysed by flow cytofluorometry. Results are displayed as a histogram of the number of cells (Events axis) expressing a given fluorescence (FL1-H axis). Monoclonal 5C 1H2 allowed to discriminate the ChemR23 recombinant sub-population of cells from the negative control cells as evidenced by the relative proportions of both type of cells. The background fluorescence of the assay is given by the second staining (grey filling).

Figure 14 shows the $EC_{50}$ for activation of ChemR23 by the truncated hTIG2 polypeptide determined as explained in Example 11.

Figure 15 shows the tissue distribution of hTIG2 mRNA determined as explained in Example 10.

Figure 16 shows the tissue distribution of ChemR23 mRNA determined as explained in Example 10. DC stands for dendritic cells.

Figure 17 shows the polypeptide (SEQ ID NO: 46) and polynucleotide (SEQ ID NO: 47) of the truncated human TIG2.

Figure 18 shows the polypeptide sequences of other truncated human TIG2 polypeptides (SEQ ID NO 48 to 58) compared to TIG2 of SEQ ID NO:46 and full length TIG2 (SEQ ID NO:8).

DETAILED DESCRIPTION OF THE INVENTION

The invention relates to the discovery that TIG2 polypeptide is a natural ligand for the orphan ChemR23 GPCR. The interaction is useful for screening assays for agents that modulate the interaction and thus the function of ChemR23. The known ligand and its interaction with the receptor also provides for the diagnosis of conditions involving dysregulated receptor activity.

1. Assays For The Identification Of Agents That Modulate The Activity Of ChemR23

Agents that modulate the activity of ChemR23 can be identified in a number of ways that take advantage of the interaction of the receptor with TIG2. For example, the ability to reconstitute ChemR23/TIG2 binding either in vitro, on cultured cells or in vivo provides a target for the identification of agents that disrupt that binding. Assays based on disruption of binding can identify agents, such as small organic molecules, from libraries or collections of such molecules. Alternatively, such assays can identify agents in samples or extracts from natural sources, e.g., plant, fungal or bacterial extracts or even in human tissue samples (e.g., tumor tissue). In one aspect, the extracts can be made from cells expressing a library of variant nucleic acids, peptides or polypeptides, including, for example, variants of TIG2 polypeptide itself. Modulators of ChemR23/TIG2 binding can then be screened using a binding assay or a functional assay that measures downstream signaling through the receptor. Both binding assays and functional assays are validated using TIG2 polypeptide.

Another approach that uses the ChemR23/TIG2 interaction more directly to identify agents that modulate ChemR23 function measures changes in ChemR23 downstream signaling induced by candidate agents or candidate modulators. These functional assays can be performed in isolated cell membrane fractions or on cells expressing the receptor on their surfaces.

The following description provides methods for both binding and functional assays based upon the interaction of ChemR23 and TIG2.

A. ChemR23 polypeptides.

Assays using the interaction of ChemR23 and TIG2 require a source of ChemR23 polypeptide. The polynucleotide and polypeptide sequence of human ChemR23 are presented herein as SEQ ID NOs: 1 and 2. The human ChemR23 polynucleotide sequence is also available at GenBank Accession No. Y14838, and was reported in Samson et al., 1998, Eur. J. Immunol. 28: 1689-1700, incorporated herein by reference. ChemR23 polypeptide sequence is also recorded at accession Nos. 075748 and CAA75112 in the Swissprot database. Related sequences include those for CMKRL1 (GenBank Accession Nos. XM_006864 and NM004072 (nucleotide sequences) and Swissprot Accession No. Q99788 (polypeptide sequence)), human DEZb (GenBank Accession No. U79527 (nucleotide sequence)), human DEZa (GenBank Accession No. U79526 (nucleotide sequence), mouse DEZ (GenBank Accession No. U79525 (nucleotide sequence) and Swissprot Accession No. P97468 (polypeptide sequence)), and rat ChemR23 (GenBank Accession No. AJ002745 (nucleotide sequence) and Swissprot Accession No. 035786 (polypeptide sequence).

One skilled in the art can readily amplify a ChemR23 sequence from a sample containing mRNA encoding the protein through basic PCR and molecular cloning techniques using primers or probes designed from the known sequences.

The expression of recombinant polypeptides is well known in the art. Those skilled in the art can readily select vectors and expression control sequences for the expression of ChemR23 polypeptides useful according to the invention in eukaryotic or prokaryotic cells. ChemR23 must be associated with cell membrane or detergents like synthetic liosomes in order to have binding or signaling function. Methods for the preparation of cellular membrane fractions are well known in the art, e.g., the method reported by Hubbard & Cohn, 1975, J. Cell Biol. 64: 461-479, which is incorporated herein by reference. In order to produce membranes comprising ChemR23, one need only apply such techniques to cells endogenously or recombinantly expressing ChemR23. Alternatively, membrane-free ChemR23 can be integrated into membrane preparations by dilution of detergent solution of the polypeptide (see, e.g., Salamon et al., 1996, Biophys. J. 71:283-294, which is incorporated herein by reference).

B. TIG2 polypeptides.

Human TIG2 polynucleotide and polypeptide sequences are presented herein as SEQ ID Nos 7 and 8, respectively. TIG2 sequences are also available from GenBank (e.g., Human polynucleotide sequences include Accession Nos. XM 004765, U77594, NM 002889, human polypeptide sequence is available at Accession Nos. Q99969, BAA76499, AAB47975, NP002880, and XP004765; Gallus gallus polynucleotide sequences include Accession Nos. BG713704, BG713660 and BG713614; mouse polynucleotide sequences include BF020273, AW113641 and bf018000; rat polynucleotide sequences include AW915104; Sus scrofa polynucleotide sequences include BF078978 and BF713092 (overlapping ESTs, last 7 amino acids of TIG2 sequence in BF713092); and Bos taurus polynucleotide sequences include BG691132). An alignment of TIG2 sequences is presented in Figure 9.

As with ChemR23, TIG2 polynucleotides can be cloned through standard PCR and molecular cloning techniques using the known sequences as a source of amplification primers or probes. Similarly, cloned TIG2 polypeptides can be expressed in eukaryotic or prokaryotic cells as known in the art. As a non-limiting example, a mammalian TIG2 expression vector system can comprise a bicistronic expression vector containing the promoter of human EF1$\alpha$ (described by Mishizuma & Nagata, 1990, Nucl. Acids Res. 18: 5322), a polylinker, the ECMV internal ribosome entry site (IRES, described by Ghattas et al., 1991, Mol. Cell. Biol. 11: 5848-5859) and the neomycin resistance gene followed by an SV40 polyA signal. A TIG2 expression construct for expression in yeast is described in Example 4.

TIG2 can also be expressed in vitro through in vitro transcription and translation. Further, if desired for a given assay or technique, TIG2 polypeptides useful according to the invention can be produced as fusion proteins or tagged proteins. For example, either full length TIG2 or a portion thereof (i.e., at least 10 amino acids, preferably at least 20 amino acids or more, up to one amino acid less than full length TIG2) can be fused to Glutathione-S-Transferase (GST), secreted alkaline phosphatase (SEAP), a FLAG tag, a Myc tag, or a 6X-His peptide to facilitate the purification or detection of the TIG2 polypeptide. Methods and vectors for the production of tagged or fusion proteins are well known in the art, as are methods of isolating and detecting such fused or tagged proteins.

TIG2 polypeptides and particularly truncated forms can also be prepared by chemical synthesis as known in the art.

Recombinant TIG2 polypeptides can be used in purified form. Alternatively, conditioned medium from TIG2 transfected cells can be used. The amounts of TIG2 necessary in a given binding or functional assay according to the invention will vary depending upon the assay, but will generally use 1 pM to 1 nM of labeled and 10 pM to 1$\mu$M of unlabeled TIG2 per assay. The affinities and $EC_{50}$s of tagged TIG2 polypeptides for ChemR23 may vary relative to those of full length wild type TIG2 polypeptide, and the amount necessary for a given assay can therefore be adjusted relative to the wild-type values. If necessary for a given assay, TIG2 can be labeled by incorporation of radiolabeled amino acids in the medium during synthesis, e.g., $^{35}$S-Met, $^{14}$C-Leu, or others as appropriate. Methods of chemical labeling with $^{125}$I are known in the art. Fluorescent labels can also be attached to TIG2 polypeptides or to other ChemR23 ligands using standard labeling techniques.

C. <u>Assays to Identify Modulators of ChemR23 Activity</u>

The discovery that TIG2 is a ligand of the ChemR23 receptor permits screening assays to identify agonists, antagonists and inverse agonists of receptor activity. The screening assays will have two general approaches.

1) Ligand binding assays, in which cells expressing ChemR23, membrane extracts from such cells, or immobilized lipid membranes comprising ChemR23 are exposed to a labeled TIG2 polypeptide and candidate compound. Following incubation, the reaction mixture is measured for specific binding of the labeled TIG2 polypeptide to the ChemR23 receptor. Compounds that interfere with or displace labeled TIG2 polypeptide can be agonists, antagonists or inverse agonists of ChemR23 activity. Functional analysis can be performed on positive compounds to determine which of these categories they fit.

2) Functional assays, in which a signaling activity of ChemR23 is measured.

a) For agonist screening, cells expressing ChemR23 or membranes prepared from them are incubated with candidate compound, and a signaling activity of ChemR23 is measured. The assays are validated using a TIG2 polypeptide as agonist, and the activity induced by compounds that modulate receptor activity is compared to that induced by TIG2. An agonist or partial agonist will have a maximal biological activity corresponding to at least 10% of the maximal activity of wild type human TIG2 when the agonist or partial agonist is present at 10 ☒ M or less, and preferably will have 50%, 75%, 100% or more, including 2-fold, 5-fold, 10-fold or more activity than wild-type human TIG2.

b) For antagonist or inverse agonist screening, cells expressing ChemR23 or membranes isolated from them are assayed for signaling activity in the presence of a TIG2 polypeptide with or without a candidate compound. Antagonists or inverse agonists will reduce the level of TIG2-stimulated receptor activity by at least 10%, relative to reactions lacking the antagonist or inverse agonist.

c) For inverse agonist screening, cells expressing constitutive ChemR23 activity or membranes isolated from them are used in a functional assay that measures an activity of the receptor in the presence and absence of a candidate compound. Inverse agonists are those compounds that reduce the constitutive activity of the receptor by at least 10%. Overexpression of ChemR23 (i.e., expression of 5-fold or higher excess of ChemR23 polypeptide relative to the level naturally expressed in macro phages in vivo) may lead to constitutive activation. Chem R23 can be overexpressed by placing it under the control of a strong constitutive promoter, e.g., the CMV early promoter. Alternatively, certain mutations of conserved GPCR amino acids or amino acid domains tend to lead to constitutive activity. See for example: Kjelsberg et al., 1992, J. Biol. Chem. 267:1430; McWhinney et al., 2000. J. Biol. Chem. 275:2087; Ren et al., 1993, J. Biol. Chem. 268:16483; Samama et al., 1993, J.Biol.Chem 268:4625; Parma et al., 1993, Nature 365:649; Parma et al., 1998, J. Pharmacol. Exp. Ther. 286:85; and Parent et al., 1996, J. Biol. Chem. 271:7949.

Ligand binding and displacement assays:

One can use ChemR23 polypeptides expressed on a cell, or isolated membranes containing receptor polypeptides, along with a TIG2 polypeptide in order to screen for compounds that inhibit the binding of TIG2 to ChemR23. When identified in an assay that measures binding or TIG2 polypeptide displacement alone, compounds will have to be subjected to functional testing to determine whether they act as agonists, antagonists or inverse agonists.

For displacement experiments, cells expressing a ChemR23 polypeptide (generally 25,000 cells per assay or 1 to 100 μg of membrane extracts) are incubated in binding buffer (e.g., 50 mM Hepes pH 7.4; 1 mM $CaCl_2$; 0.5% Bovine Serum Albumin (BSA) Fatty Acid-Free; and 0,5 mM $MgCl_2$) for 1.5 hrs (at, for example, 27°C) with labeled TIG2 polypeptide in the presence or absence of increasing concentrations of a candidate modulator. To validate and calibrate the assay, control competition reactions using increasing concentrations of unlabeled TIG2 polypeptide can be performed. After incubation, cells are washed extensively, and bound, labeled TIG2 is measured as appropriate for the given label (e.g., scintillation counting, enzyme assay, fluorescence, etc.). A decrease of at least 10% in the amount of labeled TIG2 polypeptide bound in the presence of candidate modulator indicates displacement of binding by the candidate modulator. Candidate modulators are considered to bind specifically in this or other assays described herein if they displace 50% of labeled TIG2 (sub-saturating TIG2 dose) at a concentration of 10 μM or less (i.e., $EC_{50}$ is 10 μM or less).

Alternatively, binding or displacement of binding can be monitored by surface plasmon resonance (SPR). Surface plasmon resonance assays can be used as a quantitative method to measure binding between two molecules by the change in mass near an immobilized sensor caused by the binding or loss of binding of a TIG2 polypeptide from the aqueous phase to a ChemR23 polypeptide immobilized in a membrane on the sensor. This change in mass is measured as resonance units versus time after injection or removal of the TIG2 polypeptide or candidate modulator and is measured using a Biacore Biosensor (Biacore AB). ChemR23 can be immobilized on a sensor chip (for example, research grade CM5 chip; Biacore AB) in a thin film lipid membrane according to methods described by Salamon et al. (Salamon et al., 1996, Biophys J. 71: 283-294; Salamon et al., 2001, Biophys. J. 80: 1557-1567; Salamon et al., 1999, Trends Biochem. Sci. 24: 213-219, each of which is incorporated herein by reference.). Sarrio et al. demonstrated that SPR can be used to detect ligand binding to the GPCR A(1) adenosine receptor immobilized in a lipid layer on the chip (Sarrio et al., 2000, Mol. Cell. Biol. 20: 5164-5174, incorporated herein by reference). Conditions for TIG2 binding to ChemR23 in an SPR assay can be fine-tuned by one of skill in the art using the conditions reported by Sarrio et al. as a starting point.

SPR can assay for modulators of binding in at least two ways. First, a TIG2 polypeptide can be pre-bound to immobilized ChemR23 polypeptide, followed by injection of candidate modulator at approximately 10 μl/min flow rate and a concentration ranging from 1 nM to 100 μM, preferably about 1 μM. Displacement of the bound TIG2 can be quantitated, permitting detection of modulator binding. Alternatively, the membrane-bound ChemR23 polypeptide can be pre-incubated with candidate modulator and challenged with a TIG2 polypeptide. A difference in TIG2 binding to the ChemR23 exposed to modulator relative to that on a chip not pre-exposed to modulator will demonstrate binding. In either assay, a decrease of 10% or more in the amount of a TIG2 polypeptide bound is in the presence of candidate modulator, relative to the amount of a TIG2 polypeptide bound in the absence of candidate modulator indicates that the candidate modulator inhibits the interaction of ChemR23 and TIG2.

Another method of measuring inhibition of binding of a TIG2 polypeptide to ChemR23 uses fluorescence resonance energy transfer (FRET). FRET is a quantum mechanical phenomenon that occurs between a fluorescence donor (D) and a fluorescence acceptor (A) in close proximity to each other (usually < 100 A of separation) if the emission spectrum of D overlaps with the excitation spectrum of A. The molecules to be tested, e.g., a TIG2 polypeptide and a ChemR23 polypeptide, are labeled with a complementary pair of donor and acceptor fluorophores. While bound closely together by the ChemR23:TIG2 interaction, the fluorescence emitted upon excitation of the donor fluorophore will have a different wavelength than that emitted in response to that excitation wavelength when the polypeptides are not bound, providing for quantitation of bound versus unbound polypeptides by measurement of emission intensity at each wavelength. Donor:Acceptor pairs of fluorophores with which to label the polypeptides are well known in the art. Of particular interest are variants of the *A. victoria* GFP known as Cyan FP (CFP, Donor(D)) and Yellow FP (YFP, Acceptor(A)). The GFP variants can be made as fusion proteins with the respective members of the binding pair to serve as D-A pairs in a FRET scheme to measure protein-protein interaction. Vectors for the expression of GFP variants as fusions are known in the art. As an example, a CFP-TIG2 fusion and a YFP-ChemR23 fusion can be made. The addition of a candidate modulator to the mixture of labeled TIG2 and ChemR23 proteins will result in an inhibition of energy transfer evidenced by, for example, a decrease in YFP fluorescence relative to a sample without the candidate modulator. In an assay using FRET for the detection of ChemR23:TIG2 interaction, a 10% or greater decrease in the intensity of fluorescent emission at the acceptor wavelength in samples containing a candidate modulator, relative to samples without the candidate modulator, indicates that the candidate modulator inhibits ChemR23:TIG2 interaction.

A variation on FRET uses fluorescence quenching to monitor molecular interactions. One molecule in the interacting pair can be labeled with a fluorophore, and the other with a molecule that quenches the fluorescence of the fluorophore when brought into close apposition with it. A change in fluorescence upon excitation is indicative of a change in the association of the molecules tagged with the fluorophore:quencher pair. Generally, an increase in fluorescence of the labeled ChemR23 polypeptide is indicative that the TIG2 polypeptide bearing the quencher has been displaced. For quenching assays, a 10% or greater increase in the intensity of fluorescent emission in samples containing a candidate modulator, relative to samples without the candidate modulator, indicates that the candidate modulator inhibits ChemR23:TIG2 interaction.

In addition to the surface plasmon resonance and FRET methods, fluorescence polarization measurement is useful to quantitate protein-protein binding. The fluorescence polarization value for a fluorescently-tagged molecule depends on the rotational correlation time or tumbling rate. Protein complexes, such as those formed by ChemR23 associating with a fluorescently labeled TIG2 polypeptide, have higher polarization values than uncomplexed, labeled TIG2. The inclusion of a candidate inhibitor of the ChemR23:TIG2 interaction results in a decrease in fluorescence polarization, relative to a mixture without the candidate inhibitor, if the candidate inhibitor disrupts or inhibits the interaction of ChemR23 with TIG2. Fluorescence polarization is well suited for the identification of small molecules that disrupt the formation of polypeptide or protein complexes. A decrease of 10% or more in fluorescence polarization in samples containing a candidate modulator, relative to fluorescence polarization in a sample lacking the candidate modulator, indicates that the candidate modulator inhibits ChemR23:TIG2 interaction.

Another alternative for monitoring ChemR23:TIG2 interactions uses a biosensor assay. ICS biosensors have been described by AMBRI (Australian Membrane Biotechnology Research Institute; http//www.ambri.com.au/). In this technology, the association of macromolecules such as ChemR23 and TIG2, is coupled to the closing of gramacidin-facilitated ion channels in suspended membrane bilayers and thus to a measurable change in the admittance (similar to impedence) of the biosensor. This approach is linear over six orders of magnitude of admittance change and is ideally suited for large scale, high throughput screening of small molecule combinatorial libraries. A 10% or greater change (increase or decrease) in admittance in a sample containing a candidate modulator, relative to the admittance of a sample lacking the candidate modulator, indicates that the candidate modulator inhibits the interaction of ChemR23 and TIG2.

It is important to note that in assays of protein-protein interaction, it is possible that a modulator of the interaction need not necessarily interact directly with the domain(s) of the proteins that physically interact. It is also possible that a modulator will interact at a location removed from the site of protein-protein interaction and cause, for example, a conformational change in the ChemR23 polypeptide. Modulators (inhibitors or agonists) that act in this manner are nonetheless of interest as agents to modulate the activity of ChemR23.

It should be understood that any of the binding assays described herein can be performed with a non-TIG2 ligand (for example, agonist, antagonist, etc.) of ChemR23, e.g., a small molecule identified as described herein. In practice, the use of a small molecule ligand or other non-TIG2 ligand has the benefit that non-polypeptide chemical compounds are generally cheaper and easier to produce in purified form than polypeptides such as TIG2. Thus, a non-TIG2 ligand is better suited to high-throughput assays for the identification of agonists, antagonists or inverse agonists than full length TIG2. This advantage in no way erodes the importance of assays using TIG2, however, as such assays are well suited for the initial identification of non-TIG2 ligands.

Any of the binding assays described can be used to determine the presence of an agent in a sample, e.g., a tissue sample, that binds to the ChemR23 receptor molecule, or that affects the binding of TIG2 to the receptor. To do so, ChemR23 polypeptide is reacted with TIG2 polypeptide or another ligand in the presence or absence of the sample, and TIG2 or ligand binding is measured as appropriate for the binding assay being used. A decrease of 10% or more in the binding of TIG2 or other ligand indicates that the sample contains an agent that modulates TIG2 or ligand binding to the receptor polypeptide.

Functional assays of receptor activity

i. GTPase/GTP Binding Assays:

For GPCRs such as ChemR23, a measure of receptor activity is the binding of GTP by cell membranes containing receptors. In the method described by Traynor and Nahorski, 1995, Mol. Pharmacol. 47: 848-854, incorporated herein by reference, one essentially measures G-protein coupling to membranes by measuring the binding of labeled GTP. For GTP binding assays, membranes isolated from cells expressing the receptor are incubated in a buffer containing 20 mM HEPES, pH 7.4, 100 mM NaCl, and 10 mM MgCl2, 80 pM $^{35}$S-GTPγS and 3 μM GDP. The assay mixture is incubated for 60 minutes at 30°C, after which unbound labeled GTP is removed by filtration onto GF/B filters. Bound, labeled GTP is measured by liquid scintillation counting. In order to assay for modulation of TIG2-induced ChemR23 activity, membranes prepared from cells expressing a ChemR23 polypeptide are mixed with a TIG2 polypeptide, and the GTP binding assay is performed in the presence and absence of a candidate modulator of ChemR23 activity. A decrease of 10% or more in labeled GTP binding as measured by scintillation counting in an assay of this kind containing candidate modulator, relative to an assay without the modulator, indicates that the candidate modulator inhibits ChemR23 activity.

A similar GTP-binding assay can be performed without TIG2 to identify compounds that act as agonists. In this case, TIG2-stimulated GTP binding is used as a standard. A compound is considered an agonist if it induces at least 50% of the level of GTP binding induced by full length wild-type TIG2 when the compound is present at 1 μM or less, and preferably will induce a level the same as or higher than that induced by TIG2.

GTPase activity is measured by incubating the membranes containing a ChemR23 polypeptide with γ$^{32}$P-GTP. Active GTPase will release the label as inorganic phosphate, which is detected by separation of free inorganic phosphate in a 5% suspension of activated charcoal in 20 mM $H_3PO_4$, followed by scintillation counting. Controls include assays using membranes isolated from cells not expressing ChemR23 (mock-transfected), in order to exclude possible non-specific effects of the candidate compound.

In order to assay for the effect of a candidate modulator on ChemR23-regulated GTPase activity, membrane samples are incubated with a TIG2 polypeptide, with and without the modulator, followed by the GTPase assay. A change (increase or decrease) of 10% or more in the level of GTP binding or GTPase activity relative to samples without modulator is indicative of ChemR23 modulation by a candidate modulator.

ii. Downstream Pathway Activation Assays:

a. Calcium flux - The Aequorin-based Assay.

The aequorin assay takes advantage of the responsiveness of mitochondrial apoaequorin to intracellular calcium release induced by the activation of GPCRs (Stables et al., 1997, Anal. Biochem. 252:115-126; Detheux et al., 2000, J. Exp. Med., 192 1501-1508; both of which are incorporated herein by reference). Briefly, ChemR23-expressing clones are transfected to coexpress mitochondrial apoaequorin and Gα16. Cells are incubated with 5 μM Coelenterazine H (Molecular Probes) for 4 hours at room temperature, washed in DMEM-F12 culture medium and resuspended at a concentration of 0.5 x $10^6$ cells/ml. Cells are then mixed with test agonist peptides and light emission by the aequorin is recorded with a luminometer for 30 sec. Results are expressed as Relative Light Units (RLU). Controls include assays using membranes isolated from cells not expressing ChemR23 (mock-transfected), in order to exclude possible non-specific effects of the candidate compound.

Aequorin activity or intracellular calcium levels are "changed" if light intensity increases or decreases by 10% or more in a sample of cells, expressing a ChemR23 polypeptide and treated with a candidate modulator, relative to a sample of cells expressing the ChemR23 polypeptide but not treated with the candidate modulator or relative to a sample of cells not expressing the ChemR23 polypeptide (mock-transfected cells) but treated with the candidate modulator.

When performed in the absence of a TIG2 polypeptide, the assay can be used to identify an agonist of ChemR23 activity. When the assay is performed in the presence of a TIG2 polypeptide, it can be used to assay for an antagonist.

b. Adenylate Cyclase Assay:

Assays for adenylate cyclase activity are described by Kenimer & Nirenberg, 1981, Mol. Pharmacol. 20: 585-591, incorporated herein by reference. That assay is a modification of the assay taught by Solomon et al., 1974, Anal. Biochem. 58: 541-548, also incorporated herein by reference. Briefly, 100 μl reactions contain 50 mM Tris-Hcl (pH 7.5), 5 mM $MgCl_2$, 20 mM creatine phosphate (disodium salt), 10 units (71 μg of protein) of creatine phosphokinase, 1 mM α-$^{32}$P-ATP (tetrasodium salt, 2 μCi), 0.5 mM cyclic AMP, G-$^3$H-labeled cyclic AMP (approximately 10,000 cpm), 0.5 mM Ro20-1724, 0.25% ethanol, and 50-200 μg of protein homogenate to be tested (i.e., homogenate from cells expressing or not expressing a ChemR23 polypeptide, treated or not treated with a TIG2 polypeptide with or without a candidate modualtor). Reaction mixtures are generally incubated at 37°C for 6 minutes. Following incubation, reaction mixtures are deproteinized by the addition of 0.9 ml of cold 6% trichloroacetic acid. Tubes are centrifuged at 1800 x g for 20 minutes and each supernatant solution is added to a Dowex AG50W-X4 column. The cAMP fraction from the column is eluted with 4 ml of 0.1 mM imidazole-HCl (pH 7.5) into a counting vial. Assays should be performed in triplicate. Control reactions should also be performed using protein homogenate from cells that do not express a ChemR23 polypeptide. According to the invention, adenylate cyclase activity is "changed" if it increases or decreases by 10% or more in a sample taken from cells treated with a candidate modulator of ChemR23 activity, relative to a similar sample of cells not treated with the candidate modulator or relative to a sample of cells not expressing the ChemR23 polypeptide (mock-transfected cells) but treated with the candidate modulator.

c. cAMP Assay:

Intracellular or extracellular cAMP is measured using a cAMP radioimmunoassay (RIA) or cAMP binding protein according to methods widely known in the art. For example, Horton & Baxendale, 1995, Methods Mol. Biol. 41: 91-105, which is incorporated herein by reference, describes an RIA for cAMP.

A number of kits for the measurement of cAMP are commercially available, such as the High Efficiency Fluorescence Polarization-based homogeneous assay marketed by LJL Biosystems and NEN Life Science Products. Control reactions should be performed using extracts of mock-transfected cells to exclude possible non-specific effects of some candidate modulators.

The level of cAMP is "changed" if the level of cAMP detected in cells, expressing a ChemR23 polypeptide and treated with a candidate modulator of ChemR23 activity (or in extracts of such cells), using the RIA-based assay of Horton & Baxendale, 1995, supra, increases or decreases by at least 10% relative to the cAMP level in similar cells not treated with the candidate modulator.

d. Phospholipid breakdown, DAG production and Inositol Triphosphate levels:

Receptors that activate the breakdown of phospholipids can be monitored for changes due to the activity of known or suspected modulators of ChemR23 by monitoring phospholipid breakdown, and the resulting production of second messengers DAG and/or inositol triphosphate ($IP_3$). Methods of measuring each of these are described in Phospholipid Signaling Protocols, edited by Ian M. Bird. Totowa, NJ, Humana Press, 1998, which is incorporated herein by reference. See also Rudolph et al., 1999, J. Biol. Chem. 274: 11824-11831, incorporated herein by reference, which also describes an assay for phosphatidylinositol breakdown. Assays should be performed using cells or extracts of cells expressing ChemR23, treated or not treated with a TIG2 polypeptide with or without a candidate modulator. Control reactions should be performed using mock-transfected cells, or extracts from them in order to exclude possible non-specific effects of some candidate modulators.

According to the invention, phosphatidylinositol breakdown, and diacylglycerol and/or inositol triphosphate levels are "changed" if they increase or decrease by at least 10% in a sample from cells expressing a ChemR23 polypeptide and treated with a candidate modulator, relative to the level observed in a sample from cells expressing a ChemR23 polypeptide that is not treated with the candidate modulator.

e. PKC activation assays:

Growth factor receptor tyrosine kinases tend to signal via a pathway involving activation of Protein Kinase C (PKC), which is a family of phospholipid- and calcium-activated protein kinases. PKC activation ultimately results in the transcription of an array of proto-oncogene transcription factor-encoding genes, including c-fos, c-myc and c-jun, proteases, protease inhibitors, including collagenase type I and plasminogen activator inhibitor, and adhesion molecules, including intracellular adhesion molecule I (ICAM I). Assays designed to detect increases in gene products induced by PKC can be used to monitor PKC activation and thereby receptor activity. In addition, the activity of receptors that signal via PKC can be monitored through the use of reporter gene constructs driven by the control sequences of genes activated by PKC activation. This type of reporter gene-based assay is discussed in more detail below.

For a more direct measure of PKC activity, the method of Kikkawa et al., 1982, J. Biol. Chem. 257: 13341, incorporated herein by reference, can be used. This assay measures phosphorylation of a PKC substrate peptide, which is subsequently separated by binding to phosphocellulose paper. This PKC assay system can be used to measure activity of purified kinase, or the activity in crude cellular extracts. Protein kinase C sample can be diluted in 20 mM HEPES/ 2 mM DTT immediately prior to assay.

The substrate for the assay is the peptide Ac-FKKSFKL-NH2 (SEQ ID NO:35), derived from the myristoylated alanine-rich protein kinase C substrate protein (MARCKS). The $K_m$ of the enzyme for this peptide is approximately 50

μM. Other basic, protein kinase C-selective peptides known in the art can also be used, at a concentration of at least 2 -3 times their $K_m$. Cofactors required for the assay include calcium, magnesium, ATP, phosphatidylserine and diacylglycerol. Depending upon the intent of the user, the assay can be performed to determine the amount of PKC present (activating conditions) or the amount of active PCK present (non-activating conditions). For most purposes according to the invention, non-activating conditions will be used, such that the PKC that is active in the sample when it is isolated is measured, rather than measuring the PKC that can be activated. For non-activating conditions, calcium is omitted in the assay in favor of EGTA.

The assay is performed in a mixture containing 20 mM HEPES, pH 7.4, 1-2 mM DTT, 5 mM $MgCl_2$, 100 μM ATP, ~1 μCi γ-$^{32}$P-ATP, 100 μg/ml peptide substrate (~100 μM), 140 μM /3.8 μM phosphatidylserine/diacylglycerol membranes, and 100 μM calcium (or 500 μM EGTA). 48 μl of sample, diluted in 20 mM HEPES, pH 7.4, 2 mM DTT is used in a final reaction volume of 80 μl. Reactions are performed at 30°C for 5-10 minutes, followed by addition of 25 μl of 100 mM ATP, 100 mM EDTA, pH 8.0, which stops the reactions.

After the reaction is stopped, a portion (85 μl) of each reaction is spotted onto a Whatman P81 cellulose phosphate filter, followed by washes: four times 500 ml in 0.4% phosphoric acid, (5-10 min per wash); and a final wash in 500 ml 95% EtOH, for 2-5 min. Bound radioactivity is measured by scintillation counting. Specific activity (cpm/nmol) of the labeled ATP is determined by spotting a sample of the reaction onto P81 paper and counting without washing. Units of PKC activity, defined as nmol phosphate transferred per min, are calculated as follows:

The activity, in UNITS (nmol/min) is:

$$= \frac{(\text{cpm on paper}) \times (105\ \mu\text{l total} / 85\ \mu\text{l spotted})}{(\text{assay time, min})\ (\text{specific activity of ATP cpm/nmol})}.$$

An alternative assay can be performed using a Protein Kinase C Assay Kit sold by PanVera (Cat. # P2747).

Assays are performed on extracts from cells expressing a ChemR23 polypeptide, treated or not treated with a TIG2 polypeptide with or without a candidate modulator. Control reactions should be performed using mock-transfected cells, or extracts from them in order to exclude possible non-specific effects of some candidate modulators.

According to the invention, PKC activity is "changed" by a candidate modulator when the units of PKC measured by either assay described above increase or decrease by at least 10%, in extracts from cells expressing ChemR23 and treated with a candidate modulator, relative to a reaction performed on a similar sample from cells not treated with a candidate modulator.

f. Kinase assays:

MAP kinase activity can be assayed using any of several kits available commercially, for example, the p38 MAP Kinase assay kit sold by New England Biolabs (Cat # 9820) or the FlashPlate™ MAP Kinase assays sold by Perkin-Elmer Life Sciences.

MAP Kinase activity is "changed" if the level of activity is increased or decreased by 10% or more in a sample from cells, expressing a ChemR23 polypeptide, treated with a candidate modulator relative to MAP kinase activity in a sample from similar cells not treated with the candidate modulator.

Direct assays for tyrosine kinase activity using known synthetic or natural tyrosine kinase substrates and labeled phosphate are well known, as are similar assays for other types of kinases (e.g., Ser/Thr kinases). Kinase assays can be performed with both purified kinases and crude extracts prepared from cells expressing a ChemR23 polypeptide, treated with or without a TIG2 polypeptide, with or without a candidate modulator. Control reactions should be performed using mock-transfected cells, or extracts from them in order to exclude possible non-specific effects of some candidate modulators. Substrates can be either full length protein or synthetic peptides representing the substrate. Pinna & Ruzzene (1996, Biochem. Biophys. Acta 1314: 191-225, incorporated herein by reference) list a number of phosphorylation substrate sites useful for measuring kinase activities. A number of kinase substrate peptides are commercially available. One that is particularly useful is the "Src-related peptide," RRLIEDAEYAARG (SEQ ID NO: 11; available from Sigma # A7433), which is a substrate for many receptor and nonreceptor tyrosine kinases. Because the assay described below requires binding of peptide substrates to filters, the peptide substrates should have a net positive charge to facilitate binding. Generally, peptide substrates should have at least 2 basic residues and a free amino terminus. Reactions generally use a peptide concentration of 0.7-1.5 mM.

Assays are generally carried out in a 25 μl volume comprising 5 μl of 5X kinase buffer (5 mg/mL BSA, 150 mM Tris-Cl (pH 7.5), 100 mM $MgCl_2$; depending upon the exact kinase assayed for, $MnCl_2$ can be used in place of or in addition to the $MgCl_2$), 5 μl of 1.0 mM ATP (0.2 mM final concentration), γ-32P-ATP (100-500 cpm/pmol), 3 μl of 10 mM peptide substrate (1.2 mM final concentration), cell extract containing kinase to be tested (cell extracts used for kinase assays should contain a phosphatase inhibitor (e.g. 0.1-1 mM sodium orthovanadate)), and $H_2O$ to 25 μl. Reactions are performed at 30°C, and are initiated by the addition of the cell extract.

Kinase reactions are performed for 30 seconds to about 30 minutes, followed by the addition of 45 μl of ice-cold 10% trichloroacetic acid (TCA). Samples are spun for 2 minutes in a microcentrifuge, and 35μl of the supernatant is spotted onto Whatman P81 cellulose phosphate filter circles. The filters are washed three times with 500 ml cold 0.5% phosphoric acid, followed by one wash with 200 ml of acetone at room temperature for 5 minutes. Filters are dried and incorporated 32P is measured by scintillation counting. The specific activity of ATP in the kinase reaction (e.g., in cpm/ pmol) is determined by spotting a small sample (2-5 μl) of the reaction onto a P81 filter circle and counting directly, without washing. Counts per minute obtained in the kinase reaction (minus blank) are then divided by the specific activity to determine the moles of phosphate transferred in the reaction.

Tyrosine kinase activity is "changed" if the level of kinase activity is increased or decreased by 10% or more in a sample from cells, expressing a ChemR23 polypeptide, treated with a candidate modulator relative to kinase activity in a sample from similar cells not treated with the candidate modulator.

g. Transcriptional reporters for downstream pathway activation:

The intracellular signal initiated by binding of an agonist to a receptor, e.g., ChemR23, sets in motion a cascade of intracellular events, the ultimate consequence of which is a rapid and detectable change in the transcription or translation of one or more genes. The activity of the receptor can therefore be monitored by measuring the expression of a reporter gene driven by control sequences responsive to ChemR23 activation.

As used herein "promoter" refers to the transcriptional control elements necessary for receptor-mediated regulation of gene expression, including not only the basal promoter, but also any enhancers or transcription-factor binding sites necessary for receptor-regulated expression. By selecting promoters that are responsive to the intracellular signals resulting from agonist binding, and operatively linking the selected promoters to reporter genes whose transcription, translation or ultimate activity is readily detectable and measurable, the transcription based reporter assay provides a rapid indication of whether a given receptor is activated.

Reporter genes such as luciferase, CAT, GFP, β-lactamase or β-galactosidase are well known in the art, as are assays for the detection of their products.

Genes particularly well suited for monitoring receptor activity are the "immediate early" genes, which are rapidly induced, generally within minutes of contact between the receptor and the effector protein or ligand. The induction of immediate early gene transcription does not require the synthesis of new regulatory proteins. In addition to rapid responsiveness to ligand binding, characteristics of preferred genes useful to make reporter constructs include: low or undetectable expression in quiescent cells; induction that is transient and independent of new protein synthesis; subsequent shut-off of transcription requires new protein synthesis; and mRNAs transcribed from these genes have a short half-life. It is preferred, but not necessary that a transcriptional control element have all of these properties for it to be useful.

An example of a gene that is responsive to a number of different stimuli is the c-fos proto-oncogene. The c-fos gene is activated in a protein-synthesis-independent manner by growth factors, hormones, differentiation-specific agents, stress, and other known inducers of cell surface proteins. The induction of c-fos expression is extremely rapid, often occurring within minutes of receptor stimulation. This characteristic makes the c-fos regulatory regions particularly attractive for use as a reporter of receptor activation.

The c-fos regulatory elements include (see, Verma et al., 1987, Cell 51: 513-514): a TATA box that is required for transcription initiation; two upstream elements for basal transcription, and an enhancer, which includes an element with dyad symmetry and which is required for induction by TPA, serum, EGF, and PMA.

The 20 bp c-fos transcriptional enhancer element located between -317 and -298 bp upstream from the c-fos mRNA cap site, is essential for serum induction in serum starved NIH 3T3 cells. One of the two upstream elements is located at -63 to -57 and it resembles the consensus sequence for cAMP regulation.

The transcription factor CREB (cyclic AMP responsive element binding protein) is, as the name implies, responsive to levels of intracellular cAMP. Therefore, the activation of a receptor that signals via modulation of cAMP levels can be monitored by measuring either the binding of the transcription factor, or the expression of a reporter gene linked to a CREB-binding element (termed the CRE, or cAMP response element). The DNA sequence of the CRE is TGACGTCA (SEQ ID NO: 12). Reporter constructs responsive to CREB binding activity are described in U.S. Patent No. 5,919,649.

Other promoters and transcriptional control elements, in addition to the c-fos elements and CREB-responsive constructs, include the vasoactive intestinal peptide (VIP) gene promoter (cAMP responsive; Fink et al., 1988, Proc. Natl. Acad. Sci. 85:6662-6666); the somatostatin gene promoter (cAMP responsive; Montminy et al., 1986, Proc. Natl. Acad. Sci. 8.3:6682-6686); the proenkephalin promoter (responsive to cAMP, nicotinic agonists, and phorbol esters; Comb et al., 1986, Nature 323:353-356); the phosphoenolpyruvate carboxy-kinase (PEPCK) gene promoter (cAMP responsive; Short et al., 1986, J. Biol. Chem. 261:9721-9726).

Additional examples of transcriptional control elements that are responsive to changes in GPCR activity include, but are not limited to those responsive to the AP-1 transcription factor and those responsive to NF-κB activity. The consensus AP-1 binding site is the palindrome TGA(C/G)TCA (Lee et al., 1987, *Nature* 325: 368-372; Lee et al., 1987, *Cell* 49: 741-752). The AP-1 site is also responsible for mediating induction by tumor promoters such as the phorbol ester 12-*O*-tetradecanoylphorbol-β-acetate (TPA), and are therefore sometimes also referred to as a TRE, for TPA-response element. AP-1 activates numerous genes that are involved in the early response of cells to growth stimuli. Examples of AP-1-responsive genes include, but are not limited to the genes for Fos and Jun (which proteins themselves make up AP-1 activity), Fos-related antigens (Fra) 1 and 2, IκBα, ornithine decarboxylase, and annexins I and II.

The NF-κB binding element has the consensus sequence GGGGACTTTCC (SEQ ID NO:36). A large number of genes have been identified as NF-κB responsive, and their control elements can be linked to a reporter gene to monitor GPCR activity. A small sample of the genes responsive to NF-κB includes those encoding IL-1β (Hiscott et al., 1993, Mol. Cell. Biol. 13: 6231-6240), TNF-α (Shakhov et al., 1990, J. Exp. Med. 171: 35-47), CCR5 (Liu et al., 1998, AIDS Res. Hum. Retroviruses 14: 1509-1519), P-selectin (Pan & McEver, 1995, J. Biol. Chem. 270: 23077-23083), Fas ligand (Matsui et al., 1998, J. Immunol. 161: 3469-3473), GM-CSF (Schreck & Baeuerle, 1990, Mol. Cell. Biol. 10: 1281-1286) and IκBα (Haskill et al., 1991, Cell 65: 1281-1289). Each of these references is incorporated herein by reference. Vectors encoding NF-κB-responsive reporters are also known in the art or can be readily made by one of skill in the art using, for example, synthetic NF-κB elements and a minimal promoter, or using the NF-κB-responsive sequences of a gene known to be subject to NF-κB regulation. Further, NF-κB responsive reporter constructs are commercially available from, for example, CLONTECH.

A given promoter construct should be tested by exposing ChemR23-expressing cells, transfected with the construct, to a TIG2 polypeptide. An increase of at least two-fold in the expression of reporter in response to TIG2 polypeptide indicates that the reporter is an indicator of ChemR23 activity.

In order to assay ChemR23 activity with a TIG2-responsive transcriptional reporter construct, cells that stably express a ChemR23 polypeptide are stably transfected with the reporter construct. To screen for agonists, the cells are left untreated, exposed to candidate modulators, or exposed to a a TIG2 polypeptide, and expression of the reporter is measured. The TIG2-treated cultures serve as a standard for the level of transcription induced by a known agonist. An increase of at least 50% in reporter expression in the presence of a candidate modulator indicates that the candidate is a modulator of ChemR23 activity. An agonist will induce at least as much, and preferably the same amount or more, reporter expression than the TIG2 polypeptide. This approach can also be used to screen for inverse agonists where cells express a ChemR23 polypeptide at levels such that there is an elevated basal activity of the reporter in the absence of TIG2 or another agonist. A decrease in reporter activity of 10% or more in the presence of a candidate modulator, relative to its absence, indicates that the compound is an inverse agonist.

To screen for antagonists, the cells expressing ChemR23 and carrying the reporter construct are exposed to a TIG2 polypeptide (or another agonist) in the presence and absence of candidate modulator. A decrease of 10% or more in reporter expression in the presence of candidate modulator, relative to the absence of the candidate modulator, indicates that the candidate is a modulator of ChemR23 activity.

Controls for transcription assays include cells not expressing ChemR23 but carrying the reporter construct, as well as cells with a promoterless reporter construct. Compounds that are identified as modulators of ChemR23-regulated transcription should also be analyzed to determine whether they affect transcription driven by other regulatory sequences and by other receptors, in order to determine the specificity and spectrum of their activity.

The transcriptional reporter assay, and most cell-based assays, are well suited for screening expression libraries for proteins for those that modulate ChemR23 activity. The libraries can be, for example, cDNA libraries from natural sources, e.g., plants, animals, bacteria, etc., or they can be libraries expressing randomly or systematically mutated variants of one or more polypeptides. Genomic libraries in viral vectors can also be used to express the mRNA content of one cell or tissue, in the different libraries used for screening of ChemR23.

Any of the assays of receptor activity, including the GTP-binding, GTPase, adenylate cyclase, CAMP, phospholipid-breakdown, diacylglyceorl, inositol triphosphate, PKC, kinase and transcriptional reporter assays, can be used to determine the presence of an agent in a sample, e.g., a tissue sample, that affects the activity of the ChemR23 receptor molecule. To do so, ChemR23 polypeptide is assayed for activity in the presence and absence of the sample or an extract of the sample. An increase in ChemR23 activity in the presence of the sample or extract relative to the absence of the sample indicates that the sample contains an agonist of the receptor activity. A decrease in receptor activity in the presence of TIG2 or another agonist and the sample, relative to receptor activity in the presence of TIG2 polypeptide alone, indicates that the sample contains an antagonist of ChemR23 activity. If desired, samples can then be fractionated and further tested to isolate or purify the agonist or antagonist. The amount of increase or decrease in measured activity necessary for a sample to be said to contain a modulator depends upon the type of assay used. Generally, a 10% or greater change (increase or decrease) relative to an assay performed in the absence of a sample indicates the presence of a modulator in the sample. One exception is the transcriptional reporter assay, in which at least a two-fold increase or 10% decrease in signal is necessary for a sample to be said to contain a modulator. It is preferred that an agonist stimulates at least 50%, and preferably 75% or 100% or more, e.g., 2-fold, 5-fold, 10-fold or greater receptor activation than wild-type TIG2.

Other functional assays include, for example, microphysiometer or biosensor assays (see Hafner, 2000, *Biosens. Bioelectron.* 15: 149-158, incorporated herein by reference).

II. Diagnostic Assays Based upon the Interaction of ChemR23 and TIG2:

Signaling through GPCRs is instrumental in the pathology of a large number of diseases and disorders. ChemR23, which is expressed in cells of the lymphocyte lineages and which has been shown to act as a co-receptor for immunodeficiency viruses can have a role in immune processes, disorders or diseases. The ChemR23 expression pattern also includes bone and cartilage, indicating that this receptor can play a role in diseases, disorders or processes (e.g., fracture healing) affecting these tissues. Expression in adult parathyroid suggests possible importance in phosphocalic metabolism.

Because of its expression in cells of the lymphocyte lineages, ChemR23 can be involved in the body's response to viral infections or in diseases induced by various viruses, including HIV types I and II, or bacteria. The expression pattern of ChemR23 and the knowledge with respect to disorders generally mediated by GPCRs suggests that ChemR23 can be involved in disturbances of cell migration, cancer, development of tumours and tumour metastasis, inflammatory and neo-plastic processes, wound and bone healing and dysfunction of regulatory growth functions, diabetes, obesity, anorexia, bulimia, acute heart failure, hypotension, hypertension, urinary retention, osteoporosis, angina pectoris, myocardial infarction, restenosis, atherosclerosis, diseases characterised by excessive smooth muscle cell proliferation, aneurysms, diseases characterised by loss of smooth muscle cells or reduced smooth muscle cell proliferation, stroke, ischemia, ulcers, allergies, benign prostatic hypertrophy, migraine, vomiting, psychotic and neurological disorders, including anxiety, schizophrenia, manic depression, depression, delirium, dementia and severe mental retardation, degenerative diseases, neurodegenerative diseases such as Alzheimer's disease or Parkinson's disease, and dyskinasias, such as Huntington's disease or Gilles de la Tourett's syndrome and other related diseases.

The interaction of ChemR23 with TIG2 can be used as the basis of assays for the diagnosis or monitoring of diseases, disorders or processes involving ChemR23 signaling. Diagnostic assays for ChemR23-related diseases or disorders can have several different forms. First, diagnostic assays can measure the amount of ChemR23 and/or TIG2 polypeptide, genes or mRNA in a sample of tissue. Assays that measure the amount of mRNA encoding either or both of these polypeptides also fit in this category. Second, assays can evaluate the qualities of the receptor or the ligand. For example, assays that determine whether an individual expresses a mutant or variant form of either ChemR23 or TIG2, or both, can be used diagnostically. Third, assays that measure one or more activities of ChemR23 polypeptide can be used diagnostically.

Therefore, the present invention relates to a method according to the present invention, wherein said disease or disorder is a ChemR23-related disease or a ChemR23-related disorder chosen from the group consisting of Cancer, Tumor metastasis, Inflammatory diseases, Autoimmune diseases, Inherited or acquired immune deficiencies, Osteoporosis, Bone healing, Bone tissue grafts, Graft rejection, Psoriasis, Eczema, Inflammatory infection and trophic diseases of skin, Viral, bacterial and parasitic infections, Female infertility, and, Ovary and uterus tumors. Alternatively, the present invention relates to a method according to the present invention, wherein said disease or disorder is a TIG2-related disease or a TIG2-related disorder chosen from the group consisting of Osteoporosis, Bone healing, Bone tissue grafts, Graft rejection, Psoriasis, Eczema, Inflammatory infection and trophic diseases of skin, Viral, bacterial and parasitic infection, Female infertility, and, Ovary and uterus tumors.

According to the present method, said TIG2 polypeptide may be a polypeptide having at least 31% identity or higher identity, such as 45%, 55%, 65%, 75%, 85%, 95% or even 100% to the polypeptide represented by SEQ ID NO: 8; and wherein said polypeptide binds specifically to and activates a signaling activity of a ChemR23 polypeptide having the sequence of SEQ ID NO:2. Alternatively, said TIG2 polypeptide may be a fragment of the full length polypeptide of SEQ ID NO:8, wherein the fragment retains at least 50% of the binding activity and level of signaling activation of the full length polypeptide of SEQ ID NO:8. According to the present invention, said TIG2 polypeptide may comprise one or more additions, insertions, deletions or substitutions relative to SEQ ID NO: 8. Said TIG2 polypeptide may a truncated TIG2 polypeptide represented by SEQ ID NO:46 or represented by any of SEQ ID NO:48 to 58; said TIG2 polypeptide may comprise additional sequences forming a TIG2 fusion protein, wherein said additional sequences may be chosen from the group consisting of glutathione-S-transferase (GST), maltose binding protein, alkaline phosphatase, thioredoxin, green fluorescent protein (GFP), histidine tags (e.g., 6X or greater His), or epitope tags (e.g., Myc tag, FLAG tag) sequences.

A. Assays that measure the amount of ChemR23 or TIG2

ChemR23 and TIG2 levels can be measured and compared to standards in order to determine whether an abnormal level of the receptor or its ligand is present in a sample, either of which indicate probable dysregulation of ChemR23 signaling. Polypeptide levels are measured, for example, by immunohistochemistry using antibodies specific for the polypeptide. A sample isolated from an individual suspected of suffering from a disease or disorder characterized by ChemR23 activity is contacted with an antibody for ChemR23 or TIG2, and binding of the antibody is measured as known in the art (e.g., by measurement of the activity of an enzyme conjugated to a secondary antibody).

Another approach to the measurement of ChemR23 and/or TIG2 polypeptide levels uses flow cytometry analysis of cells from an affected tissue. Methods of flow cytometry, including the fluorescent labeling of antibodies specific for ChemR23 or TIG2, are well known in the art. Other approaches include radioimmunoassay or ELISA. Methods for each of these are also well known in the art.

The amount of binding detected is compared to the binding in a sample of similar tissue from a healthy individual, or from a site on the affected individual that is not so affected. An increase of 10% or more relative to the standard is diagnostic for a disease or disorder characterized by ChemR23 dysregulation.

ChemR23 and TIG2 expression can also be measured by determining the amount of mRNA encoding either or both of the polypeptides in a sample of tissue. mRNA can be quantitated by quantitative or semi-quantitative PCR. Methods of "quantitative" amplification are well known to those of skill in the art, and primer sequences for the amplification of both ChemR23 and TIG2 are disclosed herein. A common method of quantitative PCR involves simultaneously co-amplifying a known quantity of a control sequence using the same primers. This provides an internal standard that can be used to calibrate the PCR reaction. Detailed protocols for quantitative PCR are provided in PCR Protocols, A Guide to Methods and Applications, Innis et al., Academic Press, Inc. N.Y., (1990), which is incorporated herein by reference. An increase of 10% or more in the amount of mRNA encoding ChemR23 or TIG2 in a sample, relative to the amount expressed in a sample of like tissue from a healthy individual or in a sample of tissue from an unaffected location in an affected individual is diagnostic for a disease or disorder characterized by dysregulation of ChemR23 signaling.

B. Qualitative assays

Assays that evaluate whether or not the ChemR23 polypeptide or the mRNA encoding it are wild-type or not can be used diagnostically. In order to diagnose a disease or disorder characterized by ChemR23 or TIG2 dysregulation in this manner, RNA isolated from a sample is used as a template for PCR amplification of TIG2 and/or ChemR23. The amplified sequences are then either directly sequenced using standard methods, or are first cloned into a vector, followed by sequencing. A difference in the sequence that changes one or more encoded amino acids relative to the sequence of wild-type ChemR23 or TIG2 can be diagnostic of a disease or disorder characterized by dysregulation of ChemR23 signaling. It can be useful, when a change in coding sequence is identified in a sample, to express the variant receptor or ligand and compare its activity to that of wild type ChemR23 or TIG2. Among other benefits, this approach can provide novel mutants, including constitutively active and null mutants.

In addition to standard sequencing methods, amplified sequences can be assayed for the presence of specific mutations using, for example, hybridization of molecular beacons that discriminate between wild-type and variant sequences. Hybridization assays that discriminate on the basis of changes as small as one nucleotide are well known in the art. Alternatively, any of a number of "minisequencing" assays can be performed, including, those described, for example, in U.S. Patents 5,888,819, 6,004,744 and 6,013,431 (incorporated herein by reference). These assays and others known in the art can determine the presence, in a given sample, of a nucleic acid with a known polymorphism.

If desired, array or microarray-based methods can be used to analyze the expression or the presence of mutation, in ChemR23 or TIG2 sequences. Array-based methods for minisequencing and for quantitation of nucleic acid expression are well known in the art.

C. Functional assays.

Diagnosis of a disease or disorder characterized by the dysregulation of ChemR23 signaling can also be performed using functional assays. To do so, cell membranes or cell extracts prepared from a tissue sample are used in an assay of ChemR23 activity as described herein (e.g., ligand binding assays, the GTP-binding assay, GTPase assay, adenylate cyclase assay, cAMP assay, phospholipid breakdown, diacyl glycerol or inositol triphosphate assays, PKC activation assay, or kinase assay). The activity detected is compared to that in a standard sample taken from a healthy individual or from an unaffected site on the affected individual. As an alternative, a sample or extract of a sample can be applied to cells expressing ChemR23, followed by measurement of ChemR23 signaling activity relative to a standard sample. A difference of 10% or more in the activity measured in any of these assays, relative to the activity of the standard, is diagnostic for a disease or disorder characterized by dysregulation of ChemR23 signaling.

Modulation of ChemR23 Activity in a Cell According to the Invention

The discovery of TIG2 as a ligand of ChemR23 provides methods of modulating the activity of a ChemR23 polypeptide in a cell. ChemR23 activity is modulated in a cell by delivering to that cell an agent that modulates the function of a ChemR23 polypeptide. This modulation can be performed in cultured cells as part of an assay for the identification of additional modulating agents, or, for example, in an animal, including a human. Agents include TIG2 polypeptides as defined herein, as well as additional modulators identified using the screening methods described herein.

An agent can be delivered to a cell by adding it to culture medium. The amount to deliver will vary with the identity of the agent and with the purpose for which it is delivered. For example, in a culture assay to identify antagonists of ChemR23 activity, one will preferably add an amount of TIG2 polypeptide that half- maximally activates the receptors (e.g., approximately $EC_{50}$), preferably without exceeding the dose required for receptor saturation. This dose can be determined by titrating the amount of TIG2 polypeptide to determine the point at which further addition of TIG2 has no additional effect on ChemR23 activity.

When a modulator of ChemR23 activity is administered to an animal for the treatment of a disease or disorder, the amount administered can be adjusted by one of skill in the art on the basis of the desired outcome. Successful treatment is achieved when one or more measurable aspects of the pathology (e.g., tumor cell growth, accumulation of inflammatory cells) is changed by at least 10% relative to the value for that aspect prior to treatment.

Candidate Modulators Useful According to the Invention

Candidate modulators can be screened from large libraries of synthetic or natural compounds. Numerous means are currently used for random and directed synthesis of saccharide, peptide, lipid, carbohydrate, and nucleic acid based compounds. Synthetic compound libraries are commercially available from a number of companies including, for example, Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries of small organic molecules are available and can be prepared. Alternatively, libraries of natural compounds in the form of bacterial, fungal, plant and animal extracts are available from e.g., Pan Laboratories (Bothell, WA) or MycoSearch (NC), or are readily produceable by methods well known in the art. Additionally, natural and synthetically produced libraries and compounds are readily modified through conventional chemical, physical, and biochemical means.

As noted previously herein, candidate modulators can also be variants of known polypeptides (e.g., TIG2, antibodies) or nucleic acids (e.g., aptamers) encoded in a nucleic acid library. Cells (e.g., bacteria, yeast or higher eukaryotic cells) transformed with the library can be grown and prepared as extracts, which are then applied in ChemR23 binding assays or functional assays of ChemR23 activity.

Antibodies Useful According to the Invention

The invention provides for antibodies to ChemR23 and TIG2. Antibodies can be made using standard protocols known in the art (See, for example, Antibodies: A Laboratory Manual ed. by Harlow and Lane (Cold Spring Harbor Press: 1988)). A mammal, such as a mouse, hamster, or rabbit can be immunized with an immunogenic form of the peptide (e.g., a ChemR23 or TIG2 polypeptide or an antigenic fragment which is capable of eliciting an antibody response, or a fusion protein as described herein above). Immunogens for raising antibodies are prepared by mixing the polypeptides (e.g., isolated recombinant polypeptides or synthetic peptides) with adjuvants. Alternatively, ChemR23 or TIG2 polypeptides or peptides are made as fusion proteins to larger immunogenic proteins. Polypeptides can also be covalently linked to other larger immunogenic proteins, such as keyhole limpet hemocyanin. Alternatively, plasmid or viral vectors encoding ChemR23 or TIG2, or a fragment of these proteins, can be used to express the polypeptides and generate an immune response in an animal as described in Costagliola et al., 2000, J. Clin. Invest. 105:803-811, which is incorporated herein by reference. In order to raise antibodies, immunogens are typically administered intradermally, subcutaneously, or intramuscularly to experimental animals such as rabbits, sheep, and mice. In addition to the antibodies discussed above, genetically engineered antibody derivatives can be made, such as single chain antibodies.

The progress of immunization can be monitored by detection of antibody titers in plasma or serum. Standard ELISA, flow cytometry or other immunoassays can also be used with the immunogen as antigen to assess the levels of antibodies. Antibody preparations can be simply serum from an immunized animal, or if desired, polyclonal antibodies can be isolated from the serum by, for example, affinity chromatography using immobilized immunogen.

To produce monoclonal antibodies, antibody-producing splenocytes can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar *et al.,*

(1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a TIG2 or ChemR23 peptide or polypeptide, and monoclonal antibodies isolated from the media of a culture comprising such hybridoma cells.

Transgenic Animals Useful According to the Invention

Transgenic animals expressing ChemR23 or TIG2 or variants thereof are useful to study the signaling through ChemR23, as well as for the study of drugs or agents that modulate the activity of ChemR23. A transgenic animal is a non-human animal containing at least one foreign gene, called a transgene, which is part of its genetic material. Preferably, the transgene is contained in the animal's germ line such that it can be transmitted to the animal's offspring. A number of techniques may be used to introduce the transgene into an animal's genetic material, including, but not limited to, microinjection of the transgene into pronuclei of fertilized eggs and manipulation of embryonic stem cells (U.S. Patent No. 4,873,191 by Wagner and Hoppe; Palmiter and Brinster, 1986, *Ann. Rev. Genet.,* 20:465-499; French Patent Application 2593827 published Aug. 7, 1987, all of which are incorporated herein by reference). Transgenic animals can carry the transgene in all their cells or can be genetically mosaic.

According to the method of conventional transgenesis, additional copies of normal or modified genes are injected into the male pronucleus of the zygote and become integrated into the genomic DNA of the recipient mouse. The transgene is transmitted in a Mendelian manner in established transgenic strains. Transgenes can be constitutively expressed or can be tissue specific or even responsive to an exogenous drug, e.g., Tetracycline. A transgenic animal expressing one transgene can be crossed to a second transgenic animal expressing a second transgene such that their offspring will carry and express both transgenes.

Knock-Out Animals

Animals bearing a homozygous deletion in the chromosomal sequences encoding either ChemR23 or TIG2 or variants can be used to study the function of the receptor and ligand. Of particular interest is whether a TIG2 knockout has a distinct phenotype, which may point to whether TIG2 is the only ligand that binds ChemR23 or if it is a member of a family. Of further particular interest is the identification of identification of ChemR23/TIG2 in specific physiological and/or pathological processes.

i. Standard knock out animals

Knock out animals are produced by the method of creating gene deletions with homologous recombination. This technique is based on the development of embryonic stem (ES) cells that are derived from embryos, are maintained in culture and have the capacity to participate in the development of every tissue in the animals when introduced into a host blastocyst. A knock out animal is produced by directing homologous recombination to a specific target gene in the ES cells, thereby producing a null allele of the gene. The technology for making knock-out animals is well described (see, for example, Huszar et al., 1997, *Cell,* 88:131; and Ohki-Hamazaki et al., 1997, *Nature,* 390:165, both of which are incorporated herein by reference). One of skill in the art can generate a homozygous ChemR23 or TIG2 knock-out animal (e.g., a mouse) using the sequences for ChemR23 and TIG2 (disclosed herein and known in the art) to make the gene targeting construct.

ii. Tissue specific knock out

The method of targeted homologous recombination has been improved by the development of a system for site-specific recombination based on the bacteriophage P1 site specific recombinase Cre. The Cre-loxP site-specific DNA recombinase from bacteriophage P1 is used in transgenic mouse assays in order to create gene knockouts restricted to defined tissues or developmental stages. Regionally restricted genetic deletion, as opposed to global gene knockout, has the advantage that a phenotype can be attributed to a particular cell/tissue (Marth, 1996, *Clin. Invest.* 97: 1999). In the Cre-loxP system one transgenic mouse strain is engineered such that loxP sites flank one or more exons of the gene of interest. Homozygotes for this so called 'floxed gene' are crossed with a second transgenic mouse that expresses the Cre gene under control of a cell/tissue type transcriptional promoter. Cre protein then excises DNA between loxP recognition sequences and effectively removes target gene function (Sauer, 1998, *Methods,* 14:381). There are now many in vivo examples of this method, including, for instance, the inducible inactivation of mammary tissue specific genes (Wagner et al., 1997, *Nucleic Acids Res.,* 25:4323). One of skill in the art can therefore generate a tissue-specific knock-out animal in which ChemR23 or TIG2 is homozygously eliminated in a chosen tissue or cell type.

Kits Useful According to the Invention

The invention provides for kits useful for screening for modulators of ChemR23 activity, as well as kits useful for diagnosis of diseases or disorders characterized by dysregulation of ChemR23 signaling. Kits useful according to the invention can include an isolated ChemR23 polypeptide (including a membrane-or cell-associated ChemR23 polypeptide, e.g., on isolated membranes, cells expressing ChemR23, or, on an SPR chip) and an isolated TIG2 polypeptide. A kit can also comprise an antibody specific for ChemR23 and/or an antibody for TIG2. Alternatively, or in addition, a kit can contain cells transformed to express a ChemR23 polypeptide and/or cells transformed to express a TIG2 polypeptide. In a further embodiment, a kit according to the invention can contain a polynucleotide encoding a ChemR23 polypeptide and/or a polynucleotide encoding a TIG2 polypeptide. In a still further embodiment, a kit according to the invention may comprise the specific primers useful for amplification of ChemR23 or TIG2 as described below. All kits according to the invention will comprise the stated items or combinations of items and packaging materials therefor. Kits will also include instructions for use.

According to the present kit, said TIG2 polypeptide may be a polypeptide having at least 31% identity or higher identity, such as 45%, 55%, 65%, 75%, 85%, 95% or even 100% to the polypeptide represented by SEQ ID NO:8; and wherein said polypeptide binds specifically to and activates a signaling activity of a ChemR23 polypeptide having the sequence of SEQ ID NO:2. Alternatively, said TIG2 polypeptide may be a fragment of the full length polypeptide of SEQ ID NO:8, wherein the fragment retains at least 50% of the binding activity and level of signaling activation of the full length polypeptide of SEQ ID NO:8. According to the present invention, said TIG2 polypeptide may comprise one or more additions, insertions, deletions or substitutions relative to SEQ ID NO: 8. Said TIG2 polypeptide may a truncated TIG2 polypeptide represented by SEQ ID NO:46 or represented by any of SEQ ID NO:48 to 58; said TIG2 polypeptide may comprise additional sequences forming a TIG2 fusion protein, wherein said additional sequences may be chosen from the group consisting of glutathione-S-transferase (GST), maltose binding protein, alkaline phosphatase, thioredoxin, green fluorescent protein (GFP), histidine tags (e.g., 6X or greater His), or epitope tags (e.g., Myc tag, FLAG tag) sequences.

EXAMPLES

In the following examples, all chemicals are obtained from Sigma, unless stated. The cell culture media are from Gibco BRL and the peptides are from Bachem.

Example 1: Cloning of human ChemR23 receptor.

Human ChemR23 was cloned as described in Samson et al. (1998) (SEQ ID Nos 1 and 2). As an example of one set of steps one could use to clone other ChemR23 polypeptides useful according to the invention, the method is described here. In order to clone the ChemR23 sequence, a classical cloning procedure was performed on human genomic DNA. A clone, designated HOP 102, was amplified from human genomic DNA by using degenerate oligonucleotides. HOP 102 shared 45-50% identity with fMLP and C5a receptors and somewhat lower similarities with the family of chemokine receptors. This partial clone was used as a probe to screen a human genomic library and three overlapping lambda clones were isolated. A restriction map of the clones was established and a 1.7 kb XbaI fragment was subcloned in pBS SK+ (Stratagene) and sequenced on both strands. The sequence was found to include the HOP 102 probe entirely, with 100% identity. This novel gene was named ChemR23 (GenBank Accession No. Y14838).

Amplification of coding sequence of ChemR23 resulted in a fragment of 1.1 kb. This fragment was subcloned into the pCDNA3 (Invitrogen) vector and sequenced on both strands.

Example 2: Purification of the natural ligand of ChemR23 and identification of TIG2.

Approximately one liter of a human ascitic fluid from a patient with ovarian cancer was prefiltered and then filtered successively through 0.45 and 0.22 $\mu$m Millex filters (Millipore).

In step 1, the ascite was directly loaded onto a C18 reverse-phase column (10 mm x 100 mm POROS 20 R2 beads, Applied Biosystems) pre-equilibrated with 5% $CH_3CN$/0.1% TFA at a flow-rate of 20 ml/min at room temperature. A 5-95% gradient of $CH_3CN$ in 0.1% TFA was then applied with a slope of 6%/min. 5-milliliter fractions were collected, and 20 $\mu$l of each fraction was set aside and assayed for [$Ca^{2+}$] transients in ChemR23-expressing CHO cells. In step 2, the active fractions (approx. 10 fractions eluting between 25 and 40% $CH_3CN$) were pooled, adjusted at pH 5, filtered through a 20 $\mu$m Millex filter (Millipore), diluted 3-fold in acetate buffer at pH 4.8 and then applied to a cation-exchange HPLC column (Polycat 9.6 mm x 250 mm, Vydac) pre-equilibrated with acetate buffer at pH 4.8 and 4°C. A 0-1M gradient of NaCl in acetate buffer at pH 4.8 was applied with 10%/min at a flow-rate of 4 ml/min. 1-milliliter fractions were collected and a 25 $\mu$l-aliquot from each fraction was used for the [$Ca^{2+}$] assay after desalting on a 10 kDa-cut-off membrane (Ultrafree, Millipore).

In step 3, the active fractions (eluted with approx. 700 mM NaCl) were pooled and desalted onto a 10 kDa-cut-off Ultrafree membrane to approx. 10 mM NaCl concentration. The eluates from distinct cation-exchange HPLC runs were pooled and loaded onto a second cation-exchange HPLC column (Polycat 2.1 mm x 250 mm, Vydac) pre-equilibrated with acetate buffer at pH 4.8 and 4°C. A 0-1 M gradient of NaCl in acetate buffer at pH 4.8 was applied at a flow-rate of 1 ml/min. with a slope of 2 %/min. 0.5-milliliter fractions were collected and a 20 µl-aliquot from each fraction was used for intracellular calcium assay after desalting onto a 10 kDa-cut-off Ultrafree membrane.

In step 4, the active fractions were pooled, diluted 8-fold with $H_2O$/0.1% $H_3PO_4$ and loaded onto an analytical C18 reverse-phase column (4.6 mm x 250 mm, Vydac) pre-equilibrated with 5% $CH_3CN$/0.1% $H_3PO_4$ at a flow-rate of 1 ml/min at room temperature. A 5-95% gradient of $CH_3CN$ in 0.1% $H_3PO_4$ was applied with a 0.3%/min. gradient between 25 and 40% of $CH_3CN$. Individual UV absorption peaks (214 nm) were collected manually, and approx. 5% from each fraction volume was assayed for biological activity.

In step 5, the active peaks (approximatively 28% $CH_3CN$) were diluted 6-fold with $H_2O$/0.1% TFA and directly loaded onto a second C18 reverse-phase column (1 mm x 50 mm, Vydac) pre-equilibrated with 5% $CH_3CN$/0.1% TFA at a flow-rate of 0.1 ml/min. at room temperature. A 5-95% gradient of $CH_3CN$ in 0.1% TFA was applied with a 0.3%/min. gradient between 30 and 45% of $CH_3CN$. The final peak was collected manually at 40% $CH_3CN$ and analysed by mass spectrometry. 800 ml of ovarian cancer ascites fluid yielded 50 fmoles of TIG2.

The active fraction was completely dried in a speed-vac and resuspended in 10 µl of 0.1M Tris at pH 8.7. After boiling the sample during 15 min at 95°C, the sample was incubated at 37°C overnight in the presence of 250 ng of modified trypsin (Promega). The digested sample was then purified by solid-phase extraction onto a C18 ZipTip (Millipore). The eluted sample (1.5 µl in 70% $CH_3CN$/0.1% TFA) was applied onto a MALDI target in the presence of 120 mg/ml dihydroxy-benzoic acid matrix and then analysed on a MALDI-Q-TOF prototype (Micromass). Direct monoisotopic mass fingerprinting allowed to identify 7 tryptic peptides, i.e. 63 amino acids with a sequence recovery of 38.7%.

Table 1: Sequences of Peptides found in monoisotopic mass fingerprinting

The two peptides indicated with an asterisk were microsequenced by MS/MS fragmentation. The position of the peptides is defined in comparison with TIG2 amino acid sequence (Seq ID N° 5)

| Residues # | Sequence | M + H |
|---|---|---|
| 72-78 | (K) LQQTSCR (K) [Seq Id. No. 13] | 835.41 |
| 81-88 | (R) DWKKPECK (V) [Seq. Id. No. 14] | 1033.51 |
| 29-39* | (R) GLQVALEEFHK (H) [Seq. Id. No. 15] | 1270.68 |
| 98-109 | (K) CLACIKLGSEDK (V) [Seq. Id. No. 16] | 1279.64 |
| 114-125* | (R) LVHCPIETQVLR (E) [Seq. Id No. 17] | 1407.78 |
| 28-39 | (R) RGLQVALEEFHK (H) [Seq. Id. No. 18] | 1426.78 |
| 126-137 | (R) EAEEHQETQCLR (V) [Seq. Id. No. 19] | 1472.64 |

Example 3: Cloning and recombinant expression of human TIG2.

In order to clone the TIG2 sequence (Fig. 6, GenBank Accession No. Q99969) a polymerase chain reaction (PCR) was performed on kidney cDNA (Clontech Laboratories). Primers were synthesized based upon the human TIG2 sequence and were as follows:

hTig2 fw: 5' CAGGAATTCAGCATGCGACGGCTGCTGA 3' SEQ ID NO: 20

hTig2 rv: 5' GCTCTAGATTAGCTGCGGGGCAGGGCCTT 3' SEQ ID NO: 21

Amplification was performed with Qiagen Taq polymerase in the conditions described by the supplier and with the following cycles: 3 min at 94°C, 35 cycles of 1 min at 94°C, 90 sec at 58°C and 90 sec at 72°C, followed by a final incubation of 10 min at 72°C. The amplification resulted in a fragment of 500 bp containing the entire coding sequence of the Tig2 gene. This fragment was subcloned into the vector pCDNA3 (Invitrogen) for DNA sequencing analysis.

Maxiprep (Quiagen) DNA was used in transient transfections of HEK293 cells expressing large T antigen (293T) and COS-7 cells using Fugene6 in 10 cm plates. In parallel, transfections were performed in the same cell lines with the expression vector alone (Mock transfected). 24 h after transfection, the medium was replaced by 9 ml DMEM-F12, 1% BSA, and 3ml of supernatant were collected each 24h for three days (48, 72 and 96h post transfection). CHO cells were transfected with the same plasmid and transfected cells were selected with G418. The activity of the conditioned medium was verified on ChemR23 expressing cells using the aequorin assay.

Example 4. Recombinant expression of TIG2 in yeasts.

The coding sequences of human and mouse TIG2 are amplified by PCR using the following primers (Two different primers are used for amplification of 5' end of human TIG2 to take into account the different predictions of the signal peptide of this protein):

| | | |
|---|---|---|
| mTig2f: | 5' TCTCTCGAGAAAAGAGAGGCTGAAGCTACACGTGGGACAGAGCCCGAA 3' | SEQ ID NO: 22 |
| hTig2af: | 5' TCTCTCGAGAAAAGAGAGGCTGAAGCTGGCGTCGCCGAGCTCACGGAA 3' | SEQ ID NO: 23 |
| hTig2bf: | 5' TCTCTCGAGAAAAGAGAGGCTGAAGCTGTGGGCGTCGCCGAGCTCACG 3' | SEQ ID NO: 24 |
| mTig2r: | 5' AGGGAATTCTTATTTGGTTCTCAGGGCCCT 3' | SEQ ID NO: 25 |
| hTig2r: | 5' AGGGAATTCTTAGCTGCGGGGCAGGGCCTT 3' | SEQ ID NO: 26 |

The amplified TIG2 sequences are cloned, sequenced and inserted in pPIC9K, a multicopy Pichia expression plasmid (InVitrogen) containing the signals directing secretion of expressed proteins. Following transformation, Pichia pastoris cells are selected using G418 antibiotic. After selection, 20 clones are analyzed for their expression and the clone with the highest expression is amplified for large scale expression in shaker flasks. The medium is collected, centrifuged and used for partial purification with a protocol derived from the one used for TIG2 initial purification (see above).

Example 5. Recombinant expression of chimaeric TIG2 fused with Secreted Alkaline Phosphatase (SEAP).

The coding sequences of mouse and human TIG2 are amplified by PCR, cloned and sequenced. PCR and sequencing primers are as follows:

| | | |
|---|---|---|
| mTig2f: | CAGGAATTCGCCATGAAGTGCTTGCTGA | (SEQ ID NO: 27) |
| hTig2f: | CAGGAATTCAGCATGCGACGGCTGCTGA | (SEQ ID NO: 28) |
| mTig2r: | GCTCTAGATTTGGTTCTCAGGGCCCTGGA | (SEQ ID NO: 29) |
| hTig2r: | GCTCTAGAGCTGCGGGGCAGGGCCTTGGA | (SEQ ID NO: 30) |

The cloned TIG2 sequences are then subcloned into the mammalian bicistronic expression vector, pEFIN, to obtain a fusion protein with TIG2 linked at its carboxy terminal end to secreted alkaline phosphatase, tagged with six histidine residues (His6). Mammalian cells, including COS-7, HEK-293 expressing the large T antigen (293 T) and CHO-K1 cells, are transfected with this plasmid using Fugene 6™ and incubated for 3-4 days in complete Ham's F12 medium (Nutrient Mixture Ham's F12 (Life Technologies) containing 10% fetal bovine serum; 100 IU/ml penicillin, 100 μg/ml streptomycin and 2.5 μg/ml fungizone (Amphotericin B). The supernatant containing TIG2-SEAP-His6 is collected after centrifugation, filtered (0.45 μm) and stored at 4°C after adding 20 mM Hepes (pH 7.4) and 0.02% sodium azide.

For one-step affinity purification of the TIG2 fusion protein, the supernatant is applied to 1 ml of Hisbond resin (Qiagen). After washing, bound TIG2-SEAP-His6 is eluted with a gradient of imidazol. The concentration of isolated TIG2-SEAP-His6 is determined by a sandwich type enzyme-linked immunosorbent assay. Briefly microtiter plates are coated with anti-placental alkaline phosphatase antibody. After blocking with 1 mg/ml bovine serum albumin (BSA) in phosphate buffered saline, the samples are titrated and incubated for 1 h at room temperature. After washing, plates are incubated with biotinylated rabbit anti-placental alkaline phosphatase diluted 1:500 for 1 h at room temperature, washed again, and incubated with peroxidase-conjugated streptavidin for 30 min. After washing, bound peroxidase is reacted with 3, 3',5,5'-tetramethylbenzidine. The reaction is stopped by adding 1 N $H_2SO_4$, and absorbance at 450 nm is measured. Alkaline phosphatase activity is determined by a chemiluminescent assay using the Great Escape™ detection kit (Clontech). Purified placental alkaline phophatase is used to generate a standard curve. The enzymatic activity is expressed as relative light units/sec.

Example 6: Functional assay for ChemR23.

ChemR23 expressing clones have been obtained by transfection of CHO-K1 cells to coexpressing mitochondrial apoaequorin and Gα16, limiting dilution and selection by northern blotting. Positive clones were used for screening with human ovarian cancer ascites extracts prepared as described above. A functional assay based on the luminescence of mitochondrial aequorin intracellular $Ca^{2+}$ release (Stables et al., 1997, Anal. Biochem. 252:115-126; incorporated herein by reference) was performed as described (Detheux et al., 2000, J. Exp. Med., 192 1501-1508; incorporated herein by reference). Briefly, cells were collected from plates in PBS containing 5 mM EDTA, pelleted and resuspended at 5 x $10^6$ cells/ml in DMEM-F12 medium. Cells were incubated with 5 μM Coelenterazine H (Molecular Probes) for 4 hours at room temperature. Cells were then washed in DMEM-F12 medium and resuspended at a concentration of 0.5 x $10^6$ cells/ml. Cells were then mixed with test agonist peptides or plates containing tissue extracts and the light emission was recorded for 30 sec using a Microlumat luminometer (Perkin Elmer). Results are expressed as Relative Light Units (RLU).

Example 7: Activation of cells expressing ChemR23 by recombinant TIG2.

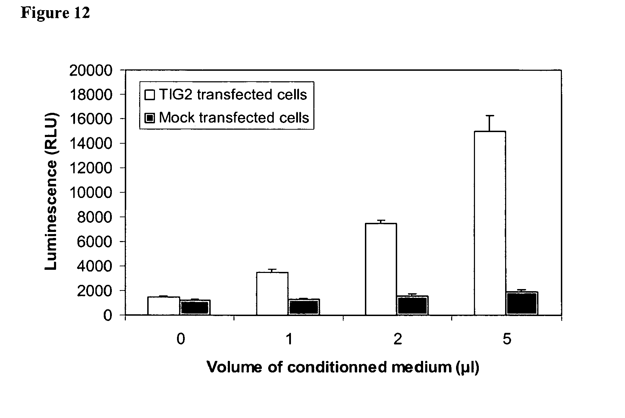

The conditioned medium of COS-7, CHO-K1 and 293 T cells transfected with a mammalian TIG2 expression vector system (pCDNA-TIG2) or pcDNA3 alone, was collected and used for aequorin assays on CHO cells expressing ChemR23. Results are shown in Figure 12. Increasing amounts of conditioned supernatant resulted in an increase in luminescence in aequorin system cells expressing ChemR23.

Example 8: Production of antibodies specific for ChemR23.

Antibodies directed against ChemR23 were produced by repeated injections of plasmids encoding ChemR23 into mice. Sera were collected starting after the second injection and the titre and specificity of the antibodies was assessed by flow cytofluorometry with CHO-K1 cells transfected with the ChemR23 cDNA and CHO-K1 cells transfected with the cDNA of unrelated GPCR cDNA. Several sera were positive and were used for immunohistochemistry and other related applications, including flow cytometry analysis of human primary cells.

Monoclonal antibodies were obtained from immune mice by standard hybridoma technology using the NSO murine myeloma cell line as immortal partner. Supernatants were tested for anti ChemR23 antibody activity using the test used for assessing the antisera. Cells from the positive wells were expanded and frozen and the supernatants collected.

Figure 13 shows the results of experiments to characterize the antibodies raised against ChemR23. A mixture of recombinant cells made up of 2/3 recombinant ChemR23 CHO cells and 1/3 mock-transfected CHO cells (negative control) was reacted with either a supernatant of cells expressing the anti ChemR23 5C 1H2 monoclonal antibody (thick line) or a supernatant from cells with no known antibody activity (thin line, grey filling). After staining with FITC labeled anti mouse Ig these preparations were analyzed by flow cytofluorometry. Results are displayed as a histogram of the number of cells (Events axis) expressing a given fluorescence (FL1-H axis). Monoclonal 5C 1H2 allowed the discrimination of the ChemR23 recombinant sub-population of cells from the negative control cells, as evidenced by the relative proportions of both types of cells. The background fluorescence of the assay is given by the second staining (grey filling).

Example 9. Binding displacement assay.

For displacement experiments, ChemR23-CHO-K1 cells (25,000 cells/tube) are incubated for 90 min. at 27°C with 1 nM of SEAP-HIS6 or TIG2-SEAP-HIS6 in the presence of increasing concentrations of unlabeled TIG2 in 250 μl of binding buffer (50 mM Hepes pH 7.4; 1 mM Ca $Cl_2$; 0.5% Bovine Serum Albumin (BSA) Fatty Acid-Free; 5 mM $MgCl_2$). For saturation experiments, ChemR23-CHO-K1 cells (25,000 cells/tube) are incubated for 90 min at 27°C with increasing concentrations of TIG2-SEAP-HIS6 in the presence or absence of 1 μM unlabeled TIG2. After incubation, cells are washed 5 times and lysed in 50 μl of 10 mM Tris-HCl (pH 8.0), 1% triton X100. Samples are heated at 65°C for 10 min to inactivate cellular phosphatases. Lysates are collected by centrifugation, and alkaline phosphatase activity in 25 μl of lysate is determined by the chemiluminescence assay described above.

Example 10. Tissue distribution of TIG2 and ChemR23

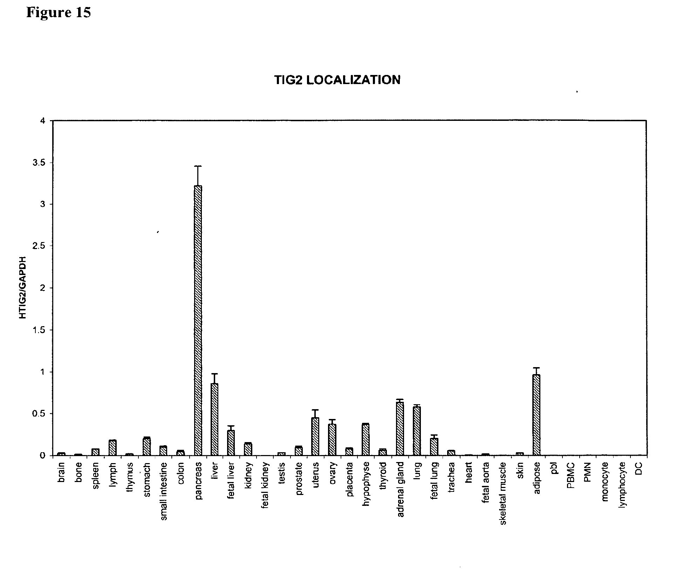

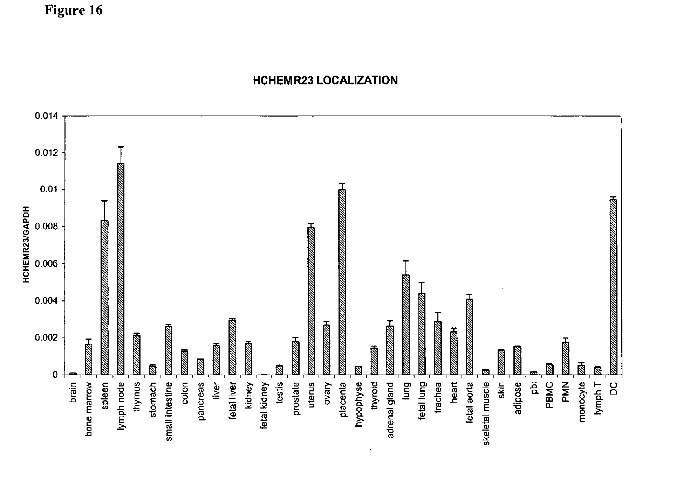

Semi-quantitative RT-PCR was performed using gene-specific primers to hTIG2 and ChemR23 on polyA+ RNA and total RNA from various human tissues (CLONTECH and Ambion). Briefly, total RNA from blood cells were prepared with Rneasy Mini Kit (Qiagen). The hTIG2 primers were forward (5'-GCAGACAAGCTGCCGGA-3'; SEQ ID NO: 37), TaqMan probe (5'-AACCCGAGTGCAAAGTCAGGCCC-3'; SEQ ID NO: 38), and reverse (5'-AGTTTGATGCAGGCCAGGC-3'; SEQ ID NO: 39). The hChemR23 primers were forward (5'-GTCCCAGAACCACCGCAG-3'; SEQ ID NO: 40), TaqMan probe (5'-TTCGCCTGGCTTACATGGCCTGC-3'; SEQ ID NO: 41), and reverse (5'-AAGAAAGCCAGGACCCAGATG-3'; SEQ ID NO: 42). Primers designed to the housekeeping gene GAPDH Forward (5'-GAAGGTGAAGGTCGGAGTC-3'; SEQ ID NO: 43), TaqMan pobe (5'- AGCTCTCCCGCCGGCCTCTG-3'; SEQ ID NO: 44), and reverse (5'-GAAGATGGTGATGGGATTTC-3'; SEQ ID NO: 45) were used to produced reference mRNA profiles. The distribution of hTIG2 and ChemR23 in various tissues is shown in Figures 15 and 16, respectively. The level of expression of hTIG2 or ChemR23 are expressed as a ratio of hTIG2 or ChemR23 to GAPDH reference mRNA expression.

Example 11. Identification of a truncated human TIG2 polypeptide as ligand for ChemR23.

The truncated human TIG2 polypeptide was isolated from human tumoral ascites fluid from a patient with ovarian cancer. The procedure used for said isolation is disclosed in example 2 of the present patent application; except for the fact that after peptide sequencing a polypeptide was revealed as represented by SEQ ID NO:46.

The nucleotide sequence encoding said truncated polypeptide was isolated according to a method as disclosed in example 3. Recombinant expression of the truncated TIG2 in yeast and mammalian cells was performed as disclosed in examples 4 and 5.

A functional assay revealed that both TIG2 polypeptides as represented by SEQ ID NO:8 and represented by SEQ ID NO:46 are ligands for ChemR23. The method followed for said assay is as disclosed in example 6.

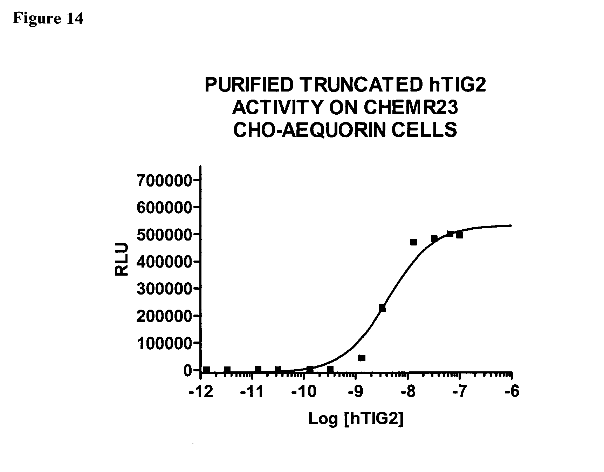

Figure 14 shows the concentration response curve for the truncated hTIG2 peptide (SEQ ID NO: 46) to ChemR23 expressed in CHO cells. The assay was carried out as described in the preceeding paragraph. As shown in the figure, the truncated hTIG2 molecule activates ChemR23 with an $EC_{50}$ of 4.27 nM. Results are expressed as Relative Light Units (RLU). Similar activation studies, binding displacement assays and antibody production are being performed for the truncated TIG2 peptide as performed for the full length TIG2 polypeptide as disclosed in examples 7, 8 and 9.

The tissue distribution of the truncated TIG2 polypeptide is being studied in order to determine if this is distinct from the distribution of the full length TIG2 polypeptide. This may reveal a specific function of said truncated form in respect of the full length form.

## Claims

1. A method of diagnosing in vitro a disease or disorder **characterized by** the dysregulation of ChemR23 signalling, said method comprising:

   (a) a ChemR23 polypeptide present in a tissue sample with a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide;
   (b) detecting binding of said TIG2 polypeptide to said tissue sample; and
   (c) comparing the binding detected in step (b) with a standard, wherein a difference in binding relative to said standard is diagnostic of the disease or disorder **characterized by** the dysregulation of ChemR23.

2. A method of diagnosing in vitro a disease or disorder **characterized by** the dysregulation of ChemR23 signalling, said method comprising:

   (a) contacting a ChemR23 polypeptide present in a tissue sample with a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide;
   (b) detecting a signalling activity of said ChemR23 polypeptide; and
   (c) comparing the signalling activity detected in step (b) with a standard, wherein a difference in signalling activity relative to said standard is diagnostic of a disease or disorder **characterized by** the dysregulation of ChemR23.

3. A method of diagnosing in vitro a disease or disorder **characterized by** the_dysregulation of TIG2 activity, said method comprising:

   (a) contacting a TIG2 polypeptide present in a tissue sample with a ChemR23 polypeptide;
   (b) detecting binding of said ChemR23 polypeptide to said TIG2 polypeptide; and
   (c) comparing the binding detected in step (b) with a standard, wherein a difference in binding relative to said standard is diagnostic of a disease or disorder **characterized by** the dysregulation of TIG2 activity.

4. A method of diagnosing in vitro a disease or disorder **characterized by** the dysregulation of TIG2 activity, said method comprising:

   (a) contacting a TIG2 polypeptide present in a tissue sample with a ChemR23 polypeptide;
   (b) detecting a signalling activity of said ChemR23 polypeptide; and
   (c) comparing the signalling activity detected in step (b) with a standard, wherein a difference in signalling activity relative to said standard is diagnostic of a disease or disorder **characterized by** the dysregulation of TIG2 activity.

5. A method of diagnosing in vitro a disease or disorder **characterized by** the dysregulation of ChemR23 signalling and/or TIG2 activity, said method comprising :

   (a) contacting a tissue sample with an antibody specific for a ChemR23 polypeptide and an antibody specific for a TIG2 polypeptide;
   (b) detecting binding of said antibodies to said tissue sample; and
   (c) comparing the binding detected in step (b) with a standard, wherein a difference in the binding of either antibody or both, relative to said standard, is diagnostic of a disease or disorder **characterized by** the dysregulation of ChemR23 and/or TIG2 activity.

6. A method of diagnosing in vitro a disease or disorder **characterized by** the_dysregulation of ChemR23 signalling and/or TIG2 activity, said method comprising :

(a) isolating nucleic acid from a tissue sample;
(b) amplifying a TIG2 polynucleotide and a ChemR23 polynucleotide, using said nucleic acid as a template; and
(c) comparing the amount of amplified TIG2 polynucleotide and ChemR23 polynucleotide produced in step (b) with a standard, wherein a difference in said amount of amplified TIG2 polynucleotide and/or ChemR23 polynucleotide relative to said standard is diagnostic of a disease or disorder **characterized by** the dysregulation of ChemR23 signalling and/or TIG2 activity.

**7.** A method of diagnosing in vitro a disease or disorder **characterized by** the dysregulation of ChemR23 signalling and/or TIG2 activity, said method comprising :

(a) isolating nucleic acid from a tissue sample;
(b) amplifying a TIG2 polynucleotide and a ChemR23 polynucleotide, using said nucleic acid as a template; and
(c) comparing the sequence of said amplified TIG2 polynucleotide and ChemR23 polunucleotide produced in step (b) with a standard, wherein a difference in said sequence relative to said standard is diagnostic of a disease or disorder **characterized by** the dysregulation of ChemR23 signalling or TIG2 activity.

**8.** The method according to any of claims 1 to 7, wherein said standard comprises a sample taken from an individual which is not affected by a disease or disorder **characterized by** dysregulation of ChemR23 signalling and/or TIG2 activity.

**9.** The method according to any of claims 1 to 7, wherein said standard comprises a recombinant cell expressing said ChemR23 or said truncated TIG2 polypeptide.

**10.** The method according to claim 3 or 4, wherein said standard comprises an isolated truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide, or, a membrane comprising ChemR23.

**11.** The method according to claim 7 wherein the step of comparing the sequence comprises minisequencing.

**12.** The method according to claim 7 wherein said standard is SEQ ID NO: 7.

**13.** The method according to claim 7 wherein said standard is SEQ ID NO: 1.

**14.** The method according to claim 6 wherein the step of comparing the amount is performed on a microarray.

**15.** The method according to claim 7 wherein the step of comparing the sequence is performed on a microarray.

**16.** A kit for the diagnosis of a disease or disorder **characterized by** dysregulation of ChemR23 signalling, said kit comprising a cell transformed with a polynucleotide encoding a ChemR23 polypeptide, a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide and packaging materials therefore.

**17.** Use of a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the preparation of a medicament.

**18.** Use of a polynucleotide coding for a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the preparation of a medicament.

**19.** Use of a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the production of a kit.

**20.** Use of a polynucleotide coding for a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the production of a kit.

**21.** Use according to claim 19 or 20, wherein the kit is used for the diagnosis of a disease **characterized by** the dysregulation of ChemR23 signalling.

**22.** Use of a truncated TIG2 polypeptide, represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the preparation of a medicament for modulating in vivo the activity of a ChemR23 polypeptide in a cell.

**23.** Use of a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the preparation of a medicament for the treatment of a ChemR23-related disease or a ChemR23-related disorder.

**24.** Use of a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the production of an antibody.

**25.** An antibody specific for a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide.

**26.** Use of a truncated TIG2 polypeptide, represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide for the production of a kit for screening agents that modulate the signalling of ChemR23.

**27.** Use of a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2, for the production of a composition comprising an isolated ChemR23 polypeptide and/or an isolated TIG2 polypeptide.

**28.** A composition comprising an isolated truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide.

**29.** The method, kit, use, antibody or composition according to any of claims 1 to 28, wherein said TIG2 polypeptide comprises additional sequences forming a TIG2 fusion protein.

**30.** A method, use, composition or kit according to claim 29, wherein said additional sequences may be chosen from the group consisting of glutathione-S-transferase (GST), maltose binding protein, alkaline phosphatase, thioredoxin, green fluorescent protein (GFP), histidine tags (e.g., 6X or greater His), or epitope tags (e.g., Myc tag, FLAG tag) sequences.

**31.** Use of a polynucleotide encoding a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide or a polynucleotide represented by SEQ ID NO:7 in a method, kit, use, antibody or composition according to any of claims 1, 2, 6-9 and 11-30.

**32.** Use of a polynucleotide encoding a truncated TIG2 polypeptide represented by SEQ ID NO:46 or a TIG2 polypeptide comprising additions, insertions, deletions or substitutions relative to said truncated TIG2 polypeptide but retaining at least 50% of the binding activity and level of signalling activation of said truncated TIG2 polypeptide-or a polynucleotide represented by SEQ ID NO:7 for the preparation of a ligand for ChemR23.

**Figure 1**

1 M E D E D Y N T S I S Y G D E 15
175 ATG GAG GAT GAA GAT TAC AAC ACT TCC ATC AGT TAC GGT GAT GAA 219

16 Y P D Y L D S I V V L E D L S 30
220 TAC CCT GAT TAT TTA GAC TCC ATT GTG GTT TTG GAG GAC TTA TCC 264

31 P L E A R V T R I F L V V V Y 45
265 CCC TTG GAA GCC AGG GTG ACC AGG ATC TTC CTG GTG GTG GTC TAC 309

46 S I V C F L G I L G N G L V I 60
310 AGC ATC GTC TGC TTC CTC GGG ATT CTG GGC AAT GGT CTG GTG ATC 354

61 I I A T F K M K K T V N M V W 75
355 ATC ATT GCC ACC TTC AAG ATG AAG AAG ACA GTG AAC ATG GTC TGG 399

76 F L N L A V A D F L F N V F L 90
400 TTC CTC AAC CTG GCA GTG GCA GAT TTC CTG TTC AAC GTC TTC CTC 444

91 P I H I T Y A A M D Y H W V F 105
445 CCA ATC CAT ATC ACC TAT GCC GCC ATG GAC TAC CAC TGG GTT TTC 489

106 G T A M C K I S N F L L I H N 120
490 GGG ACA GCC ATG TGC AAG ATC AGC AAC TTC CTT CTC ATC CAC AAC 534

121 M F T S V F L L T I I S S D R 135
535 ATG TTC ACC AGC GTC TTC CTG CTG ACC ATC ATC AGC TCT GAC CGC 579

136 C I S V L L P V W S Q N H R S 150
580 TGC ATC TCT GTG CTC CTC CCT GTC TGG TCC CAG AAC CAC CGC AGC 624

151 V R L A Y M A C M V I W V L A 165
625 GTT CGC CTG GCT TAC ATG GCC TGC ATG GTC ATC TGG GTC CTG GCT 669

166 F F L S S P S L V F R D T A N 180
670 TTC TTC TTG AGT TCC CCA TCT CTC GTC TTC CGG GAC ACA GCC AAC 714

181 L H G K I S C F N N F S L S T 195
715 CTG CAT GGG AAA ATA TCC TGC TTC AAC AAC TTC AGC CTG TCC ACA 759

196 P G S S S W P T H S Q M D P V 210
760 CCT GGG TCT TCC TCG TGG CCC ACT CAC TCC CAA ATG GAC CCT GTG 804

211 G Y S R H M V V T V T R F L C 225
805 GGG TAT AGC CGG CAC ATG GTG GTG ACT GTC ACC CGC TTC CTC TGT 849

226 G F L V P V L I I T A C Y L T 240
850 GGC TTC CTG GTC CCA GTC CTC ATC ATC ACA GCT TGC TAC CTC ACC 894

241 I V C K L Q R N R L A K T K K 255
895 ATC GTC TGC AAA CTG CAG CGC AAC CGC CTG GCC AAG ACC AAG AAG 939

256 P F K I I V T I I I T F F L C 270
940 CCC TTC AAG ATT ATT GTG ACC ATC ATC ATT ACC TTC TTC CTC TGC 984

**Figure 1 – continued**

```
 271  W   C   P   Y   H   T   L   N   L   L   E   L   H   H   T      285
 985 TGG TGC CCC TAC CAC ACA CTC AAC CTC CTA GAG CTC CAC CAC ACT    1029

 286  A   M   P   G   S   V   F   S   L   G   L   P   L   A   T      300
1030 GCC ATG CCT GGC TCT GTC TTC AGC CTG GGT TTG CCC CTG GCC ACT    1074

 301  A   L   A   I   A   N   S   C   M   N   P   I   L   Y   V      315
1075 GCC CTT GCC ATT GCC AAC AGC TGC ATG AAC CCC ATT CTG TAT GTT    1119

 316  F   M   G   Q   D   F   K   K   F   K   V   A   L   F   S      330
1120 TTC ATG GGT CAG GAC TTC AAG AAG TTC AAG GTG GCC CTC TTC TCT    1164

 331  R   L   V   N   A   L   S   E   D   T   G   H   S   S   Y      345
1165 CGC CTG GTC AAT GCT CTA AGT GAA GAT ACA GGC CAC TCT TCC TAC    1209

 346  P   S   H   R   S   F   T   K   M   S   S   M   N   E   R      360
1210 CCC AGC CAT AGA AGC TTT ACC AAG ATG TCA TCA ATG AAT GAG AGG    1254

 361  T   S   M   N   E   R   E   T   G   M   L   *                  372
1255 ACT TCT ATG AAT GAG AGG GAG ACC GGC ATG CTT TGA                1290
```

**Figure 2**

MEDEDY**NTS**ISYGDEYPDYLDSIVVLEDLSPLEARVTRIFLVVVYSIVCFLGILGNGLVIIIATFKMKK

TVNMVWFLNLAVADFLFNVFLPIHITYAAMDYHWVFGTAMCKISNFLLIHNMFTSVFLLTIISSDRCIS

VLLPVWSQNHRSVRLAYMACMVIWVLAFFLSSPSLVFRDTANLHGKISCFNNFSLSTPGSSSWPTHSQM

DPVGYSRHMVVTVTRFLCGFLVPVLIITACYLTIVCKLQRNRLAKTKKPFKIIVTIIITFFLCWCPYHT

LNLLELHHTAMPGSVFSLGLPLATALAIANSCMNPILYVFMGQDFKKFKVALFSRLVNALSEDTGHSSY

P*SHR*SFTKMSSMNERTSMNERETGML

# Figure 3

```
   1 M   E   Y   D   A   Y   N   D   S   G   I   Y   D   D   E     15
 265 ATG GAG TAC GAC GCT TAC AAC GAC TCC GGC ATC TAT GAT GAT GAG    309

  16 Y   S   D   G   F   G   Y   F   V   D   L   E   E   A   S     30
 310 TAC TCT GAT GGC TTT GGC TAC TTT GTG GAC TTG GAG GAG GCG AGT    354

  31 P   W   E   A   K   V   A   P   V   F   L   V   V   I   Y     45
 355 CCG TGG GAG GCC AAG GTG GCC CCG GTC TTC CTG GTG GTG ATC TAC    399

  46 S   L   V   C   F   L   G   L   L   G   N   G   L   V   I     60
 400 AGC TTG GTG TGC TTC CTC GGT CTC CTA GGC AAC GGC CTG GTG ATT    444

  61 V   I   A   T   F   K   M   K   K   T   V   N   T   V   W     75
 445 GTC ATC GCC ACC TTC AAG ATG AAG AAG ACC GTG AAC ACT GTG TGG    489

  76 F   V   N   L   A   V   A   D   F   L   F   N   I   F   L     90
 490 TTT GTC AAC CTG GCT GTG GCC GAC TTC CTG TTC AAC ATC TTT TTG    534

  91 P   M   H   I   T   Y   A   A   M   D   Y   H   W   V   F    105
 535 CCG ATG CAC ATC ACC TAC GCG GCC ATG GAC TAC CAC TGG GTG TTC    579

 106 G   K   A   M   C   K   I   S   N   F   L   L   S   H   N    120
 580 GGG AAG GCC ATG TGC AAG ATC AGC AAC TTC TTG CTC AGC CAC AAC    624

 121 M   Y   T   S   V   F   L   L   T   V   I   S   F   D   R    135
 625 ATG TAC ACC AGC GTC TTC CTG CTG ACT GTC ATC AGC TTT GAC CGC    669

 136 C   I   S   V   L   L   P   V   W   S   Q   N   H   R   S    150
 670 TGC ATC TCC GTG CTG CTC CCC GTC TGG TCC CAG AAC CAC CGC AGC    714

 151 I   R   L   A   Y   M   T   C   S   A   V   W   V   L   A    165
 715 ATC CGC CTG GCC TAC ATG ACC TGC TCG GCC GTC TGG GTC CTG GCT    759

 166 F   F   L   S   S   P   S   L   V   F   R   D   T   A   N    180
 760 TTC TTC TTG AGC TCC CCG TCC CTT GTC TTC CGG GAC ACC GCC AAC    804

 181 I   H   G   K   I   T   C   F   N   N   F   S   L   A   A    195
 805 ATT CAT GGG AAG ATA ACC TGC TTC AAC AAC TTC AGC TTG GCC GCG    849

 196 P   E   S   S   P   H   P   A   H   S   Q   V   V   S   T    210
 850 CCT GAG TCC TCC CCA CAT CCC GCC CAC TCG CAA GTA GTT TCC ACA    894

 211 G   Y   S   R   H   V   A   V   T   V   T   R   F   L   C    225
 895 GGG TAC AGC AGA CAC GTG GCG GTC ACT GTC ACC CGC TTC CTT TGC    939

 226 G   F   L   I   P   V   F   I   I   T   A   C   Y   L   T    240
 940 GGC TTC CTG ATC CCC GTC TTC ATC ATC ACG GCC TGC TAC CTT ACC    984

 241 I   V   F   K   L   Q   R   N   R   L   A   K   N   K   K    255
 985 ATC GTC TTC AAG CTG CAG CGC AAC CGC CTG GCC AAG AAC AAG AAG   1029

 256 P   F   K   I   I   I   T   I   I   I   T   F   F   L   C    270
1030 CCC TTC AAG ATC ATC ATC ACC ATC ATC ATC ACC TTC TTC CTC TGC   1074

 271 W   C   P   Y   H   T   L   Y   L   L   E   L   H   H   T    285
1075 TGG TGC CCC TAC CAC ACC CTC TAC CTG CTG GAG CTC CAC CAC ACA   1119

 286 A   V   P   S   S   V   F   S   L   G   L   P   L   A   T    300
1120 GCT GTG CCA AGC TCT GTC TTC AGC CTG GGG CTA CCC CTG GCC ACG   1164
```

**Figure 3 – continued**

```
 301 A   V   A   I   A   N   S   C   M   N   P   I   L   Y   V      315
1165 GCC GTC GCC ATC GCC AAC AGC TGC ATG AAC CCC ATT CTG TAC GTC   1209

 316 F   M   G   H   D   F   R   K   F   K   V   A   L   F   S      330
1210 TTC ATG GGC CAC GAC TTC AGA AAA TTC AAG GTG GCC CTC TTC TCC   1254

 331 R   L   A   N   A   L   S   E   D   T   G   P   S   S   Y      345
1255 CGC CTG GCC AAC GCC CTG AGT GAG GAC ACA GGC CCC TCC TCC TAC   1299

 346 P   S   H   R   S   F   T   K   M   S   S   L   N   E   K      360
1300 CCC AGT CAC AGG AGC TTC ACC AAG ATG TCG TCT TTG AAT GAG AAG   1344

 361 A   S   V   N   E   K   E   T   S   T   L   *                  372
1345 GCT TCG GTG AAT GAG AAG GAG ACC AGT ACC CTC TGA               1380
```

# Figure 4

```
  1 M   E   Y   E   G   Y   N   D   S   S   I   Y   G   E   E      15
  1 ATG GAG TAC GAG GGT TAC AAC GAC TCC AGC ATC TAC GGT GAG GAG     45

 16 Y   S   D   G   S   D   Y   I   V   D   L   E   E   A   G      30
 46 TAT TCT GAC GGC TCG GAC TAC ATC GTG GAC TTG GAG GAG GCG GGT     90

 31 P   L   E   A   K   V   A   E   V   F   L   V   V   I   Y      45
 91 CCA CTG GAG GCC AAG GTG GCC GAG GTC TTC CTG GTG GTA ATC TAC    135

 46 S   L   V   C   F   L   G   I   L   G   N   G   L   V   I      60
136 AGC TTG GTG TGC TTC CTC GGG ATC CTA GGC AAT GGC CTG GTG ATT    180

 61 V   I   A   T   F   K   M   K   K   T   V   N   T   V   W      75
181 GTC ATC GCC ACC TTC AAG ATG AAG AAG ACG GTG AAC ACC GTG TGG    225

 76 F   V   N   L   A   V   A   D   F   L   F   N   I   F   L      90
226 TTT GTC AAC CTG GCC GTG GCT GAC TTC CTG TTC AAC ATC TTC TTG    270

 91 P   I   H   I   T   Y   A   A   M   D   Y   H   W   V   F     105
271 CCC ATC CAC ATC ACC TAT GCC GCT ATG GAC TAC CAC TGG GTG TTC    315

106 G   K   A   M   C   K   I   S   S   F   L   L   S   H   N     120
316 GGG AAA GCC ATG TGC AAG ATT AGT AGC TTT CTG CTA AGC CAC AAC    360

121 M   Y   T   S   V   F   L   L   T   V   I   S   F   D   R     135
361 ATG TAC ACC AGC GTC TTC CTG CTC ACT GTC ATC AGC TTC GAC CGC    405

136 C   I   S   V   L   L   P   V   W   S   Q   N   H   R   S     150
406 TGC ATC TCC GTG CTC CTC CCC GTC TGG TCC CAG AAC CAC CGC AGC    450

151 V   R   L   A   Y   M   T   C   V   V   V   W   V   W   L     165
451 GTG CGT CTG GCC TAC ATG ACC TGC GTG GTT GTC TGG GTC TGG CTT    495

166 S   S   E   S   P   P   S   L   V   F   G   H   V   S   T     180
496 TCT TCT GAG TCT CCC CCG TCC CTC GTC TTC GGA CAC GTC AGC ACC    540

181 S   H   G   K   I   T   C   F   N   N   F   S   L   A   A     195
541 AGC CAC GGG AAG ATA ACC TGC TTC AAC AAC TTC AGC CTG GCG GCG    585

196 P   E   P   F   S   H   S   T   H   P   R   T   D   P   V     210
586 CCC GAG CCT TTC TCT CAT TCC ACC CAC CCG CGA ACA GAC CCG GTA    630

211 G   Y   S   R   H   V   A   V   T   V   T   R   F   L   C     225
631 GGG TAC AGC AGA CAT GTG GCG GTC ACC GTC ACC CGC TTC CTC TGT    675

226 G   F   L   I   P   V   F   I   I   T   A   C   Y   L   T     240
676 GGC TTC CTG ATC CCC GTC TTC ATC ATC ACG GCC TGT TAC CTC ACC    720

241 I   V   F   K   L   Q   R   N   R   Q   A   K   T   K   K     255
721 ATC GTC TTC AAG TTG CAG CGC AAC CGC CAG GCC AAG ACC AAG AAG    765

256 P   F   K   I   I   I   T   I   I   I   T   F   F   L   C     270
766 CCC TTC AAG ATC ATC ATC ACC ATC ATC ATC ACC TTC TTC CTC TGC    810

271 W   C   P   Y   H   T   L   Y   L   L   E   L   H   H   T     285
811 TGG TGC CCC TAC CAC ACA CTC TAC CTG CTG GAG CTC CAC CAC ACG    855

286 A   V   P   A   S   V   F   S   L   G   L   P   L   A   T     300
856 GCT GTG CCA GCC TCT GTC TTC AGC CTG GGA CTG CCC CTG GCC ACA    900
```

**Figure 4 - continued**

```
 301 A   V   A   I   A   N   S   C   M   N   P   I   L   Y   V      315
 901 GCC GTC GCC ATC GCC AAC AGC TGT ATG AAC CCC ATC CTG TAC GTC    945

 316 F   M   G   H   D   F   K   K   F   K   V   A   L   F   S      330
 946 TTC ATG GGC CAC GAC TTC AAA AAA TTC AAG GTG GCC CTT TTC TCC    990

 331 R   L   V   N   A   L   S   E   D   T   G   P   S   S   Y      345
 991 CGC CTG GTG AAT GCC CTG AGC GAG GAC ACA GGA CCC TCC TCC TAC   1035

 346 P   S   H   R   S   F   T   K   M   S   S   L   I   E   K      360
1036 CCC AGT CAC AGG AGC TTC ACC AAG ATG TCC TCA TTG ATT GAG AAG   1080

 361 A   S   V   N   E   K   E   T   S   T   L   *                  372
1081 GCT TCA GTG AAT GAG AAA GAG ACC AGC ACC CTC TGA               1116
```

Figure 5

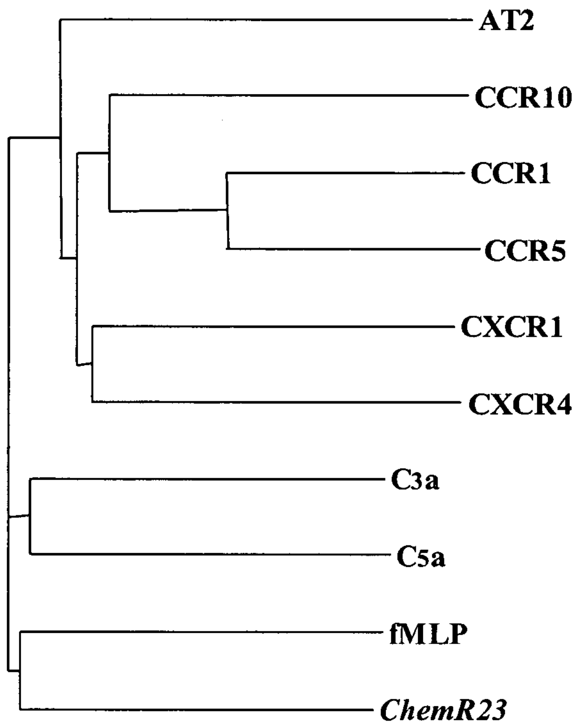

**Figure 6**

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 97 | ATG | CGA | CGG | CTG | CTG | ATC | CCT | CTG | GCC | CTG | TGG | CTG | GGT | GCG | GTG | 141 |
| 16 | G | V | G | V | A | E | L | T | E | A | Q | R | R | G | L | 30 |
| 142 | GGC | GTG | GGC | GTC | GCC | GAG | CTC | ACG | GAA | GCC | CAG | CGC | CGG | GGC | CTG | 186 |
| 31 | Q | V | A | L | E | E | F | H | K | H | P | P | V | Q | W | 45 |
| 187 | CAG | GTG | GCC | CTG | GAG | GAA | TTT | CAC | AAG | CAC | CCG | CCC | GTG | CAG | TGG | 231 |
| 46 | A | F | Q | E | T | S | V | E | S | A | V | D | T | P | F | 60 |
| 232 | GCC | TTC | CAG | GAG | ACC | AGT | GTG | GAG | AGC | GCC | GTG | GAC | ACG | CCC | TTC | 276 |
| 61 | P | A | G | I | F | V | R | L | E | F | K | L | Q | Q | T | 75 |
| 277 | CCA | GCT | GGA | ATA | TTT | GTG | AGG | CTG | GAA | TTT | AAG | CTG | CAG | CAG | ACA | 321 |
| 76 | S | C | R | K | R | D | W | K | K | P | E | C | K | V | R | 90 |
| 322 | AGC | TGC | CGG | AAG | AGG | GAC | TGG | AAG | AAA | CCC | GAG | TGC | AAA | GTC | AGG | 366 |
| 91 | P | N | G | R | K | R | K | C | L | A | C | I | K | L | G | 105 |
| 367 | CCC | AAT | GGG | AGG | AAA | CGG | AAA | TGC | CTG | GCC | TGC | ATC | AAA | CTG | GGC | 411 |
| 106 | S | E | D | K | V | L | G | R | L | V | H | C | P | I | E | 120 |
| 412 | TCT | GAG | GAC | AAA | GTT | CTG | GGC | CGG | TTG | GTC | CAC | TGC | CCC | ATA | GAG | 456 |
| 121 | T | Q | V | L | R | E | A | E | E | H | Q | E | T | Q | C | 135 |
| 457 | ACC | CAA | GTT | CTG | CGG | GAG | GCT | GAG | GAG | CAC | CAG | GAG | ACC | CAG | TGC | 501 |
| 136 | L | R | V | Q | R | A | G | E | D | P | H | S | F | Y | F | 150 |
| 502 | CTC | AGG | GTG | CAG | CGG | GCT | GGT | GAG | GAC | CCC | CAC | AGC | TTC | TAC | TTC | 546 |
| 151 | P | G | Q | F | A | F | S | K | A | L | P | R | S | * | | 164 |
| 547 | CCT | GGA | CAG | TTC | GCC | TTC | TCC | AAG | GCC | CTG | CCC | CGC | AGC | TAA | | 588 |

**Figure 7**

```
  1 M   K   C   L   L   I   S   L   A   L   W   L   G   T   V      15
102 ATG AAG TGC TTG CTG ATC TCC CTA GCC CTA TGG CTG GGC ACA GTG   146

 16 G   T   R   G   T   E   P   E   L   S   E   T   Q   R   R      30
147 GGC ACA CGT GGG ACA GAG CCC GAA CTC AGC GAG ACC CAG CGC AGG   191

 31 S   L   Q   V   A   L   E   E   F   H   K   H   P   P   V      45
192 AGC CTA CAG GTG GCT CTG GAG GAG TTC CAC AAA CAC CCA CCT GTG   236

 46 Q   L   A   F   Q   E   I   G   V   D   R   A   E   E   V      60
237 CAG TTG GCC TTC CAA GAG ATC GGT GTG GAC AGA GCT GAA GAA GTG   281

 61 L   F   S   A   G   T   F   V   R   L   E   F   K   L   Q      75
282 CTC TTC TCA GCT GGC ACC TTT GTG AGG TTG GAA TTT AAG CTC CAG   326

 76 Q   T   N   C   P   K   K   D   W   K   K   P   E   C   T      90
327 CAG ACC AAC TGC CCC AAG AAG GAC TGG AAA AAG CCG GAG TGC ACA   371

 91 I   K   P   N   G   R   R   R   K   C   L   A   C   I   K     105
372 ATC AAA CCA AAC GGG AGA AGG CGG AAA TGC CTG GCC TGC ATT AAA   416

106 M   D   P   K   G   K   I   L   G   R   I   V   H   C   P     120
417 ATG GAC CCC AAG GGT AAA ATT CTA GGC CGG ATA GTC CAC TGC CCA   461

121 I   L   K   Q   G   P   Q   D   P   Q   E   L   Q   C   I     135
462 ATT CTG AAG CAA GGG CCT CAG GAT CCT CAG GAG TTG CAA TGC ATT   506

136 K   I   A   Q   A   G   E   D   P   H   G   Y   F   L   P     150
507 AAG ATA GCA CAG GCT GGC GAA GAC CCC CAC GGC TAC TTC CTA CCT   551

151 G   Q   F   A   F   S   R   A   L   R   T   K   *             163
552 GGA CAG TTT GCC TTC TCC AGG GCC CTG AGA ACC AAA TAA           590
```

**Figure 8**

```
                     *        20         *        40         *
HUMAN : MRRLLIPLALWLGAVGVG--VAELTEAQRRGLQVALEEFHKHPPVQWAFQETSVE :  53
MOUSE : MKCLLISLALWLGTVGTRGTEPELSETQRRSLQVALEEFHKHPPVQLAFQEIGVD :  55

           60         *        80         *       100         *
HUMAN : SAVDTPFPAGIFVRLEFKLQQTSCRKRDWKKPECKVRPNGRKRKCLACIKLGSED : 108
MOUSE : RAEEVLFSAGTFVRLEFKLQQTNCPKKDWKKPECTIKPNGRRRKCLACIKMDPKG : 110

                120         *       140         *       160
HUMAN : KVLGRLVHCPIETQVLREAEEHQETQCLRVQRAGEDPHSFYFPGQFAFSKALPRS : 163
MOUSE : KILGRIVHCPILKQ---GPQDPQELQCIKIAQAGEDPHGYFLPGQFAFSRALRTK : 162
```

**Figure 9**

```
        1                                                            50
   mus  MKCLLISLAL WLGTVGTRGT EPELSETQRR SLQVALEEFH KHPPVQLAFQ
   rat  MKCLLISLAL WLGTADIHGT ELELSETQRR GLQVALEEFH RHPPVQWAFQ
  tig2  MRRLLIPLAL WLGAVGV..G VAELTEAQRR GLQVALEEFH KHPPVQWAFQ
   sus  MWQLLLPLAL WLGTMGL..G RAELTAAQLR GLQVALEEFH KHPPVQWAFR
   bos  MWQLLLPLAL GLGTMGL..G RAELTTAQHR GLQVALEEFH KHPPVLWAFQ
gallus  ~RAVGMKLLL GIAVVVLALA DAGQSPLQRR VVKDVLDYFH SRSNVQFLFR

        51                                                          100
   mus  EIGVDRAEEV LFSAGTFVRL EFKLQQTNCP KKDWKKPECT IKPNGRRRKC
   rat  EIGVDSADDL FFSAGTFVRL EFKLQQTSCL KKDWKKPECT IKPNGRKRKC
  tig2  ETSVESAVDT PFPAGIFVRL EFKLQQTSCR KRDWKKPECK VRPNGRKRKC
   sus  ETGVNSAMDT PFPAGTFVRL EFKLQQTSCR KRDWKKAECK VKPNGRKRKC
   bos  VTSVDNAADT LFPAGQFVRL EFKLQQTSCR KKDWRKEDCK VKPNGRKRKC
gallus  EQSVEGAVER VDSSGTFVQL HLNLAQTACR KQAQRKQNCR IMENRRKPVC

        101                                                         150
   mus  LACIKMDPKG ..KILGRIVH C.PILKQGP. Q..DPQELQC IKIAQAGEDP
   rat  LACIKLDPKG ..KVLGRMVH C.PILKQGPQ Q..EPQESQC SKIAQAGEDS
  tig2  LACIKLGSED ..KVLGRLVH C.PIETQVLR EAEEHQETQC LRVQRAGEDP
   sus  LACIKLNSED ..KVLGRMVH C.PIETQVQR EPEERQEAQC SRVERAGEDP
   bos  LACIKLDSKD ..QVLGRMVH C.PIQTQVQR ELDDAQDAQC SRVERAGEDP
gallus  LACYKFDSSD VPKVLDKYYN CGPSHHLAMK DIKHRDEAEC RAVEEAGKTS
```

**Figure 9 - continued**

| | 151 | 168 |
|---|---|---|
| mus | HGYFLPGQFA | FSRALRTK |
| rat | RIYFFPGQFA | FSRAL~~~ |
| tig2 | HSFYFPGQFA | FSKALPRS |
| sus | HSYYFPGQFA | FFKALPPS |
| bos | HSYYLPGQFA | FIKAL~~~ |
| gallus | DVLYLPGMFA | FSKGLP~~ |

Identities :

| | bos.pep | mus.pep | sus.pep | gallus | rat.pep |
|---|---|---|---|---|---|
| tig2.pep | 83.750 | 56.250 | 86.503 | 30.675 | 61.392 |
| bos.pep | | 54.375 | 87.500 | 31.875 | 56.329 |
| mus.pep | | | 54.375 | 31.677 | 73.125 |
| sus.pep | | | | 31.288 | 58.228 |
| gallus.pep | | | | | 30.818 |

**Figure 10**

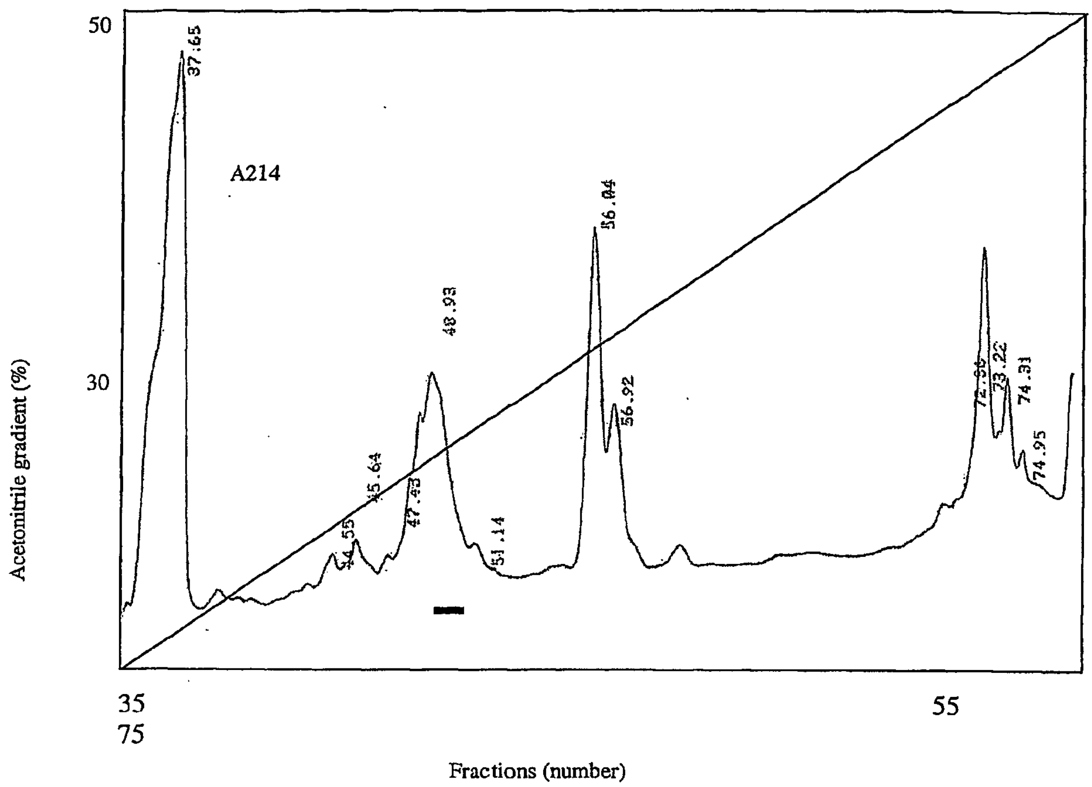
50
30
Acetonitrile gradient (%)
A214
37.65
48.93
56.04
56.92
51.14
47.43
45.64
44.55
72.86
73.22
74.31
74.95
35
75
55
Fractions (number)

Figure 11

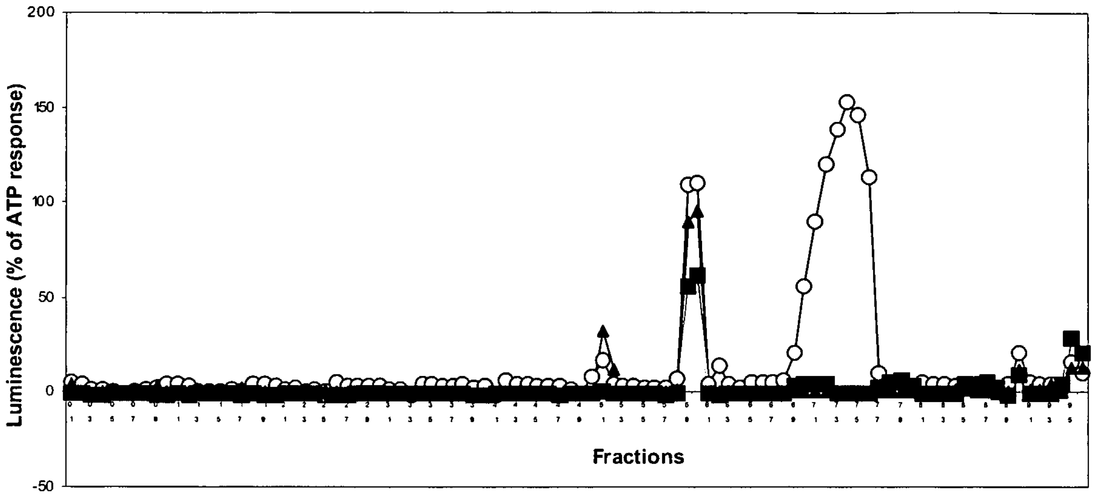

Figure 12

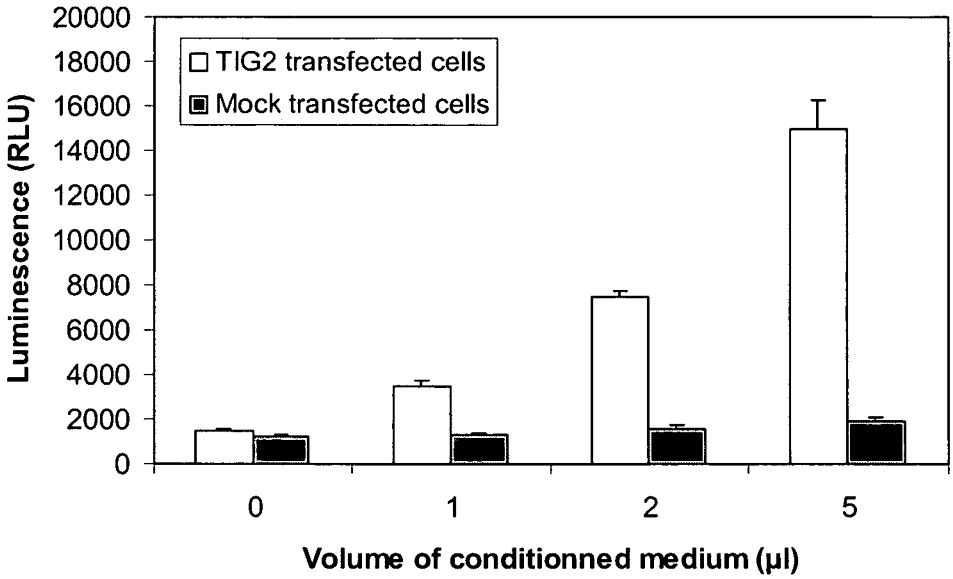
20000
18000
16000
14000
12000
10000
8000
6000
4000
2000
0
Luminescence (RLU)
TIG2 transfected cells
Mock transfected cells
0
1
2
5
Volume of conditionned medium (µl)

Figure 13

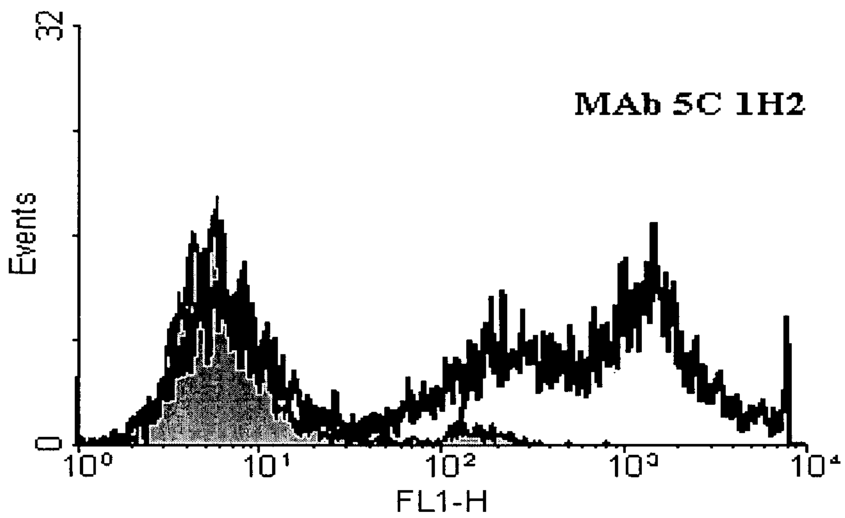

Figure 14

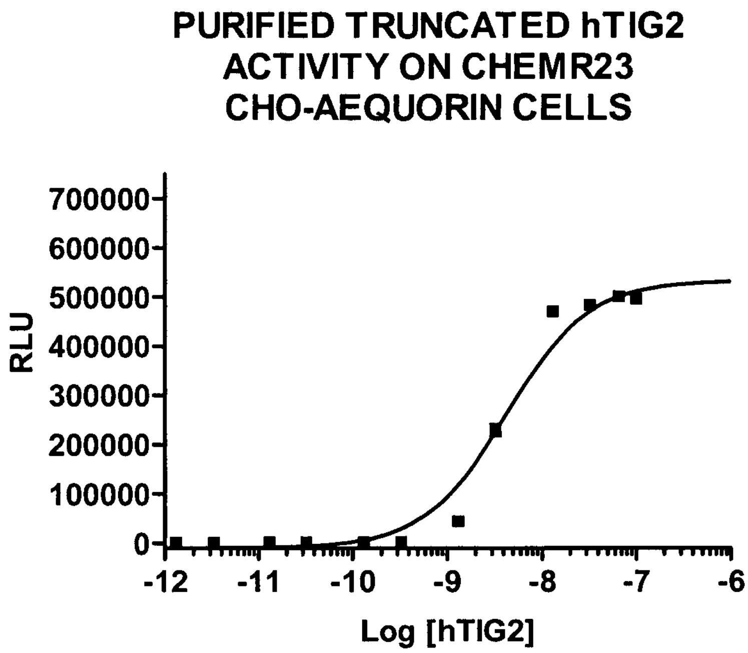
PURIFIED TRUNCATED hTIG2
ACTIVITY ON CHEMR23
CHO-AEQUORIN CELLS
RLU
700000
600000
500000
400000
300000
200000
100000
0
-12
-11
-10
-9
-8
-7
-6
Log [hTIG2]

Figure 15

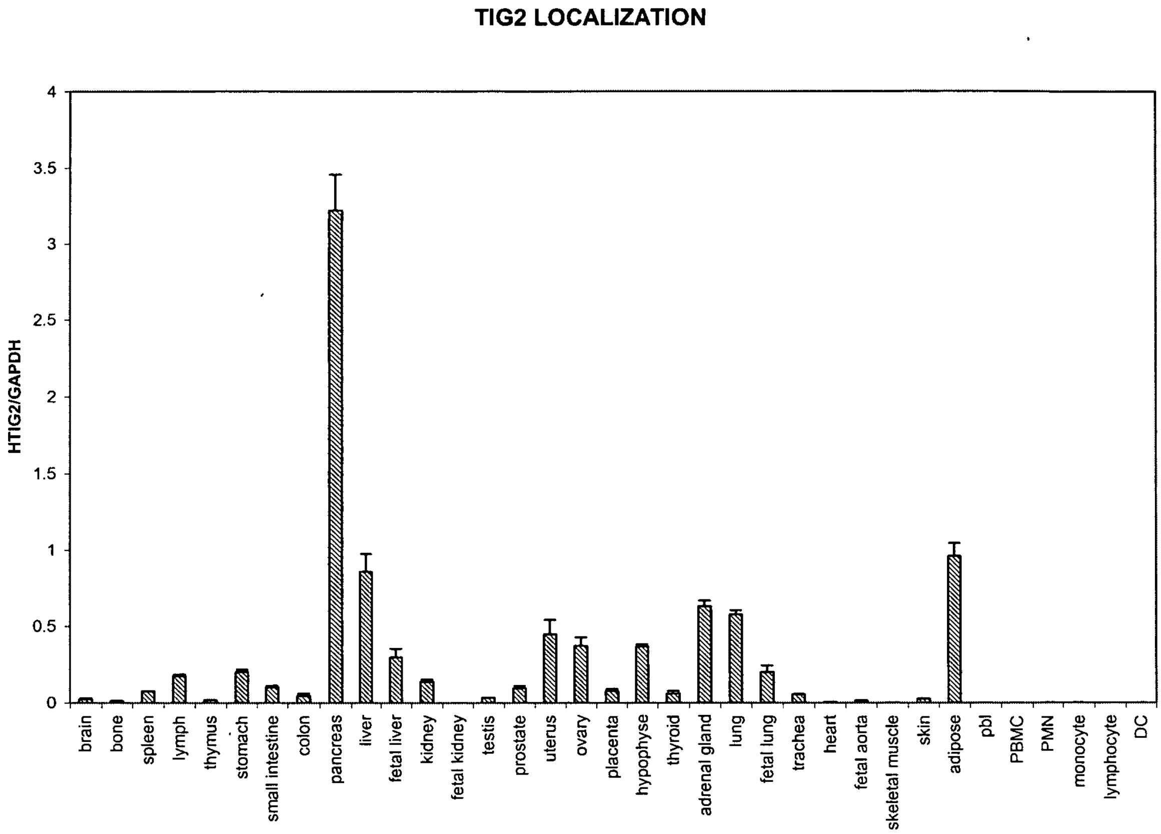
TIG2 LOCALIZATION
HTIG2/GAPDH
4
3.5
3
2.5
2
1.5
1
0.5
0
brain
bone
spleen
lymph
thymus
stomach
small intestine
colon
pancreas
liver
fetal liver
kidney
fetal kidney
testis
prostate
uterus
ovary
placenta
hypophyse
thyroid
adrenal gland
lung
fetal lung
trachea
heart
fetal aorta
skeletal muscle
skin
adipose
pbl
PBMC
PMN
monocyte
lymphocyte
DC

Figure 16

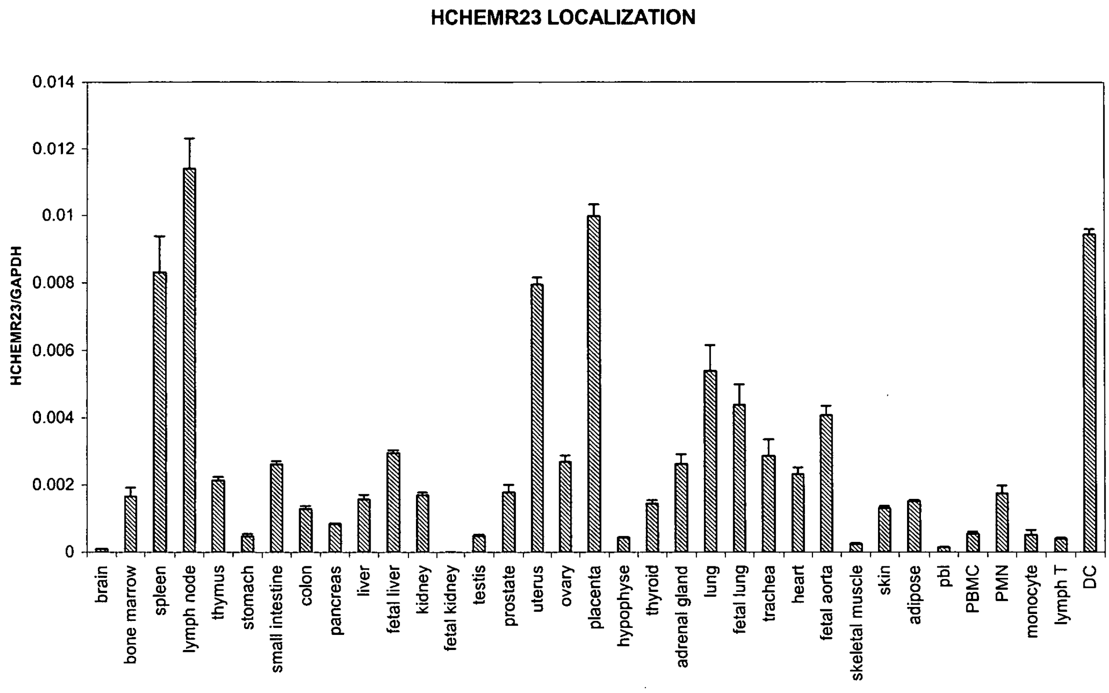
HCHEMR23 LOCALIZATION
HCHEMR23/GAPDH
0.014
0.012
0.01
0.008
0.006
0.004
0.002
0
brain
bone marrow
spleen
lymph node
thymus
stomach
small intestine
colon
pancreas
liver
fetal liver
kidney
fetal kidney
testis
prostate
uterus
ovary
placenta
hypophyse
thyroid
adrenal gland
lung
fetal lung
trachea
heart
fetal aorta
skeletal muscle
skin
adipose
pbl
PBMC
PMN
monocyte
lymph T
DC

**Figure 17**

```
  1 M   R   R   L   L   I   P   L   A   L   W   L   G   A   V     15
  1 ATG CGA CGG CTG CTG ATC CCT CTG GCC CTG TGG CTG GGT GCG GTG   45

 16 G   V   G   V   A   E   L   T   E   A   Q   R   R   G   L     30
 46 GGC GTG GGC GTC GCC GAG CTC ACG GAA GCC CAG CGC CGG GGC CTG   90

 31 Q   V   A   L   E   E   F   H   K   H   P   P   V   Q   W     45
 91 CAG GTG GCC CTG GAG GAA TTT CAC AAG CAC CCG CCC GTG CAG TGG  135

 46 A   F   Q   E   T   S   V   E   S   A   V   D   T   P   F     60
136 GCC TTC CAG GAG ACC AGT GTG GAG AGC GCC GTG GAC ACG CCC TTC  180

 61 P   A   G   I   F   V   R   L   E   F   K   L   Q   Q   T     75
181 CCA GCT GGA ATA TTT GTG AGG CTG GAA TTT AAG CTG CAG CAG ACA  225

 76 S   C   R   K   R   D   W   K   K   P   E   C   K   V   R     90
226 AGC TGC CGG AAG AGG GAC TGG AAG AAA CCC GAG TGC AAA GTC AGG  270

 91 P   N   G   R   K   R   K   C   L   A   C   I   K   L   G    105
271 CCC AAT GGG AGG AAA CGG AAA TGC CTG GCC TGC ATC AAA CTG GGC  315

106 S   E   D   K   V   L   G   R   L   V   H   C   P   I   E    120
316 TCT GAG GAC AAA GTT CTG GGC CGG TTG GTC CAC TGC CCC ATA GAG  360

121 T   Q   V   L   R   E   A   E   E   H   Q   E   T   Q   C    135
361 ACC CAA GTT CTG CGG GAG GCT GAG GAG CAC CAG GAG ACC CAG TGC  405

136 L   R   V   Q   R   A   G   E   D   P   H   S   F   Y   F    150
406 CTC AGG GTG CAG CGG GCT GGT GAG GAC CCC CAC AGC TTC TAC TTC  450

151 P   G   Q   F   A   F   S                                    157
451 CCT GGA CAG TTC GCC TTC TCC                                  471
```

**Figure 18 - 1**

1/ TIG2-1 (SEQ ID NO:48)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | | | | | | | | | | | | | | | 151 |

2/ TIG2-2 (SEQ ID NO:49)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | | | | | | | | | | | | | | 152 |

3/ TIG2-3 (SEQ ID NO:50)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | | | | | | | | | | | | | 153 |

**Figure 18 - 2**

4/ TIG2-4 (SEQ ID NO:51)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | | | | | | | | | | | | 154 |

5/ TIG2-5 (SEQ ID NO:52)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | | | | | | | | | | | 155 |

6/ TIG2-6 (SEQ ID NO:53)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | | | | | | | | | | 156 |

7/ TIG2-7 (SEQ ID NO:46)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |

**Figure 18 – 3**

| | | | | | | | | | | | | | | | | |
|---|---|---|---|---|---|---|---|---|---|---|---|---|---|---|---|---|
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | | | | | | | | | 157 |

8/ TIG2-8 (SEQ ID NO:54)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | | | | | | | | 158 |

9/ TIG2-9 (SEQ ID NO:55)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | A | | | | | | | 159 |

10/ TIG2-10 (SEQ ID NO:56)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | A | L | | | | | | 160 |

11/ TIG2-11 (SEQ ID NO:57)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |

**Figure 18 - 4**

| | | | | | | | | | | | | | | | | |
|---|---|---|---|---|---|---|---|---|---|---|---|---|---|---|---|---|
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | A | L | P | | | | | 161 |

12/ TIG2-12 (SEQ ID NO:58)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | A | L | P | R | | | | 162 |

13/ TIG2-7 (SEQ ID NO:8)

| | | | | | | | | | | | | | | | | |
|---|---|---|---|---|---|---|---|---|---|---|---|---|---|---|---|---|
| 1 | M | R | R | L | L | I | P | L | A | L | W | L | G | A | V | 15 |
| 16 | G | V | G | V | A | E | L | T | E | A | Q | R | R | G | L | 30 |
| 31 | Q | V | A | L | E | E | F | H | K | H | P | P | V | Q | W | 45 |
| 46 | A | F | Q | E | T | S | V | E | S | A | V | D | T | P | F | 60 |
| 61 | P | A | G | I | F | V | R | L | E | F | K | L | Q | Q | T | 75 |
| 76 | S | C | R | K | R | D | W | K | K | P | E | C | K | V | R | 90 |
| 91 | P | N | G | R | K | R | K | C | L | A | C | I | K | L | G | 105 |
| 106 | S | E | D | K | V | L | G | R | L | V | H | C | P | I | E | 120 |
| 121 | T | Q | V | L | R | E | A | E | E | H | Q | E | T | Q | C | 135 |
| 136 | L | R | V | Q | R | A | G | E | D | P | H | S | F | Y | F | 150 |
| 151 | P | G | Q | F | A | F | S | K | A | L | P | R | S | | | 163 |